(19) Europäisches Patentamt
European Patent Office
Office européen des brevets

(11) EP 3 540 034 A1

(12) **EUROPEAN PATENT APPLICATION**

(43) Date of publication:
**18.09.2019 Bulletin 2019/38**

(21) Application number: **18161358.9**

(22) Date of filing: **13.03.2018**

(51) Int Cl.:
*C11D 1/94* (2006.01) *C07K 14/415* (2006.01)
*C11D 3/382* (2006.01) *C11D 11/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Procter & Gamble Company**
**Cincinnati, OH 45202 (US)**

(72) Inventors:
- **GONZALES, Denis Alfred**
**1853 Strombeek-Bever (BE)**
- **BETTIOL, Jean-Luc Philippe**
**1853 Strombeek-Bever (BE)**

(74) Representative: **P&G Patent Belgium UK**
**N.V. Procter & Gamble Services Company S.A.**
**Temselaan 100**
**1853 Strombeek-Bever (BE)**

(54) **HAND DISHWASHING DETERGENT COMPOSITION**

(57) The present invention is directed to a detergent composition having enhanced suds boosting and/or increased suds longevity especially in the presence of greasy soils, wherein the detergent composition is a hand dishwashing detergent composition. The composition includes a specific surfactant system including an anionic surfactant and a primary co-surfactant, wherein the weight ratio of the anionic surfactant to the primary co-surfactant is less than 9:1, preferably from 5:1 to 1:1, more preferably from 4:1 to 2:1, and a plant derived protein or blend of plant derived proteins, preferably derived from a plant seed protein, preferably selected from the group consisting of a Canola protein, a Hemp protein, a Flaxseed protein, and mixtures thereof, preferably a Canola protein.

EP 3 540 034 A1

## Description

REFERENCE TO A SEQUENCE LISTING

This application contains Sequence Listings in computer readable form. The computer readable form is incorporated herein by reference.

FIELD OF THE INVENTION

The present invention relates to a detergent composition comprising a specific surfactant system and a plant derived protein or blend of plant derived proteins derived from a plant seed family, preferably the plant seed protein is selected from the group consisting of a Canola protein, a Hemp protein, a Flaxseed protein, and mixtures thereof, wherein the detergent composition is a hand dishwashing detergent composition.

BACKGROUND OF THE INVENTION

Detergent compositions should provide good soil and/or grease cleaning while presenting a good sudsing profile in particular a long-lasting suds profile especially in the presence of greasy soils. Users usually see suds as an indicator of the performance of the detergent composition. Moreover, the user of a detergent composition may also use the sudsing profile and the appearance of the suds (e.g., density) as an indicator that the wash solution still contains sufficient active cleaning ingredients. This is particularly the case for manual washing, also referred to herein as hand-washing, where the user usually doses the detergent composition depending on the suds remaining and renews the wash solution when the suds subsides or when the suds does not look thick enough. Thus, a detergent composition, particularly a hand dishwashing detergent composition that generates or maintains low density suds during the dishwashing process would tend to be replaced by the user more frequently than is necessary. Thus, it is desirable for a detergent composition to provide a "good sudsing profile", which includes good suds height and/or density as well as good suds duration (*i.e.,* increased suds longevity) during the initial mixing of the composition with water and/or during the entire washing operation. In recent years, users also desire that hand dishwashing detergents are formulated with ingredients that will have minimal negative impact on the environment and/or the health of the users.

Suds can be formed and stabilized by surfactants and/or proteins. By co-formulating with naturally derived plant proteins, it is possible to reduce the levels of surfactants utilized and mitigate against the negative environmental impact while still maintaining a good sudsing profile. Several families of plant derived proteins naturally derived from plant seed family such as for example Canola protein, Hemp protein and Flaxseed protein are able to aid suds performance.

Canola protein, also known as rapeseed or oil seed rape, has been studied for their foaming capacity (FC) and foaming stability (FS) in food (Kinsella, JE., Food Chem., 1981;7(4): 273-288). The Canola protein can be readily isolated/ extracted from Canola oil seed meal using multi-step process, such as for example, as described in US Patent Publication Nos. US2003/0125526A1, US2004/0254353 A1, and PCT Publication Nos. WO02/089597 and WO2009/18660A1. The inclusion of isolated Canola protein in human food results in higher FS than with Canola protein (Tan, S. H., et al., J. Food Sci., 2011 Jan; 76(1); R16-R28). However, the inclusion of Canola protein, particularly Canola protein isolates, in the context of hand dishwashing detergent compositions for improving sudsing profile, particularly increased suds longevity especially in the presence of greasy soils, has not been disclosed.

Hemp protein is an industrial byproduct of hempseed, which has its oil extracted into hempseed oil, and the remaining seed meal that is high in protein is processed into hemp protein. The Hemp protein can be isolated/ extracted from hemp seed meal using multi-step process, such as for example, as described in PCT Publication Nos. WO 2014/419074 and WO 2014/145057. The Hemp protein isolate is suitable for use as foaming agent in (foods and beverages) products and for the emulsification of oils in baked goods (*see* para. [0015], WO 2014/419074). However, there is no mention of the use of Hemp protein, particularly Hemp protein isolates, in hand dishwashing detergent compositions for improving sudsing profile, particularly increased suds longevity, especially in the presence of greasy soils.

Flaxseed protein, which is also known as flax or linseed, can be isolated/ extracted from flax oil seeds, whereby the flax oil seeds are initially extracted to remove mucilage prior to crushing to recover the flax oil and produce a flaxseed meal. The Flaxseed protein can be isolated/ extracted from flaxseed meal using the process, such as for example, as described in PCT Publication No. WO 2005/12342. The Flaxseed protein isolate is suitable for use as foaming agent in (food) products and for the emulsification of oils in baked goods (*see* para. [0014], WO 2005/12342). WO 2010/53488 A1 describes in Example 1 body wash compositions comprising flaxseed extract (Natunola™ Flax Extract 130), C10-16 alcohol ethoxylate sodium sulfate and cocamidopropyl betaine surfactants to provide desired level of moisture to the skin. According to WO ’488, para. [0020], the combination of the adduct and the flaxseed extract forms a moisture barrier to the skin to help retain natural oils and moisture. However, there is no mention of the use of Flaxseed protein and/or

Flaxseed protein isolates in hand dishwashing detergent compositions for improving sudsing profile, particularly increased suds longevity, especially in the presence of greasy soils.

Accordingly, the need remains for an improved detergent composition comprising a plant derived protein, preferably derived from a plant seed and a specific surfactant system, which provides a good sudsing profile, in particular enhanced suds boosting and/or increased suds longevity, especially in the presence of greasy soils. The composition may also provide good cleaning, particularly good grease emulsification. It is desirous to reduce the levels of surfactants in the composition versus traditional formulations without negatively impacting sudsing, grease cleaning and/or emulsification profile. The Applicant discovered that some or all of the above-mentioned needs can be at least partially fulfilled through the improved detergent composition as described herein below.

## SUMMARY OF THE INVENTION

The present invention meets one or more of these needs based on the surprising discovery that by formulating a detergent composition comprising a specific surfactant system working in synergy with a plant derived protein or blend of plant derived proteins, preferably derived from a plant seed, wherein the detergent composition is a hand dishwashing detergent composition, such a composition exhibits good sudsing profile, particularly desirable suds volume and/or increased suds longevity, especially in the presence of greasy soils. The composition also provides good grease cleaning and emulsification benefits.

According to a first aspect, the present invention is directed to a detergent composition comprising: a) from about 1 wt% to about 60 wt%, preferably from about 5 wt% to about 50 wt%, more preferably from about 8 wt% to about 45 wt%, even more preferably from about 15 wt% to about 40 wt%, by weight of the composition of a surfactant system; and b) from about 0.1 wt% to about 10 wt%, preferably from about 0.5 wt% to about 5 wt%, by weight of the composition of a plant derived protein or blend of plant derived proteins derived from a plant seed family, wherein the detergent composition is a hand dishwashing detergent composition. The surfactant system comprises: i) an anionic surfactant, preferably the anionic surfactant is selected from the group consisting of alkyl sulfate, alkyl alkoxy sulfate, and mixtures thereof; and ii) a primary co-surfactant selected from the group consisting of an amphoteric surfactant preferably an amine oxide surfactant, a zwitterionic surfactant preferably a betaine surfactant, and mixtures thereof, preferably the primary co-surfactant is an amine oxide surfactant, wherein the weight ratio of the anionic surfactant to the primary co-surfactant is less than 9:1, preferably from 5:1 to 1:1, more preferably from 4:1 to 2:1. Preferably the plant seed protein is selected from the group consisting of a Canola protein, a Hemp protein, a Flaxseed protein, and mixtures thereof, most preferably the plant seed protein is a Canola protein. Preferably, the composition is essentially free, preferably free, of animal-, fungal- and/or bacterial-derived proteins. It has been surprisingly found that the composition of the present invention creates long lasting suds under a hand dishwashing operation, especially in the presence of greasy soils.

In another aspect, the present invention is directed to a method of manually washing dishware comprising the steps of delivering a composition according to the claims to a volume of water to form a wash liquor and immersing the dishware in the wash liquor, or delivering a composition according to the claims directly onto the dishware or cleaning implement and using the cleaning implement to clean the dishware. When the composition of the invention is used according to this method a good sudsing profile, with a long-lasting effect is achieved, especially in the presence of greasy soils.

There is also provided the use of a detergent composition of the claims to provide increased suds longevity of the composition, especially in the presence of greasy soils, wherein the detergent composition is a hand dishwashing detergent composition.

One aim of the present invention is to provide a detergent composition which can exhibit good sudsing profile, in particular enhanced suds boosting and/or increased suds longevity, especially in the presence of greasy soils, preferably over the entire dishwashing process, wherein the detergent composition is a hand dishwashing detergent composition.

Another aim of the present invention is to provide such a composition having good tough food cleaning (e.g., cooked-, baked- and burnt-on soils) and/or good grease cleaning.

Yet another aim of the present invention is to provide a use of a composition, comprising a plant derived protein or blend of plant derived proteins which function to increase suds longevity and/or facilitate the reduction of surfactants in the formulation. Thus, it is an advantage of the invention to minimize production costs and/or reduce negative environmental impact.

A further aim of the present invention is to provide such a composition comprising a plant derived protein or blend of plant derived proteins, in a form which is water soluble and/or transparent resulting in improved water solubility and/or transparency of the composition, particularly in an aqueous environment.

Yet a further aim of the present invention is to provide such a composition comprising a plant derived protein or blend of plant derived proteins resulting in a composition that has low or is essentially free of phytic acid and/or protein-bound carbohydrate. This is believed to contribute to improved water solubility of the composition and/or improved plant

derived protein performance to enhance sudsing profile.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## DETAILED DESCRIPTION OF THE INVENTION

### Definitions

As used herein, the articles "a" and "an" when used in a claim, are understood to mean one or more of what is claimed or described.

As used herein, the term "amino acid identity" means the identity between a polypeptide subunit or a protomer of the plant derived protein and the reference amino acid sequence and is expressed in terms of the identity or similarity between the subunit or the protomer and the sequence. Sequence identity can be measured in terms of percentage identity; the higher the percentage, the more identical the sequences are. The percentage identity is calculated over the length of comparison. For example, the Canola Cruciferin is the predominant 11S protein in the Brassicaceae family. The mature native Canola Cruciferin contains six subunits or protomers that assemble as two trimer units in which each protomer is comprised of two polypeptide: $\alpha$-(~40 kDa, 254 to 296 amino acids) and $\beta$- (~20 kDa, 189 to 191 amino acids) chain linked *via* a disulfide bond. The amino acid identity of a Canola Cruciferin is typically calculated over the entire length of a subunit or protomer aligned against the entire length of the reference sequence (*e.g.,* SEQ ID NOs: 1-10). Methods of alignment of sequences for comparison are well known in the art and identity can be calculated by many known methods. Various programs and alignment algorithms are described in the art. It should be noted that the terms 'sequence identity' and 'sequence similarity' can be used interchangeably. For polypeptide sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps.

As used herein the term "animal protein" means protein that is derived from meat, or dairy products such as milk, eggs and the like.

As used herein the term "bacterial derived protein" means protein that are produced by bacteria.

As used herein the term "fungal derived protein" means protein that is derived from fungi.

As used herein, "plant derived protein" or "plant seed protein" means protein that is derived from plant or plant seed sources (*e.g.,* grain). Furthermore, the term "plant derived protein" or "blend of plant derived proteins" also mean a protein composition derived from plant sources that is uncontaminated by animal, fungal or bacterial products or any animal-, fungal- or bacterial-derived peptides that are derived from the fermentation media or the purification media.

As used herein, the term "dishware" includes cookware and tableware.

As used herein the term "enhanced suds boosting" means a higher volume of suds is generated upon the dissolution of the detergent composition in a washing solution for a composition comprising a plant derived protein or blend of plant derived proteins and a specific surfactant system of the present invention, as compared with the suds longevity provided by the same composition and process in the absence of the plant derived protein or blend of plant derived proteins and/or the specific surfactant system of the present invention.

As used herein, the term "essentially free" when used to described a component means less than 0.005% by weight of the total composition of the component is present in the detergent composition.

As used herein, the term "hand dishwashing detergent composition" refers to a composition or formulation designed for cleaning dishware. The composition is commercially positioned for manual-washing of dishware. Preferred compositions are in the form of a liquid.

As used herein the term "enhanced suds boosting" means a higher volume of suds is generated upon the dissolution of the detergent composition in a washing solution for a composition comprising a plant derived protein or blend of plant derived proteins and a specific surfactant system of the present invention, as compared with the suds longevity provided by the same composition and process in the absence of the plant derived protein or blend of plant derived proteins and the specific surfactant system of the present invention.

As used herein the term "increased suds longevity" means an increase in the duration of visible suds in a washing process for cleaning soiled dishware in this case when using the composition comprising a plant derived protein or blend of plant derived proteins and a specific surfactant system of the present invention, compared with the suds longevity provided by the same composition and process in the absence of the plant derived protein or blend of plant derived proteins and the specific surfactant system of the present invention.

As used herein the term "protein isolate" means a protein that has been isolated from a plant source based on

well-known extraction processes to those skilled in the art, such as for example alkali extraction and acid preparation, protein micellation method (PMM), or low pH extraction combined with protein isolate preparation (Wanadundara et al., OCL 2016, 23(4) D407). Depending on the method of protein extraction employed, the final product could vary in terms of the protein content, type and extent of interaction with non-protein components. Isolates are more pure than other forms (*e.g.,* concentrates) as other non-protein components have been removed to "isolate" the protein of interest. Preferably, the protein isolate has a protein content (as determined by Kjeldahl Nx6.25) of at least about 80 wt% or more, preferably about 90 wt% or more, more preferably 100%, is substantially undenatured (as determined by differential scanning calorimetry) and has a low residual fat content of less than about 1 wt%.

As used herein the term "protomer" means the structural unit of an oligomeric protein. It is the smallest unit composed of at least two different protein chains that form a larger heterooligomer by association of two or more copies of this unit.

As used herein the term "subunit" means a single protein molecule that assembles (or "co-assembles") with other protein molecules to form a protein complex.

As used herein the term "sudsing profile" refers to the properties of a detergent composition relating to suds character during the dishwashing process. For example, the sudsing profile of a detergent composition includes but is not limited to the suds generation upon dissolving of the detergent composition, and the volume and retention of the suds during the dishwashing process.

It is understood that the test methods that are disclosed in the Test Methods Section of the present application must be used to determine the respective values of the parameters of Applicants' inventions as described and claimed herein.

In all embodiments of the present invention, all percentages are by weight of the total composition, as evident by the context, unless specifically stated otherwise. All ratios are weight ratios, unless specifically stated otherwise, and all measurements are made at 25°C, unless otherwise designated.

Detergent Composition

The inventors have surprisingly discovered a new way of formulating a detergent composition to provide good sudsing profile, particularly increased suds longevity, preferably in the presence of greasy soil. Essentially, the solution is to formulate a specific surfactant system which synergizes with a plant derived protein or blend of plant derived proteins derived from a plant seed family. In fact, the inventors have discovered that when the specific surfactant system is co-formulated with the plant derived protein or blend of plant derived proteins, increased suds longevity, especially in the presence of greasy soil, is obtained. This enhanced sudsing is not observed with close surfactant systems outside the scope of the invention (*see* examples section). While not wishing to be bound by theory, it is believed that the specific surfactant system containing the plant derived protein or blend of plant derived proteins may more easily go to the air-water interface and remain in the suds film lamellae due to its specific physical properties. As a result, the longevity of the suds is increased due to the plant derived protein-plant derived protein interactions that form strong continuous interfacial membrane that stabilizes the suds particles at the air-water interface.

In addition, the inventors have discovered that the plant derived protein or blend of plant derived proteins and surfactant system in the detergent composition also provides enhanced suds boosting benefit. Preferably, the detergent composition of the invention also provides good grease removal, in particular good uncooked grease removal.

The detergent composition of the present invention is a manual dishwashing composition, preferably in liquid form. It typically contains from about 30 wt% to about 95 wt%, preferably from about 40 wt% to about 90 wt%, more preferably from about 50 wt% to about 85 wt% by weight of the composition of a liquid carrier in which the other essential and optional components are dissolved, dispersed or suspended. One preferred component of the liquid carrier is water.

Preferably, the detergent composition of the present invention comprises a phytic acid content of about 0.5 wt% or less, preferably about 0.2 wt% or less, preferably about 0.1 wt% or less, preferably about 0.01 wt% or less by weight of the composition, most preferably the composition is essentially free, preferably free, of the phytic acid. Plant seed meals, including Canola, Hemp and Flaxseed, contain phytic acid. Phytic acid (*i.e.,* myo-inositol 1,2,3,4,5,6-hexakis (dihydrogen phosphate)) is a form of phosphorus (P) in seeds which is stored in the form of phytate salts. The term "phytic acid" as used herein includes such phytate salt forms. Depending on the seed type, the content of phytic acid may range from about 0.3 wt% to about 10 wt%. Extraction of the plant seed meals results in the presence of phytic acid in the protein isolate recovered. Phytic acid has a negative impact on the protein isolates, specifically, the presence of phytic acid reduces the protein solubility and/or flexibility thereby preventing its absorption at the air-water interface. As the quantity of phytic acid in the protein isolate increases, the negative impact of the protein performance increases. Thus, it is desirable to incorporate seed protein isolates that have substantially reduced or are essentially free of phytic acid. Reduced amounts of phytic acid content in the protein isolates from extraction of the seed meal may be achieved by extraction at temperatures above 50°C, in the presence of $CaCl_2$ or $MgCl_2$, and/or in the presence of from about 0.01% to about 1% phytase. Following these actions, the precipitated phytate can be removed from the protein solution

such as by centrifugation.

The detergent composition of the present invention preferably comprises a protein-bound carbohydrate content of about 2 wt% or less, preferably about 1 wt% or less, preferably about 0.5 wt% or less, preferably about 0.1 wt% or less, preferably about 0.01 wt% or less by weight of the composition, most preferably the composition is essentially free, preferably free, of the protein-bound carbohydrate. The term "protein-bound carbohydrate" as used herein means an isolated protein that has carbohydrate bound (chemically or physically) to it. Carbohydrate bound proteins have decrease performance because the carbohydrate screen the active sites of the protein and reduces the protein solubility, flexibility and/or mobility thereby preventing its absorption at the air-water interface. Therefore, it is desirable to limit the level of isolated proteins that are bound to carbohydrates in the detergent composition. Reduced amounts of protein-bound carbohydrates in the protein isolates from extraction of the seed meal may be achieved by extraction with from about 0.01% to about 1% of a carbohydrate hydrolyzing enzyme, preferably carbohydrase. The carbohydrate residues can then be separated from the protein isolate fractions such as by membrane or dialysis filtration.

Preferably the pH of the detergent composition, measured as a 10% product concentration (*i.e.,* dilution) in distilled water at 20°C, is adjusted to between about 6 and about 14, more preferably between about 7 and about 12, more preferably between about 7.5 and about 10. The pH of the composition can be adjusted using pH modifying ingredients known in the art.

The composition of the present invention can be Newtonian or non-Newtonian, preferably Newtonian. The composition has a viscosity of from about 10 to about 10,000 mPa·s, preferably from about 100 to about 5,000 mPa·s, more preferably from about 300 to about 2,000 mPa·s, or most preferably from about 500 to about 1,500 mPa·s. Viscosity is measured with a Brookfield DV-II+ Pro Viscometer using spindle 31 at 12 RPM at 20°C.

Plant Derived Protein

The plant derived protein or blend of plant derived proteins are derived from a plant seed family. The plant seed family is selected from the group consisting of Brassica oleracea, Brassica rapa, Raphanus sativus, Armoracia rusticana, Brassica rapa oleifera, Brassica campestris, Brassica juncea, Brassica napus, Boehmeria cylindrica, Crotalaria juncea, Corchorus olitorius, Hibiscus cannabinus, Musa textilis, Phormium tenax, Hibiscus sabdariffa, Agave sisalana, Cannabis indica, Cannabis ruderalis, Cannabis sativa, Linum alatum, Linum album, Linum arenicola, Linum aristatum, Linum australe, Linum austriacum, Linum berlandieri, Linum bienne, Linum campanulatum, Linum carteri, Linum catharticum, Linum compactum, Linum cratericola, Linum dolomiticum, Linum elongatum, Linum flavum, Linum floridanum, Linum grandiflorum, Linum hirsutum, Linum hudsonioides, Linum imbricatum, Linum intercursum, Linum kingii, Linum leoni, Linum lewisii, Linum lundellii, Linum macrocarpum, Linum marginale, Linum medium, Linum monogynum, Linum narbonense, Linum neomexicanum, Linum perenne, Linum pratense, Linum puberulum, Linum pubescens, Linum rigidum, Linum rupestre, Linum schiedeanum, Linum striatum, Linum subteres, Linum suffruticosum, Linum sulcatum, Linum tenuifolium, Linum trigynum, Linum vernal, Linum virginianum, Linum westii, and Linum usitatissimum, preferably Brassica juncea, Brassica napus, Cannabis sativa, and Linum usitatissimum. Preferably, the plant seed protein is selected from the group consisting of a Canola protein, a Hemp protein, a Flaxseed protein, and mixtures thereof, more preferably the plant seed protein is a Canola protein, with the proviso that when the plant seed protein is a Flaxseed protein, then the primary co-surfactant cannot be zwitterionic surfactant preferably betaine.

Plant seed proteins typically contain protein components/fractions that are known as seed storage proteins that can be classified into four groups: Albumins, Globulins, Prolamins, and Glutelines. Globulins and Albumins are the two predominant seed storage proteins. The seed storage proteins from different plants (*e.g.,* Canola, Hemp, Flaxseed) share certain common characteristics. One of the main characteristics of seed storage proteins is that they can be classified by their sedimentation coefficient in Svedberg units (S). This coefficient indicates the speed of sedimentation of a macromolecule in a centrifugal field. For example, the Globulins are broadly classified into 7S, 8S and 11S, 12S, and the Albumins are broadly classified into 1.6S, 1.7S, and 2S. It should be noted however that some small variations of sedimentations are expected depending on the type of seed and/or the extraction protocol employed. Therefore, the sedimentation coefficient is not intended as being restrictive, but rather serve as a useful guide for the classification of the seed storage proteins.

The inventors have surprisingly discovered that by formulating with plant seed proteins, it is possible to obtain a good sudsing profile, in particular enhanced suds boosting and/or increased suds longevity, especially in the presence of greasy soils. Use of protein isolates that target protein in highly pure form eliminates most of the undesirable interference from non-protein components. Therefore, it is preferred that the plant seed proteins of the present invention are used in the form of plant seed protein isolates. Preferably, the protein isolates have been extracted by protein micellization process and/or ultra-filtration, optionally followed by re-blending of the separated protein isolate fractions to achieve the desired ratio of the Globulins to Albumins to maximize sudsing performance.

**Canola Protein**

Canola protein, also known as rapeseed or oil seed rape, belongs to the Brassicaceae plant family. Preferably, the Canola protein isolate is obtained by extraction from a Canola oil seed meal according to a process as disclosed for example in U.S. Patent Publication Nos. US2003/0125526A1, US2004/0254353 A1, and PCT Publication Nos. WO02/089597, WO03/043439 and WO2009/18660A1. Alternatively, the Canola protein isolate is commercially available as Nutratein®, Puratein® and Supertein® from Burcon Nutrascience (Vancouver, Canada).

Canola proteins can be divided into various fractions according to the corresponding sedimentation coefficient. For Canola proteins, the main reported fractions are: 2S and 12S. Other fractions of the Canola proteins include: 1.6S, 1.7S, 7S, 8S, and 11S. Preferably, the composition of the present invention may comprise a Canola protein which is a Canola protein isolate comprising:

a. a first Canola protein isolate component comprising a Canola Albumin (1.6S, 1.7S, 2S) protein, preferably from about 20 wt% to about 95 wt%, more preferably from about 50 wt% to about 95 wt%, by weight of the total Canola protein level in the composition;
b. a second Canola protein isolate component comprising a Canola Globulin (7S,8S) protein, preferably from about 5 wt% to about 80 wt%, more preferably from about 5 wt% to about 50 wt%, by weight of the total Canola protein level in the composition; and
c. a third Canola protein isolate component comprising a Canola Globulin (11S, 12S) protein, preferably from 0 wt% to about 30 wt%, more preferably from 0 wt% to about 2 wt%, by weight of the total Canola protein level in the composition

For some plants, the Globulins and Albumins are given names that are specific to the plant. For example, the Globulin storage protein of Canola is called Cruciferin and the Albumin storage protein of Canola is called Napin. Preferably, the composition of the present invention comprises a Canola protein which is a Canola protein isolate comprising the two predominant storage proteins which are a Canola Albumin preferably known as Napin, a Canola Globulin preferably known as Cruciferin, or mixtures thereof.

The mature Napin is a protein comprising a small (short, 4 kDa) and a large (long, 9 kDa) polypeptide chain linked together by two inter-chain disulfide bonds. Specifically, Napins are proposed for use in applications for suds generation. Without wishing to be bound by theory, it is believed that the Albumin functions for suds generation because it has small molecular mass and high flexibility that allows for fast absorption at the air-water interface. Cruciferins are Globulins and are the major storage protein in the seed. The mature Cruciferin is composed of 6 subunits or protomers that assemble as two trimer units in which each protomer is comprised of two polypeptide: $\alpha$- (~40 kDa, 254 to 296 amino acids) and $\beta$- (~20 kDa, 189 to 191 amino acids) chain linked *via* a disulfide bond and has a total molecular mass of approximately 300 kDa (Tandang-Silvas et al., Biochem. Biophys. Acta (BBA) - Proteins and Proteomics, (2010) 1804:1432-1442). Specifically, Cruciferins are proposed for use in applications for suds stabilization. It is believed that the Globulins function for suds stability because it has large molecular mass and can form protein-protein and protein-surfactant network at the air-water interface. Therefore, it is highly preferable for a composition of the present invention to include a blend of plant derived proteins comprising an Albumin (*i.e.,* Napin), a Globulin (*i.e.,* Cruciferin), or mixtures thereof, in order to enhance suds boosting and/or increase suds longevity benefits especially in the presence of greasy soils.

Preferably, the detergent composition of the present invention comprises a Canola protein which has at least 50%, preferably at least 60%, preferably at least 70%, preferably at least 80%, preferably at least 85%, preferably at least 90%, preferably at least 95%, preferably at least 98% or even 100% amino acid identity to a Canola Cruciferin protein (SEQ ID NOs: 1-10) and/or a Canola Napin protein (SEQ ID NOs: 11-28).

**Hemp Protein**

*Cannabis sativa L.,* commonly refer to as Hemp is a widely-cultivated plant of industrial importance. Hemp protein isolate (HPI) is suitable for use as a foaming agent in products which entrap gases (WO2014/19074). Preferably the Hemp protein isolate is obtained by extraction from a hemp seed meal according to the process as disclosed for example in PCT Publication Nos. WO 2014/419074 and WO 2014/145057. Alternatively, the Hemp protein isolate is commercially available as hemp protein from Myprotein (United Kingdom).

Hemp protein is made up primarily of two types of proteins, a Globulin seed storage protein called Edestin and a Hemp Albumin protein. Edestin is a globular protein having aminoacid based structure very similar to the natural human globulins. Hemp Albumin is a high quality globulin protein. Preferably, the composition of the present invention comprises a Hemp protein which is a Hemp protein isolate comprising the two predominant storage proteins which are a Hemp Albumin (1.6S, 1.7S, 2S), a Hemp Globulin preferably known as Edestin, or mixtures thereof. Preferably, the Hemp

protein isolate is obtained by extraction from a hemp seed meal after the hemp oil have been extracted from the seed. Preferably, the composition of the present invention may comprise a Hemp protein which is a Hemp protein isolate comprising:

a. a first Hemp protein isolate component comprising a Hemp Albumin protein, preferably from about 60 wt% to about 95 wt%, more preferably from about 70 wt% to about 95 wt%, by weight of the total Hemp protein level in the composition; and
b. a second Hemp protein isolate component comprising a Hemp Edestin protein, preferably from about 5 wt% to about 40 wt%, more preferably from about 5 wt% to about 30 wt%, by weight of the total Hemp protein level in the composition

The Hemp Globulin (*i.e.,* Edestin) and Hemp Albumin provides the same function for suds generation and stabilization as described above for the Canola protein. Preferably, the detergent composition of the present invention comprises a Hemp protein which has at least 50%, preferably at least 60%, preferably at least 70%, preferably at least 80%, preferably at least 85%, preferably at least 90%, preferably at least 95%, preferably at least 98% or even 100% amino acid identity to a Hemp Edestin protein: (SEQ ID NOs: 29-31) and/or to a Hemp Albumin protein (SEQ ID NO: 32).

**Flaxseed Protein**

Flaxseed (*Linum usitatissinum L.*), also commonly known as flax or linseed, is a member of the genus Linum in the family Linaceae. It is traditionally used as a food and fiber crop. Preferably, the Flaxseed protein isolate is obtained by extraction from a Flaxseed meal according to a process as disclosed for example in PCT Publication No. WO 2005/12342. Alternatively, the Flaxseed protein can be extracted from Flax seed that are commercially available from Myprotein (UK).

Flaxseed contains several different protein components/fractions, distinguished by different sedimentation coefficients (S). It should be noted however that some small variations of sedimentations are expected depending on the type of seed and/or the extraction protocol employed. Therefore, the sedimentation coefficient is not intended as being restrictive, but rather serve as a useful guide for the classification of the seed storage proteins. The proteins include a seed storage protein Albumin (2S), preferably known as Conlinin (2S), and a seed storage protein Globulin (12S), preferably known as Linin (12S). Conlinin is the major protein associated with Flaxseed. Preferably, the composition of the present invention comprises a Flaxseed protein which is a Flaxseed protein isolate comprising the two predominant storage proteins which are a Flaxseed Albumin preferably Conlinin (2S), Flaxseed Globulin preferably Linin (12S), or mixtures thereof. Preferably, the Flaxseed protein isolate is obtained by extraction from a Flaxseed meal

Preferably, the composition of the present invention may comprise a Flaxseed protein which is a Flaxseed protein isolate comprising:

a. a first Flaxseed protein isolate component comprising a Conlinin (2S) protein, preferably from 0 wt% to about 35 wt% by weight of the total Flaxseed protein level in the composition; and
b. a second Flaxseed protein isolate component comprising a Linin (12S) protein, preferably from about 65 wt% to about 100 wt% by weight of the total Flaxseed protein level in the composition.

The Flaxseed Globulin (*i.e.,* Linin) and Flaxseed Albumin (*i.e.,* Conlinin) provides the same function for suds generation and stabilization as described above for the Canola protein.

Preferably, the detergent composition of the present invention comprises a Flaxseed protein which has at least 50%, preferably at least 60%, preferably at least 70%, preferably at least 80%, preferably at least 85%, preferably at least 90%, preferably at least 95%, preferably at least 98% or even 100% amino acid identity to a Flaxseed Conlinin protein: (SEQ ID NOs: 33-34) and/or to a Flaxseed Linin protein (SEQ ID NOs: 35-46).

Preferably, the plant derived protein or blend of plant derived proteins comprise a subunit or a protomer of a Globulin, preferably the subunit or the protomer has a molecular mass ranging from 30 kDa to 80 kDa and comprises from 1% to 80% of the total plant derived protein load in the composition.

Preferably, the subunit or the protomer of the Globulin has at least 50%, preferably at least 60%, preferably at least 70%, preferably at least 80%, preferably at least 85%, preferably at least 90%, preferably at least 95%, preferably at least 98% or even 100% amino acid identity to SEQ ID NOs: 1-10, 29-31, or 33-34.

Identity, or homology, percentages as mentioned herein in respect of the present invention are those that can be calculated with the GAP program, obtainable from GCG (Genetics Computer Group Inc., Madison, WI, USA). Alternatively, a manual alignment can be performed.

Surfactant System

The detergent composition of the present invention comprises a surfactant system. Preferably the detergent composition comprises from about 1 wt% to about 60 wt%, preferably from about 5 wt% to about 50 wt%, more preferably from about 8 wt% to 40%, by weight of the total composition of a surfactant system.

The surfactant system of the composition of the present invention comprises an anionic surfactant. Preferably, the surfactant system for the detergent composition of the present invention comprises from about 50 wt% to about 85 wt%, preferably from about 55 wt% to about 80 wt%, more preferably from about 60 wt% to about 75 wt% by weight of the surfactant system of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sulfate and mixtures thereof, and preferably wherein the alkyl alkoxy sulfate is an alkyl ethoxy sulfate. Preferred anionic surfactant is an alkyl ethoxy sulfate with an average ethoxylation degree of less than about 5, preferably less than about 3, more preferably less than about 2 and more than about 0.5 and preferably wherein the alkyl ethoxy sulfate has an average alkyl carbon chain length of from about 8 to about 16, preferably from about 12 to about 15, more preferably from about 12 to about 14. Preferably, the alkyl ethoxy sulfate has an average level of branching of from about 5% to about 40%, more preferably from about 10% to about 35%, and even more preferably from about 20% to about 30%.

The average alkoxylation degree is the mol average alkoxylation degree of all the components of the mixture (*i.e.,* mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching (\%)} = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

Preferably the surfactant system for the liquid detergent of the present invention will comprise from about 1 wt% to about 40 wt%, preferably from about 6 wt% to about 32 wt%, more preferably from about 8 wt% to about 25 wt% by weight of the total detergent composition of an anionic surfactant.

The surfactant system of the detergent composition of the present invention further comprises a primary co-surfactant system, wherein the primary co-surfactant system is preferably selected from the group consisting of amphoteric surfactant preferably amine oxide, zwitterionic surfactant preferably betaine, and mixtures thereof. Preferably, the surfactant system for the detergent composition of the present invention comprises from about 15 wt% to about 50 wt%, preferably from about 20 wt% to about 45 wt%, more preferably from about 25 wt% to about 40 wt%, by weight of the surfactant system of a primary co-surfactant system. Preferably the detergent composition comprises from about 0.01 wt% to about 20 wt%, preferably from about 0.2 wt% to about 15%wt, more preferably from about 0.5 wt% to about 10 wt% by weight of the detergent composition of an amphoteric and/or a zwitterionic surfactant, more preferably an amphoteric surfactant, even more preferably an amine oxide surfactant.

Preferably the primary co-surfactant system is an amphoteric surfactant. Preferably, the primary co-surfactant system is an amine oxide surfactant selected from the group consisting of linear or branched alkyl amine oxide, linear or branched alkyl amidopropyl amine oxide, and mixtures thereof, preferably linear alkyl dimethyl amine oxide, more preferably linear C10 alkyl dimethyl amine oxide, linear C12-C14 alkyl dimethyl amine oxides and mixtures thereof, most preferably C12-C14 alkyl dimethyl amine oxide. Preferably, the composition comprises anionic surfactant and amine oxide surfactant in a ratio of less than about 9:1, more preferably from about 5:1 to about 1:1, more preferably from about 4:1 to about 2:1, preferably from about 3:1 to about 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of CI-3 alkyl groups and CI-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least about 50 wt%, more preferably at least about 75 wt% to about 100 wt% of the mid-branched amine oxides for use herein. The amine oxide further comprises two moieties, independently selected from a CI-3 alkyl, a CI-3 hydroxyalkyl group, or a polyethylene oxide group containing an average of from about 1 to about 3 ethylene oxide groups. Preferably, the two moieties are selected from a CI-3 alkyl, more preferably both are selected as a C1 alkyl.

Preferably the amine oxide surfactant is a mixture of amine oxides comprising a low-cut amine oxide and a mid-cut amine oxide. The amine oxide of the composition of the invention then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable exampes of zwitterionic surfactants include betaines, such as alkyl betaines, alkylamidobetaine, amidazoliniumbetaine, sulfobetaine (INCI Sultaines) as well as the Phosphobetaine and preferably meets formula (I):

R1-[CO-X(CH2)n]x-N+(R2)(R3)-(CH2)m-[CH(OH)-CH2]y-Y- (I)

wherein:

R1 is a saturated or unsaturated C6-22 alkyl residue, preferably C8-18 alkyl residue, in particular a saturated C10-16 alkyl residue, for example a saturated C12-14 alkyl residue;
X is NH, NR4 with CI-4 Alkyl residue R4, O or S;
n is a number from 1 to 10, preferably 2 to 5, in particular 3;
x is 0 or 1, preferably 1;
R2 and R3 are independently a C1-4 alkyl residue, potentially hydroxy substituted such as a hydroxyethyl, preferably

a methyl;
m is a number from 1 to 4, in particular 1, 2 or 3;
y is 0 or 1; and
Y is COO, SO3, OPO(OR5)O or P(O)(OR5)O, whereby R5 is a hydrogen atom H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 has the same meaning as in formula I. Particularly preferred betaines are the Carbobetaine [wherein Y-=COO-], in particular the Carbobetaine of the formula (Ia) and (Ib), more preferred are the Alkylamidobetaine of the formula (Ib).

Examples of suitable betaines and sulfobetaine are the following [designated in accordance with INCI]: Almondamidopropyl of betaines, Apricotam idopropyl betaines, Avocadamidopropyl of betaines, Babassuamidopropyl of betaines, Behenam idopropyl betaines, Behenyl of betaines, betaines, Canolam idopropyl betaines, Capryl/Capram idopropyl betaines, Carnitine, Cetyl of betaines, Cocamidoethyl of betaines, Cocam idopropyl betaines, Cocam idopropyl Hydroxysultaine, Coco betaines, Coco Hydroxysultaine, Coco/Oleam idopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucam idopropyl Hydroxysultaine, Hydrogenated Tallow of betaines, Isostearam idopropyl betaines, Lauram idopropyl betaines, Lauryl of betaines, Lauryl Hydroxysultaine, Lauryl Sultaine, Milkam idopropyl betaines, Minkamidopropyl of betaines, Myristam idopropyl betaines, Myristyl of betaines, Oleam idopropyl betaines, Oleam idopropyl Hydroxysultaine, Oleyl of betaines, Olivamidopropyl of betaines, Palmam idopropyl betaines, Palm itam idopropyl betaines, Palmitoyl Carnitine, Palm Kernelam idopropyl betaines, Polytetrafluoroethylene Acetoxypropyl of betaines, Ricinoleam idopropyl betaines, Sesam idopropyl betaines, Soyam idopropyl betaines, Stearam idopropyl betaines, Stearyl of betaines, Tallowam idopropyl betaines, Tallowam idopropyl Hydroxysultaine, Tallow of betaines, Tallow Dihydroxyethyl of betaines, Undecylenam idopropyl betaines and Wheat Germam idopropyl betaines. A preferred betaine is, for example, Cocoamidopropylbetaine.

Preferably, the surfactant system of the composition of the present invention further comprises from about 1 wt% to about 25 wt%, preferably from about 1.25 wt% to about 20 wt%, more preferably from about 1.5 wt% to about 15 wt%, most preferably from about 1.5 wt% to about 5 wt%, by weight of the surfactant system of a secondary co-surfactant system preferably comprising a non-ionic surfactant. Preferably the non-ionic surfactant is an alkyl ethoxylated non-ionic surfactant, preferably comprising on average from about 9 to about 15 preferably from about 10 to about 14 carbon atoms in its alkyl chain and on average from about 5 to about 12, preferably from about 6 to about 10, most preferably from about 7 to about 8, units of ethylene oxide per mole of alcohol.

Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (*e.g.,* Simusol® surfactants from Seppic Corporation; and Glucopon® 600 CSUP, Glucopon® 650 EC,

Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1. Preferably the non-ionic surfactant is present from about 0.01 wt% to about 20 wt%, preferably from about 0.2 wt% to about 15 wt%, more preferably from about 0.5 wt% to about 10 wt% by weight of the total detergent composition.

Salt:

The composition of the present invention may optionally comprise from about 0.01% to about 3%, preferably from about 0.05% to about 2%, more preferably from about 0.2% to about 1.5%, or most preferably from about 0.5% to about 1%, by weight of the total composition of a salt, preferably a monovalent, divalent inorganic salt or a mixture thereof, preferably the divalent inorganic salt is chloride and/or sulfate salt of magnesium, calcium or zinc, most preferably magnesium chloride, sodium chloride or mixtures thereof. The composition alternatively or further comprises a multivalent metal cation in the amount of from about 0.01 wt% to about 2 wt%, preferably from about 0.1% to about 1%, more preferably from about 0.2% to about 0.8% by weight of the composition, preferably the multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Suitable carbohydrates include alpha or beta glucan with 1,3 and/or 1.4 and/or 1,6 linkage. Glucans can be modified especially with carboxyl sulfate, glycol ether of amino groups. Glucan can be extracted from dextran. Glucan with structure close to natural glucan such as schizophyllan, scleroglucan or paramylon are particularly preferred. Preferably, the composition comprises from about 0.005% to about 1% of the carbohydrates.

Hydrotrope

The composition of the present invention may optionally comprise from about 0.1% to about 10%, or preferably from about 0.5% to about 10%, more preferably from about 1% to about 6%, or most preferably from about 0.1% to about 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See *e.g.,* The Aqueous Phase Behaviour of Surfactants, Robert Laughlin, Academic Press, 1994, pp. 538-542. Preferably an isotropic composition is provided. Preferably the composition comprises 1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

Organic solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than about 50%, preferably from about 0.01% to about 25%, more preferably from about 0.1% to about 10%, or most preferably from about 0.5% to about 5%, by weight of the total composition of an organic solvent, preferably an alcohol, more preferably an ethanol, a polyalkyleneglycol, more preferably polypropyleneglycol, and mixtures thereof.

Amphiphilic Polymer

The composition of the present invention may further comprise from about 0.01% to about 5%, preferably from

about 0.05% to about 2%, more preferably from about 0.07% to about 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from about 100 to about 5,000, preferably from about 400 to about 2,000, more preferably from about 400 to about 1,000 Daltons and the alkoxylated polyethyleneimine polymer further comprising:

(i) one or two alkoxylation modifications per nitrogen atom by a polyalkoxylene chain having an average of about 1 to about 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of about 1 to about 50 alkoxy moieties per modification wherein the terminal alkoxy moiety is capped with hydrogen, a C1-C4 alkyl or mixtures thereof; or
(iii)a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

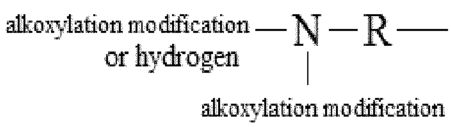
or
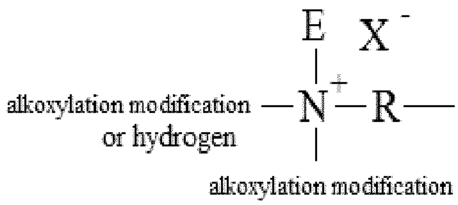

Also, for example, but not limited to, below is shown possible modifications to internal nitrogenatoms in the polyethyleneimine backbone where R represents an ethylene spacer and E represents a $C_1$-$C_4$ alkyl moiety and X- represents a suitable water soluble counterion.

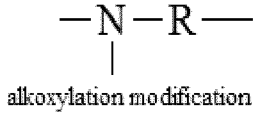
or
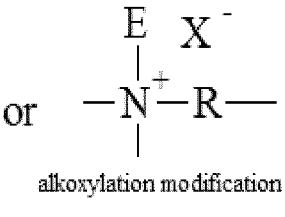

The alkoxylation modification of the polyethyleneimine backbone consists of the replacement of a hydrogen atom by a polyalkoxylene chain having an average of about 1 to about 50 alkoxy moieties, preferably from about 20 to about 45 alkoxy moieties, most preferably from about 30 to about 45 alkoxy moieties. The alkoxy moieties are selected from ethoxy (EO), propoxy (PO), butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from about 3 to about 30 and an average degree of propoxylation from about 1 to about 20, more preferably ethoxy/propoxy block moieties having an average degree of ethoxylation from about 20 to about 30 and an average degree of propoxylation from about 10 to about 20.

More preferably the ethoxy/propoxy block moieties have a relative ethoxy to propoxy unit ratio between about 3 to about 1 and about 1 to about 1, preferably between about 2 to about 1 and about 1 to about 1. Most preferably the polyalkoxylene chain is the ethoxy/propoxy block moieties wherein the propoxy moiety block is the terminal alkoxy moiety

block.

The modification may result in permanent quaternization of the polyethyleneimine backbone nitrogen atoms. The degree of permanent quaternization may be from 0% to about 30% of the polyethyleneimine backbone nitrogen atoms. It is preferred to have less than about 30% of the polyethyleneimine backbone nitrogen atoms permanently quaternized. Most preferably the degree of quaternization is about 0%.

A preferred polyethyleneimine has the general structure of Formula (II):

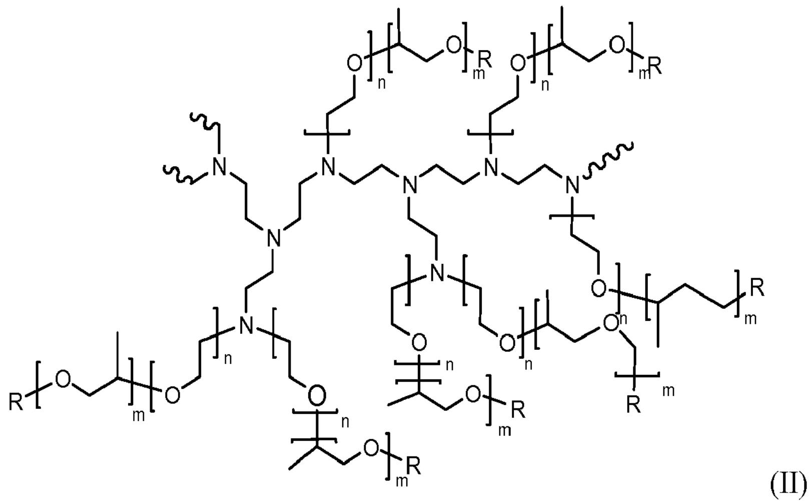

(II)

wherein the polyethyleneimine backbone has a weight average molecular weight of about 600, n of formula (II) has an average of about 10, m of formula (II) has an average of about 7 and R of formula (II) is selected from hydrogen, a $C_1$-$C_4$ alkyl and mixtures thereof, preferably hydrogen. The degree of permanent quaternization of formula (II) may be from 0% to about 22% of the polyethyleneimine backbone nitrogen atoms. The molecular weight of this polyethyleneimine preferably is between about 10,000 and about 15,000.

An alternative polyethyleneimine has the general structure of Formula (II) but wherein the polyethyleneimine backbone has a weight average molecular weight of about 600, n of Formula (II) has an average of about 24, m of Formula (II) has an average of about 16 and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms. The molecular weight of this polyethyleneimine preferably is between about 25,000 and about 30,000.

Most preferred polyethyleneimine has the general structure of Formula (II) wherein the polyethyleneimine backbone has a weight average molecular weight of about 600, n of Formula (II) has an average of about 24, m of Formula (II) has an average of about 16 and R of Formula (II) is hydrogen. The degree of permanent quaternization of Formula (II) is 0% of the polyethyleneimine backbone nitrogen atoms. The molecular weight of this polyethyleneimine preferably is about from about 25,000 to about 30,000, most preferably about 28,000.

These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

<u>EO-PO-EO tri-block co-polymer</u>

The composition of the present invention preferably comprises an EO-PO-EO tri-block co-polymer defined according to Formula (I): (EO)x(PO)y(EO)x, wherein EO represents ethylene oxide, and each x represents the number of EO units within the EO block. Each x is independently on average between 1 and 80, preferably between 3 and 60, more preferably between 5 and 50, most preferably between 5 and 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y is on average between 1 and 60, preferably between 10 and 55, more preferably between 10 and 50, more preferably between 15 and 48. The tri-block co-polymers according to the invention are preferably present in the composition at a level of from about 0.1 wt% to about 10 wt%, preferably from about 0.5 wt% to about 7.5 wt%, more preferably from about 1 wt% to about 5 wt%, by weight of the total composition.

Chelant

The detergent composition herein can comprise a chelant at a level of from about 0.1% to about 20%, preferably from about 0.2% to about 5%, more preferably from about 0.2% to about 3% by weight of total composition.
As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.
Preferably, the composition of the present invention comprises one or more chelant, preferably selected from the group comprising carboxylate chelants, amino carboxylate chelants, amino phosphonate chelants such as MGDA (methylglycine-N,N-diacetic acid), GLDA (glutamic-N,N- diacetic acid), and mixtures thereof.
Suitable chelating agents can be selected from the group consisting of amino carboxylates, amino phosphonates, polycarboxylate chelating agents and mixtures thereof.
Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Adjunct Ingredients

The cleaning composition herein may optionally comprise a number of other adjunct ingredients such as builders (*e.g.,* preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.,* salt such as NaCl, and other mono-, di- and trivalent salts) and pH adjusters and buffering means (*e.g.,* carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

In another aspect, the invention is directed to a method of manually washing dishware comprising the steps of delivering a detergent composition of the invention into a volume of water to form a wash solution and immersing the dishware in the solution. As such, the composition herein will be applied in its diluted form to the dishware. Soiled surfaces *e.g.* dishes are contacted with an effective amount, typically from about 0.5 mL to about 20 mL (per 25 dishes being treated), preferably from about 3 mL to about 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from about 0.01 mL to about 150 mL, preferably from about 3 mL to about 40 mL of a liquid detergent composition of the invention is combined with from about 2,000 mL to about 20,000 mL, more typically from about 5,000 mL to about 15,000 mL of water in a sink having a volumetric capacity in the range of from about 1,000 mL to about 20,000 mL, more typically from about 5,000 mL to about 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from 1 to 10 seconds, although the actual time will vary with each application and user. The contacting of cloth, sponge, or similar article to the surface is preferably accompanied by a concurrent scrubbing of the surface.
In another aspect, the invention is directed to a method of manually washing dishware with the composition of the present invention. The method comprises the steps of: i) delivering a composition of the present invention onto the dishware or a cleaning implement; ii) cleaning the dishware with the composition in the presence of water; and iii)

optionally, rinsing the dishware. The delivering step is preferably either directly onto the dishware surface or onto a cleaning implement, *i.e.,* in a neat form. The cleaning device or implement is preferably wet before or after the composition is delivered to it. Especially good grease removal has been found when the composition is used in neat form.

In another aspect, the invention is directed to a method of manually washing soiled articles comprising contacting a detergent composition of the invention with a surface, and wherein the composition modifies the hydrophobicity of the surface as a result of the contacting step.

Another aspect of the present invention is directed to a method of promoting suds longevity or grease emulsification in a washing process for washing soiled articles, preferably dishware. The method comprises the steps of: a) delivering a detergent composition of the invention to a volume of water to form a wash liquor; and b) immersing the soiled articles into said wash liquor. Preferably, the plant derived protein or blend of plant derived proteins according to the invention is present at a concentration of about 0.005 ppm to about 60 ppm, preferably at a concentration of about 0.02 ppm to about 12 ppm, in an aqueous wash liquor during the washing process.

Another aspect of the present invention is use, in a hand dishwashing detergent composition, of a combination of: i) a plant derived protein or blend of plant derived proteins selected from the group consisting of a Canola protein, a Hemp protein, a Flaxseed protein, and mixtures thereof, most preferably the plant seed protein is a Canola protein; and ii) a surfactant system comprising an anionic surfactant and a primary co-surfactant selected from the group consisting of amphoteric surfactant preferably an amine oxide surfactant, a zwitterionic surfactant preferably a betaine surfactant, and mixtures thereof, preferably the primary co-surfactant is amine oxide, wherein the weight ratio of anionic surfactant to the primary co-surfactant is less than about 9:1, more preferably from about 5:1 to about 1:1, more preferably from about 4:1 to about 2:1; to provide enhanced suds boosting and/or increased suds longevity in an aqueous wash liquor during a hand dish washing process.

TEST METHODS

The following assay set forth must be used in order that the invention described and claimed herein may be more fully understood.

Test Method 1: Glass Vial Suds Mileage

The method measures the evolution of suds volume over time generated by a certain solution of test detergent composition in the presence of a greasy soil, *e.g.,* olive oil. The following factors may affect the measurement results and therefore should be controlled carefully: (a) concentration of the test detergent composition; (b) hardness of the water; (c) water temperature; (d) speed of stirring; and (e) speed and number of the shaking. Following steps are followed to obtain the suds measurements for each test detergent composition:

1. Test solutions are prepared by subsequently adding aliquots into 40 mL glass vials (dimensions: 95 mm Height x 27.5 mm Diameter), preferably graduated vials at room temperature, of: a) 10 g of an aqueous detergent solution at 0.11% detergent concentration and water hardness (15 °dh), and b) 0.11 g of olive oil (Bertolli®, Extra Virgin Olive Oil). The test detergent contains 2% of the plant derived protein and is compared with a nil-plant derived protein detergent.
2. The test solutions are mixed in the closed test vials by stirring at room temperature for 2 minutes at 500 RPM on a magnetic stirring plate (IKA, model # RTC B S001; VWR magnetic stirrer, catalog # 58949-012), followed by manually shaking for 20 seconds with an upwards downwards movement (about 2 up and down cycles per second, +/- 30 cm up and 30 cm down) and the initial suds heights (HI) are recorded with a ruler. HI is a measurement of the suds height.
3. Following the shaking, the test solutions in the closed vials are further stirred at 500 RPM on the magnetic stirring plate for 60 minutes inside a water bath at 35°C to maintain a constant temperature. The samples are then shaken manually for another 20 seconds as described above. The final suds heights (H2) are recorded.
4. The Suds Stability Index (SSI) of an individual sample is expressed as (H2/H1)*100. Protein solutions that produce larger suds heights (HI and H2), preferably combined with lower drops in suds height between HI and H2, are more desirable, *i.e.*, high HI and high suds stability index. A Protein Impact Index (PII) can be further calculated by cross-comparing the Suds Stability Index of the protein comprising sample versus a reference sample single variably lacking the protein, *i.e.* (SSI (protein sample)/SSI (nil protein reference))*100.

EXAMPLES

The following examples are provided to further illustrate the present invention and are not to be construed as limitations of the present invention, as many variations of the present invention are possible without departing from its

spirit or scope.

Example 1: Plant Derived Protein Detergent Compositions Impact on Suds Mileage

The ability to maintain suds mileage in the presence of greasy soil, *i.e.* olive oil, is assessed for test detergent compositions with or without the plant derived protein. The compositions are summarized in Table 1. Composition Ex. 4 is a plant derived protein containing test detergent compositions according to the present invention, made with surfactant system comprising Alkyl(C12/C13)-0.6 ethoxylated sulfate and Alkyl(C12/C14)-dimethyl amine oxide in 4:1 weight ratio, in the presence of 2% Hemp protein. Composition Ex. 3 is a reference composition containing the same surfactant system as in Composition Ex. 4 in the absence of the Hemp protein. Composition Ex. 2 is a test composition containing the Alkyl(C12/C13)-0.6 ethoxylated sulfate minus the amine oxide in the presence of the Hemp protein. Composition Ex. 1 is a reference composition to Composition Ex. 2, and Composition Ex. 1 contains Alkyl(C12/C13)-0.6 ethoxylated sulfate minus the amine oxide in the absence of the Hemp protein. The compositions are produced through standard mixing of the components described in Table 1.

Table 1 - Detergent Compositions

| Ingredients | Reference Comp. Ex. 1 | Test Comp. Ex. 2 | Reference Comp. Ex. 3 | Test Comp. Ex. 4 |
|---|---|---|---|---|
| Sodium alkyl ethoxy sulfate (C1213EO0.6S) | 26.25% | 26.25% | 21% | 21% |
| n-C 12-14 Di Methyl Amine Oxide | - | - | 5.25% | 5.25% |
| Lutensol® XP80 (non-ionic surfactant supplied by BASF) | 1% | 1% | 1% | 1% |
| Sodium Chloride | 0.7% | 0.7% | 0.7% | 0.7% |
| Poly Propylene Glycol (MW 2000) | 0.7% | 0.7% | 0.7% | 0.7% |
| Ethanol | 2% | 2% | 2% | 2% |
| Sodium Hydroxide | 0.2% | 0.2% | 0.2% | 0.2% |
| Hemp protein* | - | 2% | - | 2% |
| Minors (perfume, preservative, dye) + water | To 100 % | To 100 % | To 100 % | To 100 % |
| pH (@ 0.12% solution) | 8.35 | 8.35 | 8.35 | 8.35 |
| * Hemp protein from Myprotein (UK). | | | | |

The compositions Ex. 1-4 are tested for the suds volume (*i.e.,* suds height) and suds stability and the data (not shown) are recorded. The Suds Stability Index ("SSI") for each of the compositions is calculated (not shown) according to Test Method 1. A Protein Impact Index (PII), which is a unitless number that indicates the relative impact of the plant derived protein on the suds mileage of a test composition as compared to a reference composition which is missing the protein, is calculated. The PII is calculated by dividing the SSI for the test composition containing the plant derived protein by the SSI for the reference composition minus the plant derived protein, followed by multiplying the quotient by 100. The higher the PII, the better the suds mileage performance of the test composition.

The PPI results for test Composition Ex. 4 vs. reference Composition Ex. 3 are summarized in Table 2, and the PPI results for test Composition Ex. 2 vs. reference Composition Ex. 1 are summarized in Table 3.

Table 2 - Performance on Suds Stability of Composition containing Hemp Protein with AES/AO Surfactant System

| Composition | Protein Impact Index (PII) |
|---|---|
| Reference Comp. Ex. 3 | 100 |
| Test Comp. Ex. 4 | 112* |
| * vs. Reference Comp. Ex. 3. | |

It is clear from the results in Table 2 that the addition of the Hemp protein to a surfactant system (AES/AO)

within the scope of the present invention leads to an enhanced increase of suds duration, particularly in the presence of greasy soil, as evidenced by a PII of 112.

Table 3 - Performance on Suds Stability of Composition containing Hemp Protein with AES Surfactant System

| Composition | Protein Impact Index |
|---|---|
| Reference Comp. Ex. 1 | 100 |
| Test Comp. Ex. 2 | 81* |
| * vs. Reference Comp. Ex. 1. | |

The data in Table 3 shows that the addition of the Hemp protein according to the invention to a surfactant system (AES and no primary co-surfactant) outside the scope of the invention results in a suds mileage represented by PII of 81. This suds mileage is a noticeable drop when compared to the test Comp. Ex. 4 with PII of 112. Therefore, the enhanced increased suds mileage performance of the composition comprising the plant derived protein with the specific surfactant system according to the present invention is unexpected and a synergy between the specific surfactant system and the protein of the invention is observed.

All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

**1.** A detergent composition comprising:

a) from 1 wt% to 60 wt%, preferably from 5 wt% to 50 wt%, more preferably from 8 wt% to 45 wt%, even more preferably from 15 wt% to 40 wt%, by weight of the composition of a surfactant system, wherein the surfactant system comprises:

i) an anionic surfactant, preferably the anionic surfactant is selected from the group consisting of alkyl sulfate, alkyl alkoxy sulfate, and mixtures thereof; and
ii) a primary co-surfactant selected from the group consisting of an amphoteric surfactant preferably an amine oxide surfactant, a zwitterionic surfactant preferably a betaine surfactant, and mixtures thereof;

wherein the weight ratio of the anionic surfactant to the primary co-surfactant is less than 9:1, more preferably from 5:1 to 1:1, more preferably from 4:1 to 2:1; and
b) from 0.1 wt% to 10 wt%, preferably from 0.5 wt% to 5 wt%, by weight of the composition of a plant derived protein or blend of plant derived proteins derived from a plant seed family selected from the group consisting of Brassica oleracea, Brassica rapa, Raphanus sativus, Armoracia rusticana, Brassica rapa oleifera, Brassica campestris, Brassica juncea, Brassica napus, Boehmeria cylindrica, Crotalaria juncea, Corchorus olitorius, Hibiscus cannabinus, Musa textilis, Phormium tenax, Hibiscus sabdariffa, Agave sisalana, Cannabis indica,

Cannabis ruderalis, Cannabis sativa, Linum alatum, Linum album, Linum arenicola, Linum aristatum, Linum australe, Linum austriacum, Linum berlandieri, Linum bienne, Linum campanulatum, Linum carteri, Linum catharticum, Linum compactum, Linum cratericola, Linum dolomiticum, Linum elongatum, Linum flavum, Linum floridanum, Linum grandiflorum, Linum hirsutum, Linum hudsonioides, Linum imbricatum, Linum intercursum, Linum kingii, Linum leoni, Linum lewisii, Linum lundellii, Linum macrocarpum, Linum marginale, Linum medium, Linum monogynum, Linum narbonense, Linum neomexicanum, Linum perenne, Linum pratense, Linum puberulum, Linum pubescens, Linum rigidum, Linum rupestre, Linum schiedeanum, Linum striatum, Linum subteres, Linum suffruticosum, Linum sulcatum, Linum tenuifolium, Linum trigynum, Linum vernal, Linum virginianum, Linum westii, and Linum usitatissimum, preferably Brassica juncea, Brassica napus, Cannabis sativa, and Linum usitatissimum, preferably the plant seed protein is selected from the group consisting of a Canola protein, a Hemp protein, a Flaxseed protein, and mixtures thereof, most preferably the plant seed protein is a Canola protein;
wherein the detergent composition is a hand dishwashing detergent composition.

**2.** The composition according to claim 1, wherein:

a. the Canola protein is a Canola protein isolate comprising a Canola Albumin preferably Napin, Canola Globulin preferably Cruciferin, or mixtures thereof, preferably the Canola protein isolate is obtained by extraction from a Canola oil seed meal;
b. the Hemp protein is a Hemp protein isolate comprising a Hemp Globulin preferably Edestin, a Hemp Albumin, or mixtures thereof, preferably the Hemp protein isolate is obtained by extraction from a hemp seed meal; and
c. the Flaxseed protein is a Flaxseed protein isolate comprising Flaxseed Albumin preferably Conlinin (2S), Flaxseed Globulin preferably Linin (12S), or mixtures thereof, preferably the Flaxseed protein isolate is obtained by extraction from a Flaxseed meal.

**3.** The composition according to any preceding claims, wherein the Canola protein is a Canola protein isolate comprising:

a. a first Canola protein isolate component comprising a Canola Albumin (1.6S, 1.7S, 2S) protein, preferably from 20 wt% to 95 wt%, more preferably from 50 wt% to 95 wt%, by weight of the total Canola protein level in the composition;
b. a second Canola protein isolate component comprising a Canola Globulin (7S,8S) protein, preferably from 5 wt% to 80 wt%, more preferably from 5 wt% to 50 wt%, by weight of the total Canola protein level in the composition; and
c. a third Canola protein isolate component comprising a Canola Globulin (11S, 12S) protein, preferably from 0 wt% to 30 wt%, more preferably from 0 wt% to 2 wt%, by weight of the total Canola protein level in the composition.

**4.** The composition according to any preceding claims, wherein the Hemp protein is Hemp protein isolate comprising:

a. a first Hemp protein isolate component comprising a Hemp Albumin protein, preferably from 60 wt% to 95 wt%, more preferably from 70 wt% to 95 wt%, by weight of the total Hemp protein level in the composition; and
b. a second Hemp protein isolate component comprising a Hemp Edestin protein, preferably from 5 wt% to 40 wt%, more preferably from 5 wt% to 30 wt%, by weight of the total Hemp protein level in the composition.

**5.** The composition according to any preceding claims, wherein the Flaxseed protein is a Flaxseed protein isolate comprising:

c. a first Flaxseed protein isolate component comprising a Conlinin (2S) protein, preferably from 0 wt% to 35 wt% by weight of the total Flaxseed protein level in the composition; and
d. a second Flaxseed protein isolate component comprising a Linin (12S) protein, preferably from 65 wt% to 100 wt% by weight of the total Flaxseed protein level in the composition.

**6.** The composition according to any preceding claims, wherein:

a. the Canola protein has at least 50%, preferably at least 60%, preferably at least 70%, preferably at least 80%, preferably at least 85%, preferably at least 90%, preferably at least 95%, preferably at least 98% or even 100% amino acid identity to a Canola Cruciferin protein: (SEQ ID NOs: 1-10) and/or to a Canola Napin protein

(SEQ ID NOs: 11-28);
b. the Hemp protein has at least 50%, preferably at least 60%, preferably at least 70%, preferably at least 80%, preferably at least 85%, preferably at least 90%, preferably at least 95%, preferably at least 98% or even 100% amino acid identity to a Hemp Edestin protein: (SEQ ID NOs: 29-31) and/or to a Hemp Albumin protein (SEQ ID NO: 32); and
c. the Flaxseed protein has at least 50%, preferably at least 60%, preferably at least 70%, preferably at least 80%, preferably at least 85%, preferably at least 90%, preferably at least 95%, preferably at least 98% or even 100% amino acid identity to a Flaxseed Conlinin protein: (SEQ ID NOs: 33-34) and/or to a Flaxseed Linin protein (SEQ ID NOs: 35-46).

**7.** The composition according to any preceding claims, wherein the plant derived protein or blend of plant derived proteins comprise a subunit or a protomer of a Globulin, preferably the subunit or the protomer has a molecular weight ranging from 30 kDa to 80 kDa and comprises from 1% to 80% of the total plant derived protein load in the composition.

**8.** The composition according to claim 7, wherein the subunit or the protomer of the Globulin has at least 50%, preferably at least 60%, preferably at least 70%, preferably at least 80%, preferably at least 85%, preferably at least 90%, preferably at least 95%, preferably at least 98% or even 100% amino acid identity to SEQ ID NOs: 1-10, 29-31, or 33-34.

**9.** The composition according to any preceding claims, wherein the composition comprises a phytic acid content of 0.5 wt% or less, preferably 0.2 wt% or less, preferably 0.1 wt% or less, preferably 0.01 wt% or less by weight of the composition, most preferably the composition is essentially free of the phytic acid.

**10.** The composition according to any preceding claims, wherein the composition comprises a protein-bound carbohydrate content of 2 wt% or less, preferably 1 wt% or less, preferably 0.5 wt% or less, preferably 0.1 wt% or less, preferably 0.01 wt% or less by weight of the composition, most preferably the composition is essentially free of the protein-bound carbohydrate.

**11.** The composition according to any preceding claims, wherein: a) the surfactant system comprises:

i) from 50 wt% to 85 wt%, preferably from 55 wt% to 80 wt%, more preferably from 60 wt% to 75 wt%, by weight of the surfactant system of the anionic surfactant; and
ii) from 15 wt% to 50 wt%, preferably from 20 wt% to 45 wt%, more preferably from 25 wt% to 40 wt%, by weight of the surfactant system of the primary co-surfactant.

**12.** The composition according to any preceding claims, wherein:

i) the anionic surfactant is an alkyl ethoxy sulfate, preferably an alkyl ethoxy sulfate with an average degree of ethoxylation of less than 5, preferably less than 3, more preferably less than 2 and more than 0.5, and preferably with a weight average level of branching of from 5% to 60%, preferably from 10% to 55%, more preferably from 15% to 50%, even more preferably from 20% to 45%, most preferably from 25% to 45%, and preferably wherein the alkyl ethoxy sulfate has an average alkyl carbon chain length of from 8 to 16, preferably from 12 to 15, more preferably from 12 to 14; and
ii) the primary co-surfactant is an amine oxide selected from the group consisting of linear or branched alkyl amine oxide, linear or branched alkyl amidopropyl amine oxide, and mixtures thereof, preferably linear alkyl dimethyl amine oxide, more preferably linear C10 alkyl dimethyl amine oxide, linear C12-C14 alkyl dimethyl amine oxides and mixtures thereof, most preferably C12-C14 alkyl dimethyl amine oxide.

**13.** The composition according to any preceding claims, further comprising from 1 wt% to 25 wt%, preferably from 1.25 wt% to 20 wt%, more preferably from 1.5 wt% to 15 wt%, most preferably from 1.5 wt% to 5 wt%, by weight of the surfactant system of a non-ionic surfactant, preferably an alkyl ethoxylated non-ionic surfactant, preferably comprising on average from 9 to 15 preferably from 10 to 14 carbon atoms in its alkyl chain and on average from 5 to 12, preferably from 6 to 10, most preferably from 7 to 8, units of ethylene oxide per mole of alcohol.

**14.** A method of manually washing dishware comprising the steps of delivering a composition according to any of the preceding claims to a volume of water to form a wash liquor and immersing the dishware in the wash liquor, or delivering a composition according to any of the preceding claims directly onto the dishware or cleaning implement

and using the cleaning implement to clean the dishware.

**15.** Use of a detergent composition according to any of claims 1 to 13 to provide increased suds longevity of the composition and/or facilitate reduction of surfactants in the composition.

Europäisches Patentamt
European Patent Office
Office européen des brevets

# EUROPEAN SEARCH REPORT

Application Number
EP 18 16 1358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/327774 A1 (LANT NEIL JOSEPH [GB] ET AL) 16 November 2017 (2017-11-16) * paragraphs [0001] - [0004], [0008], [0045] - [0079], [0095] - [0096]; claims; examples 1,2 * | 1-15 | INV. C11D1/94 C07K14/415 C11D3/382 C11D11/00 |
| Y | US 2018/016522 A1 (LANT NEIL JOSEPH [GB] ET AL) 18 January 2018 (2018-01-18) * paragraphs [0002] - [0014], [0024], [0042] - [0080], [0118] - [0122]; claims; example 1 * | 1-15 | |
| X | US 2010/196295 A1 (ALVARADO EMMA [US] ET AL) 5 August 2010 (2010-08-05) * paragraphs [0041], [0045] - [0046]; example 1 * | 1,2, 5-13,15 | |
| Y,D | WO 2014/019074 A1 (BURCON NUTRASCIENCE MB CORP [CA]) 6 February 2014 (2014-02-06) * paragraph [0015]; example 2 * | 1,2,4, 6-15 | TECHNICAL FIELDS SEARCHED (IPC) |
| Y,D | WO 2005/012342 A1 (BURCON NUTRASCIENCE MB CORP [CA]; GREEN BRENT E [CA]; MILANOVA RADKA [] 10 February 2005 (2005-02-10) * paragraphs [0011], [0014]; examples 4,7 * | 1,2,5-15 | C11D C07K |

-/--

The present search report has been drawn up for all claims

1

| Place of search | Date of completion of the search | Examiner |
|---|---|---|
| The Hague | 24 August 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 16 1358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SIONG H. TAN ET AL: "Canola Proteins for Human Consumption: Extraction, Profile, and Functional Properties", JOURNAL OF FOOD SCIENCE, vol. 76, no. 1, 1 January 2011 (2011-01-01), pages R16-R28, XP055221881, US ISSN: 0022-1147, DOI: 10.1111/j.1750-3841.2010.01930.x * page R16, left-hand column, paragraph 1 * * page R25, right-hand column, line 4 - page R26, left-hand column, paragraph 5 * * page R21, right-hand column, last paragraph - page R22, left-hand column, paragraph 4 * ----- | 1-3,6-15 | |

TECHNICAL FIELDS SEARCHED (IPC)

The present search report has been drawn up for all claims

1

| Place of search | Date of completion of the search | Examiner |
|---|---|---|
| The Hague | 24 August 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 18 16 1358

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2018

| Patent document cited in search report | Publication date | Patent family member(s) | Publication date |
|---|---|---|---|
| US 2017327774 A1 | 16-11-2017 | EP 3243894 A1 | 15-11-2017 |
| | | US 2017327774 A1 | 16-11-2017 |
| | | WO 2017196813 A1 | 16-11-2017 |
| US 2018016522 A1 | 18-01-2018 | EP 3269729 A1 | 17-01-2018 |
| | | US 2018016522 A1 | 18-01-2018 |
| | | WO 2018013395 A1 | 18-01-2018 |
| US 2010196295 A1 | 05-08-2010 | AU 2008363812 A1 | 14-05-2010 |
| | | BR PI0823238 A2 | 16-06-2015 |
| | | CA 2742587 A1 | 14-05-2010 |
| | | CN 102271656 A | 07-12-2011 |
| | | EP 2346489 A1 | 27-07-2011 |
| | | KR 20110091004 A | 10-08-2011 |
| | | MX 336184 B | 06-01-2016 |
| | | RU 2011122817 A | 20-12-2012 |
| | | US 2010196295 A1 | 05-08-2010 |
| | | WO 2010053488 A1 | 14-05-2010 |
| WO 2014019074 A1 | 06-02-2014 | AU 2013299297 A1 | 12-03-2015 |
| | | BR 112015001964 A2 | 04-07-2017 |
| | | CA 2880097 A1 | 06-02-2014 |
| | | CN 104519750 A | 15-04-2015 |
| | | EP 2879510 A1 | 10-06-2015 |
| | | JP 2015523090 A | 13-08-2015 |
| | | KR 20150036790 A | 07-04-2015 |
| | | NZ 705218 A | 23-03-2018 |
| | | RU 2015106890 A | 27-09-2016 |
| | | TW 201410156 A | 16-03-2014 |
| | | US 2014037824 A1 | 06-02-2014 |
| | | US 2015173395 A1 | 25-06-2015 |
| | | US 2018213818 A1 | 02-08-2018 |
| | | WO 2014019074 A1 | 06-02-2014 |
| | | ZA 201501372 B | 27-01-2016 |
| WO 2005012342 A1 | 10-02-2005 | AT 508644 T | 15-05-2011 |
| | | AU 2004261327 A1 | 10-02-2005 |
| | | BR PI0413240 A | 03-10-2006 |
| | | CA 2533823 A1 | 10-02-2005 |
| | | EP 1654275 A1 | 10-05-2006 |
| | | HK 1096410 A1 | 09-08-2013 |
| | | JP 4330627 B2 | 16-09-2009 |
| | | JP 2007520442 A | 26-07-2007 |
| | | KR 20060069435 A | 21-06-2006 |
| | | MX PA06001312 A | 23-08-2006 |
| | | NZ 545339 A | 26-06-2009 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 16 1358

This annex lists the patent family members relating to the patent documents cited in the above-mentioned European search report. The members are as contained in the European Patent Office EDP file on
The European Patent Office is in no way liable for these particulars which are merely given for the purpose of information.

24-08-2018

| Patent document cited in search report | Publication date | Patent family member(s) | Publication date |
| --- | --- | --- | --- |
| | | US 2005058756 A1 | 17-03-2005 |
| | | US 2007191593 A1 | 16-08-2007 |
| | | WO 2005012342 A1 | 10-02-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030125526 A1
- US 20040254353 A1
- WO 02089597 A
- WO 200918660 A1
- WO 2014419074 PCT
- WO 2014145057 PCT
- WO 2014419074 A
- WO 200512342 A
- WO 201053488 A1
- WO 03043439 PCT
- WO 201419074 A
- WO 2014145057 A
- US 3915903 A
- WO 2007135645 A


### Non-patent literature cited in the description


- **KINSELLA, JE.** *Food Chem.,* 1981, vol. 7 (4), 273-288
- **TAN, S. H. et al.** *J. Food Sci.,* January 2011, vol. 76 (1), R16-R28
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **WANADUNDARA et al.** *OCL,* 2016, vol. 23 (4), D407
- **TANDANG-SILVAS et al.** *Biochem. Biophys. Acta (BBA) - Proteins and Proteomics,* 2010, vol. 1804, 1432-1442
- **ROBERT LAUGHLIN.** The Aqueous Phase Behaviour of Surfactants. Academic Press, 1994, 538-542

SEQUENCE LISTING

<110> The Procter & Gamble Company

<120> HAND DISHWASHING DETERGENT COMPOSITION

<130> CM04957F

<140> 18161358.9
<141> 2018-03-13

<160> 46

<170> PatentIn version 3.5

<210> 1
<211> 465
<212> PRT
<213> NONE

<400> 1

```
Met Gly Pro Thr Ser Leu Leu Ser Phe Phe Phe Thr Phe Leu Thr Leu
1               5                   10                  15

Phe His Gly Phe Thr Ala Gln Gln Trp Pro Asn Glu Cys Gln Leu Asp
            20                  25                  30

Gln Leu Asn Ala Leu Glu Pro Ser Gln Ile Ile Lys Ser Glu Gly Gly
        35                  40                  45

Arg Ile Glu Val Trp Asp His His Ala Pro Gln Leu Arg Cys Ser Gly
    50                  55                  60

Phe Ala Phe Glu Arg Phe Val Ile Glu Pro Gln Gly Leu Tyr Leu Pro
65                  70                  75                  80

Thr Phe Leu Asn Ala Gly Lys Leu Thr Phe Val Val His Gly His Ala
                85                  90                  95

Leu Met Gly Lys Val Thr Pro Gly Cys Ala Glu Thr Phe Asn Asp Ser
            100                 105                 110

Pro Val Phe Gly Gln Gly Gln Gly Gln Glu Gln Gly Gln Gly Gln Gly
        115                 120                 125

Gln Gly Gln Gly Gln Gly Phe Arg Asp Met His Gln Lys Val Glu His
    130                 135                 140

Leu Arg Ser Gly Asp Thr Ile Ala Thr Pro Pro Gly Val Ala Gln Trp
145                 150                 155                 160

Phe Tyr Asn Asn Gly Asn Glu Pro Leu Ile Leu Val Ala Ala Ala Asp
```

165 170 175

Ile Ala Asn Asn Leu Asn Gln Leu Asp Arg Asn Leu Arg Pro Phe Leu
180 185 190

Leu Ala Gly Asn Asn Pro Gln Gly Gln Gln Trp Leu Gln Gly Arg Gln
195 200 205

Gln Gln Lys Gln Asn Asn Ile Phe Asn Gly Phe Ala Pro Gln Ile Leu
210 215 220

Ala Gln Ala Phe Lys Ile Ser Val Glu Thr Ala Gln Lys Leu Gln Asn
225 230 235 240

Gln Gln Val Asn Arg Gly Asn Ile Val Lys Val Gln Gly Gln Phe Gly
245 250 255

Val Ile Arg Pro Pro Leu Arg Gln Gly Gln Gly Gly Gln Gln Pro Gln
260 265 270

Glu Glu Gly Asn Gly Leu Glu Glu Thr Leu Cys Thr Met Arg Cys Thr
275 280 285

Glu Asn Leu Asp Asp Pro Ser Ser Ala Asp Val Tyr Lys Pro Ser Leu
290 295 300

Gly Tyr Ile Ser Thr Leu Asn Ser Tyr Asn Leu Pro Ile Leu Arg Phe
305 310 315 320

Leu Arg Leu Ser Ala Leu Arg Gly Ser Ile His Asn Asn Ala Met Val
325 330 335

Leu Pro Gln Trp Asn Val Asn Ala Asn Ala Ala Leu Tyr Val Thr Lys
340 345 350

Gly Lys Ala His Ile Gln Asn Val Asn Asp Asn Gly Gln Arg Val Phe
355 360 365

Asp Gln Glu Ile Ser Lys Gly Gln Leu Leu Val Val Pro Gln Gly Phe
370 375 380

Ala Val Val Lys Arg Ala Thr Ser Gln Gln Phe Gln Trp Ile Glu Phe
385 390 395 400

Lys Ser Asn Asp Asn Ala Gln Ile Asn Thr Leu Ala Gly Arg Thr Ser
405 410 415

Val Met Arg Gly Leu Pro Leu Glu Val Ile Ser Asn Gly Tyr Gln Ile
420 425 430

Ser Pro Gln Glu Ala Arg Ser Val Lys Phe Ser Thr Leu Glu Thr Thr
435 440 445

Leu Thr Gln Ser Ser Gly Pro Met Gly Tyr Gly Met Pro Arg Val Glu
450 455 460

Ala
465

<210> 2
<211> 488
<212> PRT
<213> NONE

<400> 2

Met Ala Arg Leu Ser Ser Leu Leu Ser Phe Ser Leu Ala Leu Leu Thr
1 5 10 15

Phe Leu His Gly Ser Thr Ala Gln Gln Phe Pro Asn Glu Cys Gln Leu
20 25 30

Asp Gln Leu Asn Ala Leu Glu Pro Ser His Val Leu Lys Ala Glu Ala
35 40 45

Gly Arg Ile Glu Val Trp Asp His His Ala Pro Gln Leu Arg Cys Ser
50 55 60

Gly Val Ser Phe Val Arg Tyr Ile Ile Glu Ser Lys Gly Leu Tyr Leu
65 70 75 80

Pro Ser Phe Phe Ser Thr Ala Arg Leu Ser Phe Val Ala Lys Gly Glu
85 90 95

Gly Leu Met Gly Arg Val Val Leu Cys Ala Glu Thr Phe Gln Asp Ser
100 105 110

Ser Val Phe Gln Pro Ser Gly Gly Ser Pro Phe Gly Glu Gly Gln Gly
115 120 125

Gln Gly Gln Gln Gly Gln Gly Gln Gly His Gln Gly Gln Gly Gln Gly
130 135 140

Gln Gln Gly Gln Gln Gly Gln Gln Gly Gln Gln Ser Gln Gly Gln Gly
145 150 155 160

```
Phe Arg Asp Met His Gln Lys Val Glu His Ile Arg Thr Gly Asp Thr
                165                 170                 175

Ile Ala Thr His Pro Gly Val Ala Gln Trp Phe Tyr Asn Asp Gly Asn
            180                 185                 190

Gln Pro Leu Val Ile Val Ser Val Leu Asp Leu Ala Ser His Gln Asn
        195                 200                 205

Gln Leu Asp Arg Asn Pro Arg Pro Phe Tyr Leu Ala Gly Asn Asn Pro
    210                 215                 220

Gln Gly Gln Val Trp Ile Glu Gly Arg Glu Gln Gln Pro Gln Lys Asn
225                 230                 235                 240

Ile Leu Asn Gly Phe Thr Pro Glu Val Leu Ala Lys Ala Phe Lys Ile
                245                 250                 255

Asp Val Arg Thr Ala Gln Gln Leu Gln Asn Gln Gln Asp Asn Arg Gly
            260                 265                 270

Asn Ile Ile Arg Val Gln Gly Pro Phe Ser Val Ile Arg Pro Pro Leu
        275                 280                 285

Arg Ser Gln Arg Pro Gln Glu Glu Val Asn Gly Leu Glu Glu Thr Ile
    290                 295                 300

Cys Ser Ala Arg Cys Thr Asp Asn Leu Asp Asp Pro Ser Asn Ala Asp
305                 310                 315                 320

Val Tyr Lys Pro Gln Leu Gly Tyr Ile Ser Thr Leu Asn Ser Tyr Asp
                325                 330                 335

Leu Pro Ile Leu Arg Phe Leu Arg Leu Ser Ala Leu Arg Gly Ser Ile
            340                 345                 350

Arg Gln Asn Ala Met Val Leu Pro Gln Trp Asn Ala Asn Ala Asn Ala
        355                 360                 365

Val Leu Tyr Val Thr Asp Gly Glu Ala His Val Gln Val Val Asn Asp
    370                 375                 380

Asn Gly Asp Arg Val Phe Asp Gly Gln Val Ser Gln Gly Gln Leu Leu
385                 390                 395                 400

Ser Ile Pro Gln Gly Phe Ser Val Val Lys Arg Ala Thr Ser Glu Gln
                405                 410                 415
```

```
Phe Arg Trp Ile Glu Phe Lys Thr Asn Ala Asn Ala Gln Ile Asn Thr
            420                 425                 430

Leu Ala Gly Arg Thr Ser Val Leu Arg Gly Leu Pro Leu Glu Val Ile
        435                 440                 445

Ser Asn Gly Tyr Gln Ile Ser Leu Glu Glu Ala Arg Arg Val Lys Phe
    450                 455                 460

Asn Thr Ile Glu Thr Thr Leu Thr His Ser Ser Gly Pro Ala Ser Tyr
465                 470                 475                 480

Gly Gly Pro Arg Lys Ala Asp Ala
                485


<210>  3
<211>  509
<212>  PRT
<213>  NONE

<400>  3

Met Val Lys Val Pro His Leu Leu Val Ala Thr Phe Gly Val Leu Leu
1               5                   10                  15

Val Leu Asn Gly Cys Leu Ala Arg Gln Ser Leu Gly Val Pro Pro Gln
            20                  25                  30

Leu Gly Asn Ala Cys Asn Leu Asp Asn Leu Asp Val Leu Gln Pro Thr
        35                  40                  45

Glu Thr Ile Lys Ser Glu Ala Gly Arg Val Glu Tyr Trp Asp His Asn
    50                  55                  60

Asn Pro Gln Ile Arg Cys Ala Gly Val Ser Val Ser Arg Val Ile Ile
65                  70                  75                  80

Glu Gln Gly Gly Leu Tyr Leu Pro Thr Phe Phe Ser Ser Pro Lys Ile
                85                  90                  95

Ser Tyr Val Val Gln Gly Met Gly Ile Ser Gly Arg Val Val Pro Gly
            100                 105                 110

Cys Ala Glu Thr Phe Met Asp Ser Gln Pro Met Gln Gly Gln Gln Gln
        115                 120                 125

Gly Gln Pro Trp Gln Gly Gln Gln Gly Gln Gln Gly Gln Gln Gly Gln
    130                 135                 140
```

```
Gln Gly Gln Gln Gly Gln Gln Gly Gln Gln Gly Gln Gln Gly Gln Gln
145                 150                 155                 160

Gly Gln Gln Gly Gln Gln Gly Gln Gln Gln Gln Gly Phe Arg Asp Met
                165                 170                 175

His Gln Lys Val Glu His Val Arg His Gly Asp Ile Ile Ala Ile Thr
            180                 185                 190

Ala Gly Ser Ser His Trp Ile Tyr Asn Thr Gly Asp Gln Pro Leu Val
        195                 200                 205

Ile Ile Cys Leu Leu Asp Ile Ala Asn Tyr Gln Asn Gln Leu Asp Arg
    210                 215                 220

Asn Pro Arg Thr Phe Arg Leu Ala Gly Asn Asn Pro Gln Gly Gly Ser
225                 230                 235                 240

Gln Gln Gln Gln Gln Gln Gln Gln Asn Met Leu Ser Gly Phe Asp Pro
                245                 250                 255

Gln Val Leu Ala Gln Ala Leu Lys Ile Asp Val Arg Leu Ala Gln Glu
            260                 265                 270

Leu Gln Asn Gln Gln Asp Ser Arg Gly Asn Ile Val Arg Val Lys Gly
        275                 280                 285

Pro Phe Gln Val Val Arg Pro Pro Leu Arg Gln Pro Tyr Glu Ser Glu
    290                 295                 300

Gln Trp Arg His Pro Arg Gly Pro Pro Gln Ser Pro Gln Asp Asn Gly
305                 310                 315                 320

Leu Glu Glu Thr Ile Cys Ser Met Arg Thr His Glu Asn Ile Asp Asp
                325                 330                 335

Pro Ala Arg Ala Asp Val Tyr Lys Pro Asn Leu Gly Arg Val Thr Ser
            340                 345                 350

Val Asn Ser Tyr Thr Leu Pro Ile Leu Gln Tyr Ile Arg Leu Ser Ala
        355                 360                 365

Thr Arg Gly Ile Leu Gln Gly Asn Ala Met Val Leu Pro Lys Tyr Asn
    370                 375                 380

Met Asn Ala Asn Glu Ile Leu Tyr Cys Thr Gln Gly Gln Ala Arg Ile
385                 390                 395                 400
```

Gln Val Val Asn Asp Asn Gly Gln Asn Val Leu Asp Gln Gln Val Gln
405 410 415

Lys Gly Gln Leu Val Val Ile Pro Gln Gly Phe Ala Tyr Val Val Gln
420 425 430

Ser His Gln Asn Asn Phe Glu Trp Ile Ser Phe Lys Thr Asn Ala Asn
435 440 445

Ala Met Val Ser Thr Leu Ala Gly Arg Thr Ser Ala Leu Arg Ala Leu
450 455 460

Pro Leu Glu Val Ile Thr Asn Ala Phe Gln Ile Ser Leu Glu Glu Ala
465 470 475 480

Arg Arg Ile Lys Phe Asn Thr Leu Glu Thr Thr Leu Thr Arg Ala Arg
485 490 495

Gly Gly Gln Pro Gln Leu Ile Glu Glu Ile Val Glu Ala
500 505

<210> 4
<211> 496
<212> PRT
<213> NONE

<400> 4

Met Ala Arg Leu Ser Ser Leu Leu Tyr Phe Ser Ile Thr Val Leu Ile
1 5 10 15

Phe Leu His Gly Ser Thr Ala Gln Gln Phe Pro Asn Glu Cys Gln Leu
20 25 30

Asp Gln Leu Asn Ala Leu Glu Pro Ser His Val Leu Lys Ala Glu Ala
35 40 45

Gly Arg Ile Glu Val Trp Asp His His Ala Pro Gln Leu Arg Cys Ser
50 55 60

Gly Val Ser Phe Val Arg Tyr Ile Ile Glu Ser Gln Gly Leu Tyr Leu
65 70 75 80

Pro Ser Phe Leu Asn Thr Ala Asn Val Ser Phe Val Ala Lys Gly Gln
85 90 95

Gly Leu Met Gly Arg Val Val Pro Gly Cys Ala Glu Thr Phe Gln Asp
100 105 110

Ser Ser Val Phe Gln Pro Gly Ser Gly Ser Pro Phe Gly Glu Gly Gln
115 120 125

Gly Gln Gly Gln Gln Gly Gln Gly Gln Gly Gln Gly Gln Gly Gln Gly
130 135 140

Lys Gly Gln Gln Gly Gln Gly Lys Gly Gln Gln Gly Gln Ser Gln Gly
145 150 155 160

Gln Gln Gly Gln Gly Gln Gly Phe Arg Asp Met His Gln Lys Val Glu
165 170 175

His Ile Arg Ser Gly Asp Thr Ile Ala Thr His Pro Gly Val Ala Gln
180 185 190

Trp Phe Tyr Asn Asn Gly Asn Gln Pro Leu Val Ile Val Ala Val Met
195 200 205

Asp Leu Ala Ser His Gln Asn Gln Leu Asp Arg Asn Pro Ser Gln Phe
210 215 220

Tyr Leu Ala Gly Lys Asn Pro Gln Gly Gln Ser Trp Leu His Gly Arg
225 230 235 240

Gly Gln Gln Pro Gln Asn Asn Ile Leu Asn Gly Phe Ser Pro Glu Val
245 250 255

Leu Ala Gln Ala Phe Lys Ile Asp Val Arg Thr Ala Gln Gln Leu Gln
260 265 270

Asn Gln Gln Asp Asn Arg Gly Asn Ile Val Arg Val Gln Gly Pro Phe
275 280 285

Gly Val Ile Arg Pro Pro Leu Lys Ser Gln Arg Pro Gln Glu Thr Glu
290 295 300

Ala Asn Gly Leu Glu Glu Thr Ile Cys Ser Ala Arg Cys Thr Asp Asn
305 310 315 320

Leu Asp Asp Pro Ser Asn Ala Asp Val Tyr Lys Pro Gln Leu Gly Tyr
325 330 335

Ile Ser Ile Leu Asn Ser Tyr Asp Leu Pro Ile Leu Arg Val Leu Arg
340 345 350

Leu Ser Ala Leu Arg Gly Ser Ile Arg Gln Asn Ala Met Val Leu Pro

355 360 365

Gln Trp Lys Ser Lys Ser Asn Ala Val Leu Tyr Val Thr Asp Gly Glu
370 375 380

Ala Gln Ile Gln Val Val Asn Asp Asn Gly Asp Arg Val Phe Asp Gly
385 390 395 400

Gln Val Ser Gln Gly Gln Leu Leu Ser Ile Pro Gln Gly Phe Ser Val
405 410 415

Val Lys Arg Ala Thr Ser Asp Gln Phe Arg Trp Ile Glu Phe Lys Thr
420 425 430

Asn Ala Asn Ala Gln Ile Asn Thr Leu Ala Gly Arg Thr Ser Val Met
435 440 445

Arg Gly Leu Pro Leu Glu Val Ile Ala Asn Gly Tyr Gln Ile Ser Leu
450 455 460

Glu Glu Ala Arg Arg Val Lys Phe Asn Thr Ile Glu Thr Thr Leu Thr
465 470 475 480

His Ser Ser Gly Pro Ala Ser Tyr Gly Arg Pro Arg Lys Ala Asp Ala
485 490 495

<210> 5
<211> 490
<212> PRT
<213> NONE

<400> 5

Met Ala Arg Leu Ser Ser Leu Leu Ser Phe Ser Leu Ala Leu Leu Ile
1 5 10 15

Phe Leu His Gly Ser Thr Ala Gln Gln Phe Pro Asn Glu Cys Gln Leu
20 25 30

Asp Gln Leu Asn Ala Leu Glu Pro Ser His Val Leu Lys Ala Glu Ala
35 40 45

Gly Arg Ile Glu Val Trp Asp His His Ala Pro Gln Leu Arg Cys Ser
50 55 60

Gly Val Ser Phe Val Arg Tyr Ile Ile Glu Ser Lys Gly Leu Tyr Leu
65 70 75 80

Pro Ser Phe Phe Ser Thr Ala Lys Leu Ser Phe Val Ala Lys Gly Glu

85 90 95

Gly Leu Met Gly Arg Val Val Pro Gly Cys Ala Glu Thr Phe Gln Asp
100 105 110

Ser Ser Val Phe Gln Pro Ser Gly Gly Ser Pro Ser Gly Glu Gly Gln
115 120 125

Gly Gln Gly Gln Gln Gly Gln Gly Gln Gly His Gln Gly Gln Gly Gln
130 135 140

Gly Gln Gln Gly Gln Gln Gly Gln Gln Gly Gln Gln Ser Gln Gly Gln
145 150 155 160

Gly Phe Arg Asp Met His Gln Lys Val Glu His Ile Arg Thr Gly Asp
165 170 175

Thr Ile Ala Thr His Pro Gly Val Ala Gln Trp Phe Tyr Asn Asp Gly
180 185 190

Asn Gln Pro Leu Val Ile Val Ser Val Leu Asp Leu Ala Ser His Gln
195 200 205

Asn Gln Leu Asp Arg Asn Pro Arg Pro Phe Tyr Leu Ala Gly Asn Asn
210 215 220

Pro Gln Gly Gln Val Trp Ile Glu Gly Arg Glu Gln Gln Pro Gln Lys
225 230 235 240

Asn Ile Leu Asn Gly Phe Thr Pro Glu Val Leu Ala Lys Ala Phe Lys
245 250 255

Ile Asp Val Arg Thr Ala Gln Gln Leu Gln Asn Gln Gln Asp Asn Arg
260 265 270

Gly Asn Ile Ile Arg Val Gln Gly Pro Phe Ser Val Ile Arg Pro Pro
275 280 285

Leu Arg Ser Gln Arg Pro Gln Glu Thr Glu Val Asn Gly Leu Glu Glu
290 295 300

Thr Ile Cys Ser Ala Arg Cys Thr Asp Asn Leu Asp Asp Pro Ser Asn
305 310 315 320

Ala Asp Val Tyr Lys Pro Gln Leu Gly Tyr Ile Ser Thr Leu Asn Ser
325 330 335

Tyr Asp Leu Pro Ile Leu Arg Phe Leu Arg Leu Ser Ala Leu Arg Gly
340 345 350

Ser Ile Arg Gln Asn Ala Met Val Leu Pro Gln Trp Asn Ala Asn Ala
355 360 365

Asn Ala Val Leu Tyr Val Thr Asp Gly Glu Ala His Val Gln Val Val
370 375 380

Asn Asp Asn Gly Asp Arg Val Phe Asp Gly Gln Val Ser Gln Gly Gln
385 390 395 400

Leu Leu Ser Ile Pro Gln Gly Phe Ser Val Val Lys Arg Ala Thr Ser
405 410 415

Glu Gln Phe Arg Trp Ile Glu Phe Lys Thr Asn Ala Asn Ala Gln Ile
420 425 430

Asn Thr Leu Ala Gly Arg Thr Ser Val Leu Arg Gly Leu Pro Leu Glu
435 440 445

Val Ile Ser Asn Gly Tyr Gln Ile Ser Leu Glu Glu Ala Arg Arg Val
450 455 460

Lys Phe Asn Thr Ile Glu Thr Thr Leu Thr His Ser Ser Gly Pro Ala
465 470 475 480

Ser Tyr Gly Gly Pro Arg Lys Ala Asp Ala
485 490

<210> 6
<211> 506
<212> PRT
<213> NONE

<400> 6

Pro Cys Glu Thr Ala Val Ala Thr Phe Gly Val Leu Leu Val Leu Asn
1 5 10 15

Gly Cys Leu Ala Arg Gln Ser Leu Gly Val Pro Pro Gln Leu Gly Asn
20 25 30

Ala Cys Asn Leu Asp Asn Leu Asp Val Leu Gln Pro Thr Glu Thr Ile
35 40 45

Lys Ser Glu Ala Gly Arg Val Glu Tyr Trp Asp His Asn Asn Pro Gln
50 55 60

```
Ile Arg Cys Ala Gly Val Ser Val Ser Arg Val Ile Ile Glu Gln Gly
65                  70                  75                  80

Gly Leu Tyr Leu Pro Thr Phe Phe Ser Ser Pro Lys Ile Ser Ile Val
                85                  90                  95

Val Gln Gly Met Gly Ile Ser Gly Arg Val Val Pro Gly Cys Ala Glu
            100                 105                 110

Thr Phe Met Asp Ser Gln Pro Met Gln Gly Gln Gln Gln Gly Gln Pro
        115                 120                 125

Trp Gln Gly Gln Gln Gly Gln Gln Gly Gln Gln Gly Gln Gln Gly Gln
    130                 135                 140

Gln Gly Gln Gln Gly Gln Gln Gly Gln Gln Gly Gln Gln Gly Gln Gln
145                 150                 155                 160

Gly Gln Gln Gly Gln Gln Gln Gln Gly Phe Arg Asp Met His Gln Lys
                165                 170                 175

Val Glu His Val Arg His Gly Asp Ile Ile Ala Ile Thr Ala Gly Ser
            180                 185                 190

Ser His Trp Ile Tyr Asn Thr Gly Asp Gln Pro Leu Val Ile Ile Cys
        195                 200                 205

Leu Leu Asp Ile Ala Asn Tyr Gln Asn Gln Leu Asp Arg Asn Pro Arg
    210                 215                 220

Thr Phe Arg Leu Ala Gly Asn Asn Pro Gln Gly Gly Ser Gln Gln Gln
225                 230                 235                 240

Gln Gln Gln Gln Gln Asn Met Leu Ser Gly Phe Asp Pro Gln Val Leu
                245                 250                 255

Ala Gln Ala Leu Lys Ile Asp Val Arg Leu Ala Gln Glu Leu Gln Asn
            260                 265                 270

Gln Gln Asp Ser Arg Gly Asn Ile Val Arg Val Lys Gly Pro Phe Gln
        275                 280                 285

Val Val Arg Pro Pro Leu Arg Gln Pro Tyr Glu Ser Glu Gln Trp Arg
    290                 295                 300

His Pro Arg Gly Pro Pro Gln Ser Pro Gln Asp Asn Gly Leu Glu Glu
305                 310                 315                 320
```

Thr Ile Cys Ser Met Arg Thr His Glu Asn Ile Asp Asp Pro Ala Arg
325 330 335

Ala Asp Val Tyr Lys Pro Asn Leu Gly Arg Val Thr Ser Ala Asn Ser
340 345 350

Tyr Thr Leu Pro Ile Leu Gln Tyr Ile Arg Leu Ser Ala Thr Arg Gly
355 360 365

Ile Leu Gln Gly Asn Ala Met Val Leu Pro Lys Tyr Asn Met Asn Ala
370 375 380

Asn Glu Ile Leu Tyr Cys Thr Gln Gly Gln Ala Arg Ile Gln Val Val
385 390 395 400

Asn Asp Asn Gly Gln Asn Val Leu Asp Gln Gln Val Gln Lys Gly Gln
405 410 415

Leu Val Val Ile Pro Gln Gly Phe Ala Tyr Val Val Gln Ser His Gln
420 425 430

Asn Asn Phe Glu Trp Ile Ser Phe Lys Thr Asn Ala Asn Ala Met Val
435 440 445

Ser Thr Leu Ala Gly Arg Thr Ser Ala Leu Arg Ala Leu Pro Leu Glu
450 455 460

Val Ile Thr Asn Ala Phe Gln Ile Ser Leu Glu Glu Ala Arg Arg Ile
465 470 475 480

Lys Phe Asn Thr Leu Glu Thr Thr Leu Thr Arg Ala Arg Gly Gly Gln
485 490 495

Pro Gln Leu Ile Glu Glu Ile Val Glu Ala
500 505

<210> 7
<211> 489
<212> PRT
<213> NONE

<400> 7

Met Val Lys Leu Ala His Leu Leu Val Ala Thr Phe Gly Ala Leu Leu
1 5 10 15

Val Leu Asn Gly Cys Leu Ala Arg Gln Ser Leu Gly Val Pro Pro Gln
20 25 30

```
Ile Gly Asn Ala Cys Asn Leu Asp Asn Leu Asp Val Leu Gln Pro Thr
        35                  40                  45

Glu Thr Ile Lys Ser Glu Ala Gly Arg Val Glu Tyr Trp Asp His Asn
    50                  55                  60

Asn Pro Gln Ile Arg Cys Ala Gly Val Ser Val Ser Arg Leu Ile Ile
65                  70                  75                  80

Glu Gln Gly Gly Leu Tyr Leu Pro Thr Phe Phe Ser Ser Pro Lys Ile
                85                  90                  95

Ser Tyr Val Val Gln Gly Met Gly Ile Ser Gly Arg Val Val Pro Gly
            100                 105                 110

Cys Ala Glu Thr Phe Met Asp Ser Gln Pro Met Gln Gly Gln Gln Gln
        115                 120                 125

Gly Gln Gln Gly Gln Gln Gly Gln Gln Gly Gln Gln Gly Gln Gln Gly
    130                 135                 140

Gln Gln Gly Leu Gln Gln Gln Gly Phe Arg Asp Met His Gln Lys Val
145                 150                 155                 160

Glu His Val Arg His Gly Asp Val Ile Ala Ile Thr Ala Gly Ser Ser
                165                 170                 175

His Trp Ile Tyr Asn Thr Gly Asp Gln Pro Leu Val Ile Ile Cys Leu
            180                 185                 190

Leu Asp Ile Ala Asn Tyr Gln Asn Gln Leu Asp Arg Asn Pro Arg Thr
        195                 200                 205

Phe Arg Leu Ala Gly Asn Asn Pro Gln Gly Gly Ser Gln Gln Gln Gln
    210                 215                 220

Gln Gln Gln Gln Asn Met Leu Ser Gly Phe Asp Pro Gln Val Leu Ala
225                 230                 235                 240

Gln Ala Leu Lys Ile Asp Val Arg Leu Ala Gln Glu Leu Gln Asn Gln
                245                 250                 255

Gln Asp Ser Arg Gly Asn Ile Val Arg Val Lys Gly Pro Phe Gln Val
            260                 265                 270

Val Arg Pro Pro Leu Arg Gln Pro Tyr Glu Ser Glu Gln Trp Arg His
        275                 280                 285
```

```
Pro Arg Gly Pro Pro Gln Ser Pro Gln Asp Asn Gly Leu Glu Glu Thr
    290                 295                 300

Ile Cys Ser Met Arg Thr His Glu Asn Ile Asp Asp Pro Ala Arg Ala
305                 310                 315                 320

Asp Val Tyr Lys Pro Asn Leu Gly Arg Val Thr Ser Val Asn Ser Tyr
                325                 330                 335

Thr Leu Pro Ile Leu Gln Tyr Ile Arg Leu Ser Ala Thr Arg Gly Ile
            340                 345                 350

Leu Gln Gly Asn Ala Met Val Leu Pro Lys Tyr Asn Met Asn Ala Asn
        355                 360                 365

Glu Ile Leu Tyr Cys Thr Gln Gly Gln Ala Arg Ile Gln Val Val Asn
    370                 375                 380

Asp Asn Gly Gln Asn Val Leu Asp Gln Gln Val Gln Lys Gly Gln Leu
385                 390                 395                 400

Val Val Ile Pro Gln Gly Phe Ala Tyr Val Val Gln Ser His Gln Asn
                405                 410                 415

Asn Phe Glu Trp Ile Ser Phe Lys Thr Asn Ala Asn Ala Met Val Ser
            420                 425                 430

Thr Leu Ala Gly Arg Thr Ser Ala Leu Arg Ala Leu Pro Leu Glu Val
        435                 440                 445

Leu Thr Asn Ala Phe Gln Ile Ser Leu Glu Glu Ala Arg Arg Ile Lys
    450                 455                 460

Phe Asn Thr Leu Glu Thr Thr Leu Thr Arg Ala Arg Arg Gly Gln Pro
465                 470                 475                 480

Gln Leu Ile Glu Glu Ile Val Glu Ala
                485

<210>  8
<211>  476
<212>  PRT
<213>  NONE

<220>
<221>  misc_feature
<222>  (335)..(335)
```

<223> Xaa can be any naturally occurring amino acid

<220>
<221> misc_feature
<222> (338)..(338)
<223> Xaa can be any naturally occurring amino acid

<400> 8

```
Arg Leu Ser Ser Leu Leu Ser Phe Ser Leu Ala Leu Leu Ile Phe Leu
1               5                   10                  15

His Gly Ser Thr Ala Gln Gln Phe Pro Asn Glu Cys Gln Leu Asp Gln
            20                  25                  30

Leu Asn Ala Leu Glu Pro Ser His Val Leu Lys Ala Glu Ala Gly Arg
        35                  40                  45

Ile Glu Val Trp Asp His His Ala Pro Gln Leu His Cys Ser Gly Val
    50                  55                  60

Ser Phe Val Arg Tyr Ile Ile Glu Ser Lys Gly Leu Tyr Leu Pro Ser
65                  70                  75                  80

Phe Phe Ser Thr Ala Lys Leu Ser Phe Val Ala Lys Gly Gln Gly Leu
                85                  90                  95

Met Gly Arg Val Val Pro Gly Cys Ala Glu Thr Phe Gln Asp Ser Ser
            100                 105                 110

Val Phe Gln Pro Gly Gly Gly Ser Pro Phe Gly Glu Gly Gln Gly Gln
        115                 120                 125

Gly Gln Gln Gly Gln Gly Gln Gly Gln Gln Gly Gln Gly Gln Gly Gln
    130                 135                 140

Gln Gly Gln Gly Gln Gln Gly Gln Gly Gln Gly Phe Arg Asp Met His
145                 150                 155                 160

Gln Lys Val Glu His Ile Arg Thr Gly Asp Thr Ile Ala Thr His Pro
                165                 170                 175

Gly Val Ala Gln Trp Phe Tyr Asn Asp Gly Asn Gln Pro Leu Val Ile
            180                 185                 190

Val Ser Val Leu Asp Leu Ala Ser His Gln Asn Gln Leu Asp Arg Asn
        195                 200                 205

Pro Arg Pro Phe Tyr Leu Ala Gly Asn Asn Pro Gln Gly Gln Val Trp
    210                 215                 220
```

```
Ile Glu Gly Arg Glu Gln Gln Pro Gln Lys Asn Ile Leu Asn Gly Phe
225                 230                 235                 240

Thr Pro Glu Val Leu Ala Lys Ala Phe Lys Ile Asp Val Arg Thr Ala
                245                 250                 255

Gln Gln Leu Gln Asn Gln Gln Asp Asn Arg Gly Asn Ile Val Arg Val
            260                 265                 270

Gln Gly Pro Phe Ser Val Ile Arg Pro Pro Leu Arg Ser Gln Arg Pro
        275                 280                 285

Gln Glu Glu Val Asn Gly Leu Glu Glu Thr Ile Cys Ser Ala Arg Cys
    290                 295                 300

Thr Asp Asn Leu Asp Asp Pro Ser Asn Ala Asp Val Tyr Lys Pro Gln
305                 310                 315                 320

Leu Gly Tyr Ile Ser Thr Leu Asn Ser Tyr Asp Leu Pro Ile Xaa Arg
                325                 330                 335

Phe Xaa Arg Leu Ser Ala Leu Arg Gly Ser Ile Arg Gln Asn Ala Met
            340                 345                 350

Val Leu Pro Gln Trp Asn Ala Asn Ala Asn Ala Val Leu Tyr Val Thr
        355                 360                 365

Asp Gly Glu Ala His Val Gln Val Val Asn Asp Asn Gly Asp Arg Val
    370                 375                 380

Phe Asp Gly Gln Val Ser Gln Gly Gln Leu Leu Ser Ile Pro Gln Gly
385                 390                 395                 400

Phe Ser Val Val Lys Arg Ala Thr Ser Glu Gln Phe Arg Trp Ile Glu
                405                 410                 415

Phe Lys Thr Asn Ala Asn Ala Gln Ile Asn Thr Leu Ala Gly Arg Thr
            420                 425                 430

Ser Val Leu Arg Gly Leu Pro Leu Glu Val Ile Ser Asn Gly Tyr Gln
        435                 440                 445

Ile Ser Leu Glu Glu Ala Arg Arg Val Lys Phe Asn Thr Ile Glu Thr
    450                 455                 460

Thr Leu Thr His Ser Ser Gly Pro Ala Ser Tyr Gly
```

465 470 475

<210> 9
<211> 466
<212> PRT
<213> NONE

<400> 9

Gln Gln Phe Pro Asn Glu Cys Gln Leu Asp Gln Leu Asn Ala Leu Glu
1 5 10 15

Pro Ser His Val Leu Lys Ala Glu Ala Gly Arg Ile Glu Val Trp Asp
20 25 30

His His Ala Pro Gln Leu Arg Cys Ser Gly Val Ser Phe Val Arg Tyr
35 40 45

Ile Ile Glu Ser Lys Gly Leu Tyr Leu Pro Ser Phe Phe Ser Thr Ala
50 55 60

Lys Leu Ser Phe Val Ala Lys Gly Glu Gly Leu Met Gly Arg Val Val
65 70 75 80

Pro Gly Cys Ala Glu Thr Phe Gln Asp Ser Ser Val Phe Gln Pro Gly
85 90 95

Gly Gly Ser Pro Phe Gly Glu Gly Gln Gly Gln Gly Gln Gln Gly Gln
100 105 110

Gly Gln Gly His Gln Gly Gln Gly Gln Gly Gln Gln Gly Gln Gln Gly
115 120 125

Gln Gln Gly Gln Gln Ser Gln Gly Gln Gly Phe Arg Asp Met His Gln
130 135 140

Lys Val Glu His Ile Arg Thr Gly Asp Thr Ile Ala Thr His Pro Gly
145 150 155 160

Val Ala Gln Trp Phe Tyr Asn Asp Gly Asn Gln Pro Leu Val Ile Val
165 170 175

Ser Val Leu Asp Leu Ala Ser His Gln Asn Gln Leu Asp Arg Asn Pro
180 185 190

Arg Pro Phe Tyr Leu Ala Gly Asn Asn Pro Gln Gly Gln Val Trp Ile
195 200 205

Glu Gly Arg Glu Gln Gln Pro Gln Lys Asn Ile Leu Asn Gly Phe Thr

```
        210                 215                 220

Pro Glu Val Leu Ala Lys Ala Phe Lys Ile Asp Val Arg Thr Ala Gln
225                 230                 235                 240

Gln Leu Gln Asn Gln Gln Asp Asn Arg Gly Asn Ile Ile Arg Val Gln
                245                 250                 255

Gly Pro Phe Ser Val Ile Arg Pro Pro Leu Arg Ser Gln Arg Pro Gln
            260                 265                 270

Glu Glu Val Asn Gly Leu Glu Glu Thr Ile Cys Ser Ala Arg Cys Thr
        275                 280                 285

Asp Asn Leu Asp Asp Pro Ser Asn Ala Asp Val Tyr Lys Pro Gln Leu
    290                 295                 300

Gly Tyr Ile Ser Thr Leu Asn Ser Tyr Asp Leu Pro Ile Leu Arg Phe
305                 310                 315                 320

Leu Arg Leu Ser Ala Leu Arg Gly Ser Ile Arg Gln Asn Ala Met Val
                325                 330                 335

Leu Pro Gln Trp Asn Ala Asn Ala Asn Ala Val Leu Tyr Val Thr Asp
            340                 345                 350

Gly Glu Ala His Val Gln Val Val Asn Asp Asn Gly Asp Arg Val Phe
        355                 360                 365

Asp Gly Gln Val Ser Gln Gly Gln Leu Leu Ser Ile Pro Gln Gly Phe
    370                 375                 380

Ser Val Val Lys Arg Ala Thr Ser Glu Gln Phe Arg Trp Ile Glu Phe
385                 390                 395                 400

Lys Thr Asn Ala Asn Ala Gln Ile Asn Thr Leu Ala Gly Arg Thr Ser
                405                 410                 415

Val Leu Arg Gly Leu Pro Leu Glu Val Ile Ser Asn Gly Tyr Gln Ile
            420                 425                 430

Ser Leu Glu Glu Ala Arg Arg Val Lys Phe Asn Thr Ile Glu Thr Thr
        435                 440                 445

Leu Thr His Ser Ser Gly Pro Ala Ser Tyr Gly Gly Pro Arg Lys Ala
    450                 455                 460
```

Asp Ala
465

<210> 10
<211> 467
<212> PRT
<213> NONE

<400> 10

Gln Gln Phe Pro Asn Glu Cys Gln Leu Asp Gln Leu Asn Ala Leu Glu
1 5 10 15

Pro Ser His Val Leu Lys Ala Glu Ala Gly Arg Ile Glu Val Trp Asp
20 25 30

His His Ala Pro Gln Leu Arg Cys Ser Gly Val Ser Phe Val Arg Tyr
35 40 45

Ile Ile Glu Ser Lys Gly Leu Tyr Leu Pro Ser Phe Phe Ser Thr Ala
50 55 60

Lys Leu Ser Phe Val Ala Lys Gly Gln Gly Leu Met Gly Arg Val Val
65 70 75 80

Pro Gly Cys Ala Glu Thr Phe Gln Asp Ser Ser Val Phe Gln Pro Gly
85 90 95

Gly Gly Ser Pro Phe Gly Glu Gly Gln Gly Gln Gly Gln Gln Gly Gln
100 105 110

Gly Gln Gly His Gln Gly Gln Gly Gln Gly Gln Gln Gly Gln Gln Gly
115 120 125

Gln Gln Gly Gln Gln Ser Gln Gly Gln Gly Phe Arg Asp Met His Gln
130 135 140

Lys Val Glu His Ile Arg Ser Gly Asp Thr Ile Ala Thr His Pro Gly
145 150 155 160

Val Ala Gln Trp Phe Tyr Asn Asn Gly Asn Gln Pro Leu Val Ile Val
165 170 175

Ala Val Met Asp Leu Ala Ser His Gln Asn Gln Leu Asp Arg Asn Pro
180 185 190

Arg Pro Phe Tyr Leu Ala Gly Lys Asn Pro Gln Gly Gln Ser Trp Leu
195 200 205

```
His Gly Arg Gly Gln Gln Pro Gln Asn Asn Ile Leu Asn Gly Phe Ser
    210                 215                 220

Pro Glu Val Leu Ala Gln Ala Phe Lys Ile Asp Val Arg Thr Ala Gln
225                 230                 235                 240

Gln Leu Gln Asn Gln Gln Asp Asn Arg Gly Asn Ile Val Arg Val Gln
                245                 250                 255

Gly Pro Phe Gly Val Ile Arg Pro Pro Leu Lys Ser Gln Arg Pro Gln
            260                 265                 270

Glu Thr Glu Ala Asn Gly Leu Glu Glu Thr Ile Cys Ser Ala Arg Cys
        275                 280                 285

Thr Asp Asn Leu Asp Asp Pro Ser Asn Ala Asp Val Tyr Lys Pro Gln
    290                 295                 300

Leu Gly Tyr Ile Ser Ile Leu Asn Ser Tyr Asp Leu Pro Ile Leu Arg
305                 310                 315                 320

Val Leu Arg Leu Ser Ala Leu Arg Gly Ser Ile Arg Gln Asn Ala Met
                325                 330                 335

Val Leu Pro Gln Trp Asn Ala Asn Ala Asn Ala Val Leu Tyr Val Thr
            340                 345                 350

Asp Gly Glu Ala Gln Ile Gln Val Val Asn Asp Asn Gly Asp Arg Val
        355                 360                 365

Phe Asp Gly Gln Val Ser Gln Gly Gln Leu Leu Ser Ile Pro Gln Gly
    370                 375                 380

Phe Ser Val Val Lys Arg Ala Thr Ser Asp Gln Phe Arg Trp Ile Glu
385                 390                 395                 400

Phe Lys Thr Asn Ala Asn Ala Gln Ile Asn Thr Leu Ala Gly Arg Thr
                405                 410                 415

Ser Val Val Arg Gly Leu Pro Leu Glu Val Ile Ala Asn Gly Tyr Gln
            420                 425                 430

Ile Ser Leu Glu Glu Ala Arg Arg Val Lys Phe Asn Thr Ile Glu Thr
        435                 440                 445

Thr Leu Thr His Ser Ser Gly Pro Ala Ser Tyr Gly Gly Pro Arg Lys
    450                 455                 460
```

Ala Asp Ala
465

<210> 11
<211> 186
<212> PRT
<213> NONE

<400> 11

Met Ala Asn Lys Leu Phe Leu Val Ser Ala Thr Leu Ala Leu Phe Phe
1 5 10 15

Leu Leu Thr Asn Ala Ser Val Tyr Arg Thr Val Val Glu Val Asp Glu
20 25 30

Asp Asp Ala Thr Asn Pro Ala Gly Pro Phe Arg Ile Pro Lys Cys Arg
35 40 45

Lys Glu Phe Gln Gln Ala Gln His Leu Arg Ala Cys Gln Gln Trp Leu
50 55 60

His Lys Gln Ala Met Gln Pro Gly Gly Gly Ser Gly Pro Ser Trp Thr
65 70 75 80

Leu Asp Gly Glu Phe Asp Phe Glu Asp Asp Val Glu Asn Gln Gln Gln
85 90 95

Gly Pro Gln Gln Arg Pro Pro Pro Pro Gln Gln Cys Cys Asn Glu Leu
100 105 110

His Gln Glu Glu Pro Leu Cys Val Cys Pro Thr Leu Lys Gly Ala Ser
115 120 125

Lys Ala Val Arg Gln Gln Val Arg Gln Gln Gln Gly Gln Gln Met Gln
130 135 140

Gly Gln Gln Met Gln Gln Val Ile Ser Arg Val Tyr Gln Thr Ala Thr
145 150 155 160

His Leu Pro Arg Val Cys Asn Ile Arg Gln Val Ser Ile Cys Pro Phe
165 170 175

Gln Lys Thr Met Pro Gly Pro Gly Phe Tyr
180 185

<210> 12
<211> 180
<212> PRT

<213> NONE

<400> 12

```
Met Ala Asn Lys Leu Phe Leu Val Ser Ala Thr Leu Ala Phe Phe Phe
1               5                   10                  15

Leu Leu Thr Asn Ala Ser Ile Tyr Arg Thr Ile Val Glu Val Asp Glu
            20                  25                  30

Asp Asp Ala Thr Asn Pro Ala Gly Pro Phe Arg Ile Pro Lys Cys Arg
        35                  40                  45

Lys Glu Phe Gln Gln Ala Gln His Leu Lys Ala Cys Gln Gln Trp Leu
    50                  55                  60

His Lys Gln Ala Met Gln Ser Gly Ser Gly Pro Ser Trp Thr Leu Asp
65                  70                  75                  80

Gly Glu Phe Asp Phe Glu Asp Asp Met Glu Asn Pro Gln Gly Pro Gln
                85                  90                  95

Gln Arg Pro Pro Leu Leu Gln Gln Cys Cys Asn Glu Leu His Gln Glu
            100                 105                 110

Glu Pro Leu Cys Val Cys Pro Thr Leu Lys Gly Ala Ser Lys Ala Val
        115                 120                 125

Lys Gln Gln Val Arg Gln Gln Gln Gly Gln Gln Gly Gln Gln Leu Gln
    130                 135                 140

Gln Val Ile Ser Arg Ile Tyr Gln Thr Ala Thr His Leu Pro Lys Val
145                 150                 155                 160

Cys Asn Ile Pro Gln Val Ser Val Cys Pro Phe Gln Lys Thr Met Pro
                165                 170                 175

Gly Pro Ser Tyr
            180
```

<210> 13
<211> 178
<212> PRT
<213> NONE

<400> 13

```
Met Ala Asn Lys Leu Phe Leu Val Ser Ala Thr Leu Ala Phe Phe Phe
1               5                   10                  15
```

Leu Leu Thr Asn Ala Ser Ile Tyr Arg Thr Val Val Glu Phe Asp Glu
            20                  25                  30

Asp Asp Ala Thr Asn Pro Ala Gly Pro Phe Arg Ile Pro Lys Cys Arg
        35                  40                  45

Lys Glu Phe Gln Gln Ala Gln His Leu Lys Ala Cys Gln Gln Trp Leu
    50                  55                  60

His Lys Gln Ala Met Gln Ser Gly Ser Gly Pro Ser Trp Thr Leu Asp
65                  70                  75                  80

Gly Glu Phe Asp Phe Glu Asp Asp Met Glu Asn Pro Gln Gly Pro Gln
                85                  90                  95

Gln Arg Pro Pro Leu Leu Gln Gln Cys Cys Asn Glu Leu His Gln Glu
            100                 105                 110

Glu Pro Leu Cys Val Cys Pro Thr Leu Lys Gly Ala Ser Lys Ala Val
        115                 120                 125

Lys Gln Gln Ile Gln Gln Gln Gly Gln Gln Gln Gly Lys Leu Gln Met
    130                 135                 140

Val Ser Arg Ile Tyr Gln Thr Ala Thr His Leu Pro Lys Val Cys Lys
145                 150                 155                 160

Ile Pro Gln Val Ser Val Cys Pro Phe Gln Lys Thr Met Pro Gly Pro
                165                 170                 175

Ser Tyr

<210> 14
<211> 178
<212> PRT
<213> NONE

<400> 14

Met Ala Asn Lys Leu Phe Leu Val Ser Ala Thr Leu Ala Phe Phe Phe
1               5                   10                  15

Leu Leu Thr Asn Ala Ser Ile Tyr Arg Thr Val Val Glu Phe Asp Glu
            20                  25                  30

Asp Asp Ala Thr Asp Ser Ala Gly Pro Phe Arg Ile Pro Lys Cys Arg
        35                  40                  45

Lys Glu Phe Gln Gln Ala Gln His Leu Arg Ala Cys Gln Gln Trp Leu
    50                  55                  60

His Lys Gln Ala Met Gln Ser Gly Gly Gly Pro Ser Trp Thr Leu Asp
65                  70                  75                  80

Gly Glu Phe Asp Phe Glu Asp Asp Met Glu Asn Pro Gln Gly Pro Gln
                85                  90                  95

Gln Arg Pro Pro Leu Leu Gln Gln Cys Cys Asn Glu Leu His Gln Glu
            100                 105                 110

Glu Pro Leu Cys Val Cys Pro Thr Leu Lys Gly Ala Ser Lys Ala Val
        115                 120                 125

Lys Gln Gln Ile Gln Gln Gln Gly Gln Gln Gln Gly Lys Gln Gln Met
    130                 135                 140

Val Ser Arg Ile Tyr Gln Thr Ala Thr His Leu Pro Lys Val Cys Asn
145                 150                 155                 160

Ile Pro Gln Val Ser Val Cys Pro Phe Gln Lys Thr Met Pro Gly Pro
                165                 170                 175

Ser Tyr

<210> 15
<211> 133
<212> PRT
<213> NONE

<400> 15

Pro Lys Cys Arg Lys Glu Phe Gln Gln Ala Gln His Leu Lys Ala Cys
1               5                   10                  15

Gln Gln Trp Leu His Lys Gln Ala Met Gln Ser Gly Gly Gly Pro Ser
            20                  25                  30

Trp Thr Leu Asp Gly Glu Phe Asp Phe Glu Asp Asp Met Glu Lys Gln
        35                  40                  45

Gly Pro Gln Gln Arg Pro Pro Leu His Gln Gln Tyr Cys Asn Glu Leu
    50                  55                  60

Gln Gln Glu Glu Pro Leu Cys Val Cys Pro Thr Leu Arg Gly Ala Ser
65                  70                  75                  80

Lys Ala Val Lys Gln Gln Ile Gln Gln Gln Glu Gln Gln Gln Gly Lys
85 90 95

Gln Gln Met Val Asn Arg Ile Tyr Gln Thr Ala Thr His Leu Pro Lys
100 105 110

Val Cys Asn Ile Pro Gln Val Ser Val Cys Pro Phe Gln Lys Thr Met
115 120 125

Pro Gly Pro Ser Tyr
130

<210> 16
<211> 125
<212> PRT
<213> NONE

<400> 16

Ser Ala Gly Pro Phe Arg Ile Pro Lys Cys Arg Lys Glu Phe Gln Gln
1 5 10 15

Ala Gln His Leu Arg Ala Cys Gln Gln Trp Leu His Lys Gln Ala Met
20 25 30

Gln Ser Gly Ser Gly Pro Gln Gly Pro Gln Gln Arg Pro Pro Leu Leu
35 40 45

Gln Gln Cys Cys Asn Glu Leu His Gln Glu Glu Pro Leu Cys Val Cys
50 55 60

Pro Thr Leu Lys Gly Ala Ser Arg Ala Val Lys Gln Gln Val Arg Gln
65 70 75 80

Gln Gln Gly Gln Gln Gly Gln Gln Leu Gln Gln Val Ile Ser Arg Ile
85 90 95

Tyr Gln Thr Ala Thr His Leu Pro Lys Val Cys Asn Ile Pro Gln Val
100 105 110

Ser Val Cys Pro Phe Gln Lys Thr Met Pro Gly Pro Ser
115 120 125

<210> 17
<211> 178
<212> PRT
<213> NONE

<400> 17

Met Ala Asn Lys Leu Phe Leu Val Ser Ala Thr Leu Ala Phe Phe Phe

1 5 10 15

Leu Leu Thr Asn Ala Ser Ile Tyr Arg Thr Val Val Glu Phe Asp Glu
20 25 30

Asp Asp Ala Thr Asn Ser Ala Gly Pro Phe Arg Ile Pro Lys Cys Arg
35 40 45

Lys Glu Phe Gln Gln Ala Gln His Leu Arg Ala Cys Gln Gln Trp Leu
50 55 60

His Lys Gln Ala Met Gln Ser Gly Gly Gly Pro Ser Trp Thr Leu Asp
65 70 75 80

Gly Glu Phe Asp Phe Glu Asp Asp Met Glu Asn Pro Gln Gly Pro Gln
85 90 95

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Gln Gln Ile Gln Gln Gln Gly Gln Gln Gln Gly Lys Gln Gln Met
130 135 140

Val Ser Arg Ile Tyr Gln Thr Arg Thr Asn Leu Pro Lys Val Cys Asn
145 150 155 160

Ile Pro Gln Val Ser Val Cys Pro Phe Gln Lys Thr Met Pro Gly Pro
165 170 175

Ser Tyr

<210> 18
<211> 180
<212> PRT
<213> NONE

<400> 18

Met Ala Asn Lys Leu Phe Leu Val Ser Ala Thr Leu Ala Leu Phe Phe
1 5 10 15

Leu Leu Thr Asn Ala Ser Ile Tyr Arg Thr Val Val Glu Val Glu Glu
20 25 30

Asp Asp Ala Thr Asn Pro Ala Gly Pro Phe Arg Ile Pro Lys Cys Arg

35 40 45

Lys Glu Phe Gln Gln Ala Gln His Leu Arg Ala Cys Gln Gln Trp Leu
50 55 60

His Lys Gln Ala Met Gln Ser Gly Ser Gly Pro Ser Trp Thr Leu Asp
65 70 75 80

Gly Glu Phe Asp Phe Glu Asp Asp Met Glu Asn Pro Gln Ser Pro Gln
85 90 95

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Gln Gln Val Arg Gln Gln Gln Gly Gln Gln Gly Gln Gln Leu Gln
130 135 140

Gln Val Ile Ser Arg Ile Tyr Gln Thr Ala Thr His Leu Pro Lys Val
145 150 155 160

Cys Asn Ile Pro Gln Val Ser Val Cys Pro Phe Gln Lys Thr Met Pro
165 170 175

Gly Pro Ser Tyr
180

<210> 19
<211> 180
<212> PRT
<213> NONE

<400> 19

Met Ala Asn Lys Leu Phe Leu Val Ser Ala Thr Leu Ala Leu Phe Phe
1 5 10 15

Leu Leu Thr Asn Ala Ser Ile Tyr Arg Thr Val Val Glu Val Glu Glu
20 25 30

Asp Asp Ala Thr Asn Pro Ala Gly Pro Phe Arg Ile Pro Lys Cys Arg
35 40 45

Lys Glu Phe Gln Gln Ala Gln His Leu Arg Ala Cys Gln Gln Trp Leu
50 55 60

His Lys Gln Ala Met Gln Ser Gly Ser Gly Pro Ser Trp Thr Leu Asp

```
65                  70                  75                  80

Gly Glu Phe Asp Phe Glu Asp Asp Met Glu Asn Pro Gln Ser Pro Gln
                85                  90                  95

Gln Arg Pro Pro Leu Leu Gln Gln Cys Cys Asn Glu Leu His Gln Glu
            100                 105                 110

Glu Pro Leu Cys Val Cys Pro Thr Leu Lys Gly Ala Ser Lys Ala Val
        115                 120                 125

Lys Gln Gln Val Arg Gln Gln Gln Gly Gln Gln Gly Gln Gln Leu Gln
    130                 135                 140

Gln Val Ile Ser Arg Ile Tyr Gln Thr Ala Thr His Leu Pro Lys Val
145                 150                 155                 160

Cys Asn Ile Pro Gln Val Ser Val Cys Pro Phe Gln Lys Thr Met Pro
                165                 170                 175

Gly Pro Ser Tyr
            180


<210>  20
<211>  186
<212>  PRT
<213>  NONE

<400>  20

Met Ala Asn Lys Leu Phe Leu Val Ser Ala Thr Leu Ala Leu Phe Phe
1               5                   10                  15

Leu Leu Thr Asn Ala Ser Val Tyr Arg Thr Val Val Glu Val Asp Glu
            20                  25                  30

Asp Asp Ala Thr Asn Pro Ala Gly Pro Phe Arg Ile Pro Lys Cys Arg
        35                  40                  45

Lys Glu Phe Gln Gln Ala Gln His Leu Arg Ala Cys Gln Gln Trp Leu
    50                  55                  60

His Lys Gln Ala Met Gln Pro Gly Gly Gly Ser Gly Pro Ser Trp Thr
65                  70                  75                  80

Leu Asp Gly Glu Phe Asp Phe Glu Asp Asp Val Glu Asn Gln Gln Gln
                85                  90                  95

Gly Pro Gln Gln Arg Pro Pro Leu Leu Gln Gln Cys Cys Asn Glu Leu
```

100 105 110

His Gln Glu Glu Pro Leu Cys Val Cys Pro Thr Leu Lys Gly Ala Ser
115 120 125

Lys Ala Val Lys Gln Gln Val Arg Gln Gln Gln Gly Gln Gln Met Gln
130 135 140

Gly Gln Gln Met Gln Gln Val Ile Ser Arg Ile Tyr Gln Thr Ala Thr
145 150 155 160

His Leu Pro Arg Val Cys Asn Ile Arg Gln Val Ser Ile Cys Pro Phe
165 170 175

Gln Lys Thr Met Pro Gly Pro Gly Phe Tyr
180 185

<210> 21
<211> 180
<212> PRT
<213> NONE

<400> 21

Met Ala Asn Lys Leu Phe Leu Val Ser Ala Thr Leu Ala Phe Phe Phe
1 5 10 15

Leu Leu Thr Asn Ala Ser Val Tyr Arg Thr Val Val Glu Val Asp Glu
20 25 30

Asp Asp Ala Thr Asn Pro Gly Gly Pro Phe Arg Ile Pro Lys Cys Arg
35 40 45

Lys Glu Phe Gln Gln Ala Gln His Leu Arg Ala Cys Gln Gln Trp Leu
50 55 60

His Lys Gln Ala Met Gln Ser Gly Ser Gly Pro Ser Leu Ala Leu Ala
65 70 75 80

Gly Glu Phe Asp Phe Glu Asp Asp Met Glu Asn Pro Gln Gly Ser Gln
85 90 95

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Ser Arg Val Gln Gln Gln Gly Gln Met Gln Gly Gln Gln Gln Gln

130 135 140

Gln Ile Val Gly Arg Ile Tyr Gln Thr Ala Lys His Leu Pro Arg Val
145 150 155 160

Cys Asn Ile Pro Gln Val Ser Val Cys Pro Phe Gln Lys Thr Thr Pro
165 170 175

Gly Pro Tyr Tyr
180

<210> 22
<211> 179
<212> PRT
<213> NONE

<400> 22

Met Ala Asn Lys Leu Phe Leu Val Ser Ala Thr Leu Ala Phe Phe Phe
1 5 10 15

Leu Leu Thr Asn Ala Ser Ile Tyr Arg Thr Val Val Glu Phe Asp Glu
20 25 30

Asp Asp Ala Thr Asn Pro Ala Gly Pro Phe Arg Ile Pro Lys Cys Arg
35 40 45

Lys Glu Phe Gln Gln Ala Gln His Leu Lys Ala Cys Gln Gln Trp Leu
50 55 60

His Lys Gln Ala Met Gln Ser Gly Ser Gly Pro Ser Trp Thr Leu Asp
65 70 75 80

Gly Glu Phe Asp Phe Glu Asp Asp Met Glu Asn Pro Gln Gly Pro Gln
85 90 95

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Gln Gln Ile Gln Gln Gln Gln Gly Gln Gln Gln Gly Lys Gln Gln
130 135 140

Met Val Ser Arg Ile Tyr Gln Thr Ala Thr His Leu Pro Lys Val Cys
145 150 155 160

Asn Ile Pro Gln Val Ser Val Cys Pro Phe Gln Lys Thr Met Pro Gly

165 170 175

Pro Ser Tyr

<210> 23
<211> 173
<212> PRT
<213> NONE

<400> 23

Met Ala Asn Lys Leu Phe Leu Val Ser Ala Thr Leu Ala Phe Phe Phe
1 5 10 15

Leu Leu Thr Asn Ala Ser Val Tyr Arg Thr Val Val Glu Val Asp Glu
20 25 30

Asp Asp Ala Thr Asn Pro Ala Gly Pro Phe Arg Ile Pro Lys Cys Arg
35 40 45

Lys Glu Phe Gln Gln Ala Gln His Leu Arg Ala Cys Gln Gln Trp Leu
50 55 60

His Lys Gln Ala Met Gln Ser Gly Ser Gly Pro Ser Trp Thr Leu Asp
65 70 75 80

Gly Glu Phe Asp Phe Glu Asp Asp Met Glu Asn Pro Gln Gly Pro Gln
85 90 95

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Gln Gln Gln Gly Gln Gln Leu Gln Gln Val Ile Ser Arg Ile Tyr
130 135 140

Gln Thr Ala Thr His Leu Pro Arg Val Cys Asn Ile Pro Gln Val Ser
145 150 155 160

Ile Cys Pro Phe Gln Lys Thr Thr Pro Gly Pro Tyr Tyr
165 170

<210> 24
<211> 173
<212> PRT
<213> NONE

<400> 24

Met Ala Asn Lys Leu Phe Leu Val Ser Ala Thr Leu Ala Phe Phe Phe
1 5 10 15

Leu Leu Thr Asn Ala Ser Ile Tyr Arg Thr Ile Val Glu Leu Glu Glu
20 25 30

Asp Asp Thr Thr Asn Pro Met Gly Pro Phe Gly Gly Lys Gln Lys Cys
35 40 45

Arg Arg Glu Phe Gln Gln Ala Gln His Leu Arg Ala Cys Gln Gln Trp
50 55 60

Leu His Lys Lys Gly Met Gln Ala Arg Arg Gly Pro Trp Ser Leu Glu
65 70 75 80

Glu Glu Phe Asp Phe Glu Asp Asp Met Val Asn Ser Asp Ser Arg Arg
85 90 95

Pro Pro Leu Leu Gln Gln Cys Cys Asn Glu Leu His Gln Glu Glu Pro
100 105 110

Asp Cys Val Cys Pro Thr Leu Lys Gln Ala Ser Lys Ala Val Arg Leu
115 120 125

Gln Ile Gln Gln Gly Gln Pro Gln Val Val Arg Arg Ile Tyr Gln Thr
130 135 140

Ala Lys His Leu Pro Asn Ile Cys Gln Ile Pro Gln Val Ser Ile Cys
145 150 155 160

Pro Phe Arg Lys Thr Met Pro Phe Gln Pro Pro His Tyr
165 170

<210> 25
<211> 173
<212> PRT
<213> NONE

<400> 25

Met Ala Asn Lys Leu Phe Leu Val Ser Ala Thr Leu Ala Phe Phe Phe
1 5 10 15

Leu Leu Thr Asn Ala Ser Val Tyr Arg Thr Val Val Glu Val Asp Glu
20 25 30

Asp Asp Ala Thr Asn Pro Ala Gly Pro Phe Arg Ile Pro Lys Cys Arg
35 40 45

```
Lys Glu Phe Gln Gln Ala Gln His Leu Arg Ala Cys Gln Gln Trp Leu
    50                  55                  60

His Lys Gln Ala Met Gln Ser Gly Ser Gly Pro Ser Trp Thr Leu Asp
65                  70                  75                  80

Gly Glu Phe Asp Phe Asp Asp Asp Met Glu Asn Pro Gln Gly Pro Gln
                85                  90                  95

Gln Arg Pro Pro Leu Leu Gln Gln Cys Cys Asn Glu Leu His Gln Glu
            100                 105                 110

Glu Pro Leu Cys Val Cys Pro Thr Leu Lys Gly Ala Ser Lys Ala Val
        115                 120                 125

Lys Gln Gln Gln Gly Gln Gln Leu Gln Gln Val Ile Ser Arg Ile Tyr
    130                 135                 140

Gln Thr Ala Thr His Leu Pro Lys Val Cys Asn Ile Pro Gln Val Ser
145                 150                 155                 160

Val Cys Pro Phe Gln Lys Thr Met Pro Gly Pro Ser Tyr
                165                 170


<210>  26
<211>  172
<212>  PRT
<213>  NONE

<400>  26

Met Ala Asn Lys Leu Phe Leu Val Ser Ala Thr Leu Ala Leu Phe Phe
1               5                   10                  15

Leu Leu Thr Asn Ala Ser Ile Tyr Arg Thr Val Val Glu Val Glu Glu
            20                  25                  30

Asp Asp Ala Thr Asn Pro Ala Gly Pro Phe Arg Ile Pro Lys Cys Arg
        35                  40                  45

Lys Glu Phe Gln Gln Ala Gln His Leu Arg Ala Cys Gln Gln Trp Leu
    50                  55                  60

His Lys Gln Ala Met Gln Ser Gly Ser Gly Pro Ser Trp Thr Leu Asp
65                  70                  75                  80

Gly Glu Phe Asp Phe Glu Asp Asp Met Glu Asn Pro Gln Ser Pro Gln
                85                  90                  95
```

Gln Arg Pro Pro Leu Leu Gln Gln Cys Cys Asn Glu Leu His Gln Glu
100 105 110

Glu Pro Leu Cys Val Cys Pro Thr Leu Lys Gly Ala Ser Lys Ala Val
115 120 125

Lys Gln Gln Gly Gln Gln Leu Gln Gln Val Ile Ser Arg Ile Tyr Gln
130 135 140

Thr Ala Thr His Leu Pro Lys Val Cys Asn Ile Pro Gln Val Ser Val
145 150 155 160

Cys Pro Phe Gln Lys Thr Met Pro Gly Pro Ser Tyr
165 170

<210> 27
<211> 158
<212> PRT
<213> NONE

<400> 27

Met Ala Asn Lys Ile Phe Leu Val Cys Ala Thr Leu Ala Phe Cys Val
1 5 10 15

Leu Leu Thr Asn Ala Ser Ile Tyr Arg Thr Val Val Glu Phe Asp Glu
20 25 30

Asp Asp Thr Thr Asp Gln Ile Gly Pro Phe Arg Pro Pro Gln Lys Cys
35 40 45

Gln Arg Glu Phe Gln Gln Glu Gln His Leu Arg Ala Cys Gln Gln Trp
50 55 60

Ile Arg Gln Gln Leu Ala Gly Ser Pro Phe Ser Glu Asn Gln Trp Gly
65 70 75 80

Pro Gln Gln Gly Pro Ser Leu Arg Glu Gln Cys Cys Asn Glu Leu Tyr
85 90 95

Gln Glu Asp Gln Val Cys Val Cys Pro Thr Leu Lys Gln Ala Ala Lys
100 105 110

Ser Val Arg Val Gln Gly Gln His Gly Pro Phe Gln Ser Thr Arg Ile
115 120 125

Tyr Gln Ile Ala Lys Asn Leu Pro Asn Val Cys Asn Met Lys Gln Ile
130 135 140

Gly Thr Cys Pro Phe Ile Ala Ile Pro Phe Phe Pro Pro Tyr
145 150 155

<210> 28
<211> 106
<212> PRT
<213> NONE

<400> 28

Gly Gly Pro Ser Trp Thr Leu Asp Gly Glu Phe Asp Phe Glu Asp Asp
1 5 10 15

Met Glu Asn Pro Gln Gly Pro Gln Gln Arg Pro Pro Leu Leu Gln Gln
20 25 30

Cys Cys Asn Glu Leu His Gln Glu Glu Pro Leu Cys Val Cys Pro Thr
35 40 45

Leu Lys Gly Ala Ser Lys Ala Val Lys Gln Gln Ile Gln Gln Gln Gly
50 55 60

Gln Gln Gln Gly Lys Gln Gln Met Val Ser Arg Ile Tyr Gln Thr Ala
65 70 75 80

Thr His Leu Pro Lys Val Cys Asn Ile Pro Gln Val Ser Val Cys Pro
85 90 95

Phe Gln Lys Thr Met Pro Gly Pro Ser Tyr
100 105

<210> 29
<211> 511
<212> PRT
<213> NONE

<400> 29

Met Ala Asn Thr Lys Ala Leu Leu Ser Leu Ser Phe Cys Phe Phe Leu
1 5 10 15

Leu Leu Gln Gly Thr Ser Ala Ile Ser Arg Ser Arg Ser Arg Ser Gln
20 25 30

Asp Glu Ser Tyr Arg Gln Gln Asn Gln Cys Gln Ile Asp Arg Ile Glu
35 40 45

Ala Arg Glu Pro Asp Thr Arg Val Glu Ala Glu Ala Gly Leu Ile Glu
50 55 60

```
Ser Trp Asn Pro Asn His Asn Gln Phe Gln Cys Ala Gly Val Ala Val
65                  70                  75                  80

Val Arg Tyr Thr Ile Gln Gln Asn Gly Leu His Leu Pro Ser Tyr Thr
                85                  90                  95

Asn Thr Pro Gln Leu Val Tyr Ile Val Lys Gly Arg Gly Ile Leu Gly
            100                 105                 110

Val Thr Phe Pro Gly Cys Pro Glu Thr Phe Glu Glu Ser Gln Arg Gly
        115                 120                 125

Gln Gly Gln Gly Gln Ser Gln Gly Ser Gln Pro Asp Arg His Gln Lys
    130                 135                 140

Leu Arg His Val Arg Glu Gly Asp Ile Val Ala Ile Pro Ala Gly Val
145                 150                 155                 160

Ala Tyr Trp Ser Tyr Asn Asn Gly Asp Gln Gln Leu Val Phe Val Ser
                165                 170                 175

Leu Leu Asp Thr Ser Asn Val Asn Asn Gln Leu Asp Asp Asn Pro Arg
            180                 185                 190

Arg Phe Tyr Leu Ala Gly Asn Pro Glu Asp Glu Phe Glu Gln Leu Arg
        195                 200                 205

Arg Glu Gly Gly Arg Gly Ala Arg Phe Asp Glu Arg Ile Arg Glu Arg
    210                 215                 220

Ser Glu Gly Arg His Ser Ser Glu Asn Tyr Arg Asn Ile Phe Lys Gly
225                 230                 235                 240

Phe Asn Ser Arg Tyr Leu Glu Glu Ala Phe Asn Val Asp Ser Glu Thr
                245                 250                 255

Val Lys Arg Leu Gln Gly Gln Asn Asp Asp Arg Asn Ser Ile Ile Arg
            260                 265                 270

Val Lys Gly Thr Leu Asp Leu Val Ser Pro Leu Arg Ser Ser Gln Glu
        275                 280                 285

His Gln Arg Glu Glu Arg Tyr Glu Asp Glu Arg Gln Arg Glu Ile Glu
    290                 295                 300

Gln Glu Arg Arg Arg Met Ser Arg Gly Gly Arg Tyr Glu Ala Asn Gly
305                 310                 315                 320
```

Leu Glu Glu Thr Phe Cys Ser Met Arg Leu Arg Glu Asn Ile Gly Asp
325 330 335

Pro Ser Arg Ala Asp Val Phe Thr Pro Gln Ala Gly Arg Ile Ser Thr
340 345 350

Val Asn Ser Tyr Asn Leu Pro Ile Leu Arg Phe Leu Gln Leu Ser Ala
355 360 365

Glu Arg Gly Val Leu Tyr Lys Asn Ala Ile Tyr Thr Pro His Trp Asn
370 375 380

Val Asn Ala His Ser Val Met Tyr Val Leu Arg Gly Arg Ala Arg Val
385 390 395 400

Gln Val Val Asn His Met Gly Gln Lys Cys Phe Asp Gly Glu Val Arg
405 410 415

Gln Gly Gln Ile Val Thr Val Pro Gln Asn His Ala Val Val Lys Gln
420 425 430

Ala Ser Ser Asp Gly Phe Glu Trp Val Ser Phe Lys Thr Asn Asp Asn
435 440 445

Ala Trp Val Ser Pro Leu Ala Gly Arg Thr Ser Val Ile Arg Ala Leu
450 455 460

Pro Glu Ala Val Leu Ala Asn Ala Phe Gln Ile Ser Arg Asp Gln Ala
465 470 475 480

Arg Asn Leu Lys Tyr Asn Arg Glu Glu Thr Val Leu Leu Thr Ser Ser
485 490 495

Thr Ser Ser Arg Arg Glu Asp Arg Tyr Glu Arg Arg Ala Thr Ala
500 505 510

<210> 30
<211> 491
<212> PRT
<213> NONE

<400> 30

Met Ala Arg Ser Ser Thr Ser Leu Leu Cys Phe Thr Leu Phe Ser Leu
1 5 10 15

Leu Leu Ser His Ala Cys Phe Ala Gln Ile Glu Gln Met Pro Gln Arg
20 25 30

```
Ser Gln Arg Gly Gly Gln Gln Arg Gln Gln His Arg Trp Gln Ser Gln
        35                  40                  45

Cys Gln Phe Gln Arg Leu Asn Ala Arg Gln Pro Asn Arg Arg Val Glu
    50                  55                  60

Cys Glu Ala Gly Val Ser Glu Tyr Trp Asp Ile Gln Asn Thr Glu Asp
65                  70                  75                  80

Asp Glu Leu His Cys Ala Gly Val Glu Thr Ala Arg His Thr Ile Gln
                85                  90                  95

Arg Arg Gly Leu Leu Leu Pro Ser Phe Leu Asn Ala Pro Met Met Phe
            100                 105                 110

Tyr Val Ile Gln Gly Arg Gly Ile His Gly Ala Val Ile Pro Gly Cys
        115                 120                 125

Pro Glu Thr Phe Glu Arg Gly Thr Ser Ser Pro Ser Ser Arg Gly Tyr
    130                 135                 140

Arg Ser Glu Gly Ala Ser Ser Asp Glu Gln His Gln Lys Val Arg Glu
145                 150                 155                 160

Ile Lys Glu Gly Asp Met Val Ala Met Pro Ala Gly Val Ala Asp Trp
                165                 170                 175

Val Tyr Asn Asn Gly Asp Ser Pro Leu Val Leu Ile Ala Phe Val Asp
            180                 185                 190

Val Gly Asn Gln Ala Asn Gln Leu Asp Gln Phe Ser Arg Arg Phe His
        195                 200                 205

Leu Ala Gly Asn Pro His Arg Glu Gln Lys Thr Gln Gln Gln Val Arg
    210                 215                 220

Ala Arg Ser Gln Ser Arg Ser Gln Leu Arg Arg Glu Ser Gly Glu Gln
225                 230                 235                 240

Thr Pro Asn Gly Asn Ile Phe Ser Gly Phe Asp Thr Arg Ile Leu Ala
                245                 250                 255

Glu Ser Phe Asn Val Asp Thr Glu Leu Ala His Lys Leu Gln Asn Arg
            260                 265                 270

Asp Asp Met Arg Glu Arg Ile Val Arg Val Arg Gly Glu Asp Leu Gln
```

275 280 285

Ile Ile Ala Pro Ser Arg Ile Gln Glu Glu Glu Arg Arg His Tyr Ser
290 295 300

Arg Asp Asn Gly Leu Glu Glu Thr Phe Cys Thr Leu Arg Leu Arg Gln
305 310 315 320

Asn Ile Asp Arg Pro Ser Gln Ala Asp Ile Phe Asn Pro Arg Gly Gly
325 330 335

Arg Leu Asn Thr Leu Asn Asn Tyr Asn Leu Pro Ile Leu Arg Phe Leu
340 345 350

Gln Leu Thr Ala Glu Arg Gly Val Leu Tyr Lys Asn Gly Met Met Ala
355 360 365

Pro His Phe Asn Leu Asp Ser His Ser Val Ile Tyr Val Thr Arg Gly
370 375 380

Ser Ala Arg Leu Gln Val Val Asp Asp Asn Gly Arg Asn Val Phe Asp
385 390 395 400

Gly Glu Leu Arg Glu Gly Gln Ile Phe Val Val Pro Gln Asn Phe Ala
405 410 415

Val Val Lys Lys Ala Ser Ala Gln Gly Phe Glu Trp Ile Ala Val Lys
420 425 430

Thr Asn Asp Asn Ala Met Arg Asn Pro Leu Ala Gly Lys Val Ser Ala
435 440 445

Met Arg Ala Met Pro Asp Asp Val Leu Ala Asn Ala Phe Gln Ile Ser
450 455 460

Arg Glu Gln Ala Arg Arg Leu Lys Tyr Gly Arg Asp Glu Ile Ser Val
465 470 475 480

Phe Ser Pro Ser Ser Gln Gln Thr Arg Tyr Glu
485 490

<210> 31
<211> 493
<212> PRT
<213> NONE

<400> 31

Met Ala Ser Thr Pro Leu Leu Leu Ser Leu Ser Leu Cys Phe Leu Val

```
1               5                   10                  15

Leu Leu His Gly Cys Ser Ala Arg Ser Arg Thr Ala Met Tyr Gly Asp
            20                  25                  30

Gln Asn Glu Cys Gln Leu Asn Arg Leu Glu Ala Cys Glu Pro Asp His
        35                  40                  45

Arg Val Glu Cys Glu Gly Gly Met Ile Glu Ser Trp Asn Pro Asn His
    50                  55                  60

Glu Gln Phe Gln Cys Ala Gly Val Ala Leu Leu Arg Leu Thr Ile Gln
65                  70                  75                  80

Pro Asn Gly Leu His Leu Pro Ser Tyr Thr Asn Gly Pro Gln Leu Ile
                85                  90                  95

His Val Ile Arg Gly Arg Gly Val Leu Gly Thr Leu Phe Pro Gly Cys
            100                 105                 110

Ala Glu Thr Phe Glu Glu Ala Gln Val Ser Val Gly Gly Gly Arg Ser
        115                 120                 125

Ser Gln Arg Asp Arg His Gln Lys Thr Arg Gln Ile Lys Glu Gly Asp
    130                 135                 140

Ile Ile Ala Ile Pro Ala Gly Met Ala Tyr Trp Cys Asn Asn Asp Gly
145                 150                 155                 160

Asp Gln Pro Leu Ile Thr Val Asn Leu Ile His Ile Ile Asn Asn Gln
                165                 170                 175

Asn Gln Leu Asp Met Ser Pro Arg Arg Phe Tyr Ile Ala Gly Asn Pro
            180                 185                 190

His Gln Glu Phe Pro Gln Ser Met Met Thr Gln Gln Gly Arg Arg Tyr
        195                 200                 205

Ser Glu Glu Arg Arg Glu Arg Ile Glu Glu Glu Lys Glu Gln Glu Gly
    210                 215                 220

Leu Pro Asn Asn Val Phe Arg Gly Phe Ser Val Asn Leu Ile Gln Glu
225                 230                 235                 240

Ala Phe Asn Val Asp Ser Glu Thr Ala Arg Lys Ile Gln Asn Gln Asp
                245                 250                 255
```

```
Asp Phe Arg Gly Ser Ile Ile Arg Val Glu Gly Lys Leu Asp Leu Val
            260                 265                 270

Lys Pro Gln Arg Ser Arg Gln Glu Gln Glu Gln Glu Met Arg Arg Gln
        275                 280                 285

Glu Leu Gln Gln Thr Glu Arg Glu His Ala Arg Leu Ser Gly Arg Asp
    290                 295                 300

Tyr Asn Gly Leu Glu Glu Asn Ile Cys Thr Met Arg Leu Arg Glu Ser
305                 310                 315                 320

Met Gly Asp Pro Ala Arg Ala Asp Val Phe Ser Pro Gln Ala Gly Arg
                325                 330                 335

Leu Thr Thr Val Asn Ser Tyr Asn Leu Pro Ile Leu Ser Phe Leu Arg
            340                 345                 350

Leu Ser Ala Glu Arg Gly Phe Leu Tyr Lys Asn Ala Met Tyr Ala Pro
        355                 360                 365

Gln Tyr Thr Met Asn Ala His Asn Ile Ile Tyr Ala Ile Arg Gly Ser
    370                 375                 380

Cys Arg Cys Gln Val Val Asp Asn Asn Gly Arg Ser Val Phe Glu Gly
385                 390                 395                 400

Glu Ile Arg Gln Gly Gln Ala Leu Thr Val Pro Gln Asn Phe Ala Val
                405                 410                 415

Val Lys Met Ala Glu Lys Glu Gly Phe Glu Trp Val Ser Phe Lys Thr
            420                 425                 430

Asn Asp Arg Ala Gln Val Asn Gln Leu Ala Gly Lys Ile Ser Phe Met
        435                 440                 445

Arg Ala Met Pro Glu Asp Val Ile Ala Asn Ser Tyr Gln Ile Ser Arg
    450                 455                 460

Glu Gln Ala Arg Arg Leu Lys Tyr Asn Arg Glu Glu Ser Ser Leu Phe
465                 470                 475                 480

Thr Thr Ser His Gln Gly Ile Arg Ala Ala Val Thr Ala
                485                 490
```

<210> 32
<211> 142
<212> PRT

<213> NONE

<400> 32

Met Ala Lys Ile Ser Ser Ser Thr Leu Ala Leu Phe Ala Ala Leu Met
1 5 10 15

Leu Val Ala His Ala Val Ala Phe Arg Thr Thr Ile Thr Thr Val Glu
20 25 30

Thr Asp Asp Val Glu Asn Tyr Ser Arg Ser Gly Ser Glu Gln Glu Cys
35 40 45

Arg Arg Gln Arg Gln Asp Leu Asn His Cys Arg Met Tyr Met Arg Glu
50 55 60

Lys Met His Gly Arg Phe Glu Glu Glu Asp Glu Ile Glu Asn Tyr Ser
65 70 75 80

Gln His Leu Asp Gln Cys Cys Ser Gln Leu Arg Asn Val Asn Glu Arg
85 90 95

Cys Arg Cys Pro Ala Leu Glu Met Glu Ile Gln Lys Glu Gln Gly Gln
100 105 110

Asp Lys Gln Arg Met Met Glu Ser Ala Arg Asn Ile Pro Ser Met Cys
115 120 125

Gly Met Gln Pro Arg Thr Cys Gln Phe His Ser Arg Tyr Tyr
130 135 140

<210> 33
<211> 169
<212> PRT
<213> NONE

<400> 33

Met Ala Lys Leu Met Ser Leu Ala Ala Val Ala Thr Ala Phe Leu Phe
1 5 10 15

Leu Ile Val Val Asp Ala Ser Val Arg Thr Thr Val Ile Ile Asp Glu
20 25 30

Glu Thr Asn Gln Gly Arg Gly Gly Gly Gln Gly Gly Gln Gly Gln Gln
35 40 45

Gln Ser Cys Glu Gln Gln Ile Gln Gln Gln Asp Phe Leu Arg Ser Cys
50 55 60

Gln Gln Phe Met Trp Glu Lys Val Gln Arg Gly Gly Arg Ser His Tyr
65 70 75 80

Tyr Asn Gln Gly Arg Gly Gly Gly Glu Gln Ser Gln Tyr Phe Asp Ser
85 90 95

Cys Cys Asp Asp Leu Lys Gln Leu Ser Thr Gly Cys Thr Cys Arg Gly
100 105 110

Leu Glu Arg Ala Ile Gly Gln Met Arg Gln Glu Ile Gln Gln Gln Gly
115 120 125

Gln Gln Gln Glu Val Gln Arg Trp Ile Gln Gln Ala Lys Gln Ile Ala
130 135 140

Lys Asp Leu Pro Gly Gln Cys Arg Thr Gln Pro Ser Gln Cys Gln Phe
145 150 155 160

Gln Gly Gln Gln Gln Ser Ala Trp Phe
165

<210> 34
<211> 168
<212> PRT
<213> NONE

<400> 34

Met Ala Lys Leu Met Ser Leu Ala Ala Val Ala Thr Ala Phe Leu Phe
1 5 10 15

Leu Ile Val Val Asp Ala Ser Val Arg Thr Thr Val Ile Ile Asp Glu
20 25 30

Asp Thr Asn Gln Gly Arg Gly Gly Gln Gly Gly Gln Gly Gln Gln Gln
35 40 45

Gln Cys Glu Lys Gln Ile Gln Glu Gln Asp Tyr Leu Arg Ser Cys Gln
50 55 60

Gln Phe Leu Trp Glu Lys Val Gln Lys Gly Gly Arg Ser Tyr Tyr Tyr
65 70 75 80

Asn Gln Gly Arg Gly Gly Gly Gln Gln Ser Gln His Phe Asp Ser Cys
85 90 95

Cys Asp Asp Leu Lys Gln Leu Arg Ser Glu Cys Thr Cys Arg Gly Leu
100 105 110

Glu Arg Ala Ile Gly Gln Met Arg Gln Asp Ile Gln Gln Gln Gly Gln
115 120 125

Gln Gln Glu Val Glu Arg Trp Val Gln Gln Ala Lys Gln Val Ala Arg
130 135 140

Asp Leu Pro Gly Gln Cys Gly Thr Gln Pro Ser Arg Cys Gln Leu Gln
145 150 155 160

Gly Gln Gln Gln Ser Ala Trp Phe
165

<210> 35
<211> 355
<212> PRT
<213> NONE

<400> 35

Met Asp Met Thr Asn Asn Ser Arg Arg Glu Ala Gly Lys Ala Trp Glu
1 5 10 15

Lys Gln Ser Gly Lys Glu Glu Asp Asp Asp Ser Ser Met Asp Arg Leu
20 25 30

Asn Tyr Leu Pro Glu Pro Leu Ile Arg His Ile Leu Thr Leu Met Asp
35 40 45

Thr Arg Ser Ala Ser Cys Ser Asp Leu Ser Thr Ile Pro Lys Met Glu
50 55 60

Met His Leu Glu Thr Pro Ile Cys Ser Arg Phe Ser Ser Lys Asn Met
65 70 75 80

Ser Asn Tyr Leu Ser Ser Asp Leu Gln Lys Val Arg Gly Lys Gly Ser
85 90 95

Val Val Ser Leu Ser Ser Ile Glu Ser Gln Gln Asp Leu Phe Phe Cys
100 105 110

Ser Phe Phe Ala Gly Glu Glu Glu Val Ile Ile Asp Pro Phe Ser Asn
115 120 125

Leu Pro Cys Leu Lys Gln Leu Val Leu Asn Thr Ile Phe Asn Asn Gly
130 135 140

Asn Asp Asp Ile Arg Leu Arg Ile Ser Gly Leu Gln Leu Leu Ser Leu
145 150 155 160

Ser Leu Ser Asp Ser Gly Phe His Lys Met Glu Ile Tyr Thr Pro Lys
165 170 175

Leu Lys His Phe Thr Leu Leu Tyr Ser His His Leu Val Glu Phe Thr
180 185 190

Glu Leu Ala Leu Pro Ser Leu Asp Arg Ala Asp Ile His Val Glu Tyr
195 200 205

Leu Arg Val Leu Ser Glu Glu Phe Thr Glu Leu Ala Leu Pro Ser Leu
210 215 220

Asp Arg Ala Asp Ile His Val Glu Tyr Leu Arg Val Leu Ser Glu Asp
225 230 235 240

Glu Gly Thr Asp Gly Glu Val Glu Asp Glu Gly Met Asp Gly Glu Val
245 250 255

Glu Asp Glu Gly Thr Asp Gly Glu Phe Asp Val Glu Asp Trp Asn Glu
260 265 270

Tyr Thr Lys Gln Gln Met Val Pro Phe Phe His Gly Leu Ser Asn Ala
275 280 285

Thr Ser Leu Val Leu Cys Ser Arg Thr Ile Gln Leu Leu Trp Lys Ile
290 295 300

Ser Tyr Tyr Leu Glu Asp Gln Pro Ser Pro Phe Thr Arg Leu Lys Ser
305 310 315 320

Leu Ile Val Gly Ser Pro Ser Arg Ser Pro Asp Asp Val Leu Ser Ser
325 330 335

Leu Leu Asn Tyr Phe Leu Lys Gly Ser Ser Asp Met Lys Pro Val Val
340 345 350

Lys Phe Ser
355

<210> 36
<211> 368
<212> PRT
<213> NONE

<400> 36

Met Thr Ser Leu Arg Pro Trp Lys Ser Arg Ala Ala Ile Val Asp Gly
1 5 10 15

```
Gly Lys Cys Gly Leu Lys Val Thr Gln Leu Pro Met Met Val Ala Leu
            20                  25                  30

Cys Thr Thr Met Leu Phe Ile Val Tyr Arg Thr Thr Leu Tyr Gln Tyr
        35                  40                  45

Glu Gln Thr Glu Ile Glu Gly Lys Leu His Pro Phe Asp Ser Ile Lys
    50                  55                  60

Glu Ser Ala Leu Ala Ser Gly Leu Leu Ser Asn Leu Pro Arg Gly Ile
65                  70                  75                  80

Val Arg Pro His Ser Asp Met Glu Met Lys Pro Leu Trp Ser Ser Gly
                85                  90                  95

Ser Ser Arg Ser Lys Thr Glu Asp Asn Ala Ser Ser His His Tyr Leu
            100                 105                 110

Leu Ala Met Ser Val Gly Ile Asn Gln Lys Ser His Ile Asp Thr Val
        115                 120                 125

Val Gln Lys Val Leu Asn Phe Phe Gly Phe Asn Gly Gln His Val Leu
    130                 135                 140

Arg Trp Phe Ala Lys Arg Phe Leu His Pro Ala Val Val Ser Ile Tyr
145                 150                 155                 160

Asp Tyr Ile Phe Leu Trp Asp Glu Asp Leu Gly Val Gln Asn Phe Asp
                165                 170                 175

Pro Gly Arg Arg Val Tyr Asp Val Arg Gly Asn Ile Lys Cys Ser Glu
            180                 185                 190

Ala Ser Glu Gly Pro Pro Cys Thr Gly Phe Val Glu Gly Met Ala Pro
        195                 200                 205

Val Phe Ser Arg Ser Ala Trp Tyr Cys Ala Trp His Leu Ile Gln Asn
    210                 215                 220

Asp Leu Val His Gly Trp Gly Met Asp Ile Lys Leu Gly Tyr Cys Ala
225                 230                 235                 240

Gln Gly Asp Arg Thr Lys Asn Val Gly Ile Val Asp Ser Glu Tyr Val
                245                 250                 255

Val His Lys Ala Ile Gln Thr Leu Gly Gly Gly Ser Gly Thr Pro Glu
            260                 265                 270
```

Lys Lys Val Ser Ser Asn Asp Glu Ser Thr Thr Lys Val Thr Phe Thr
275 280 285

Met Lys Cys Ile Cys Ser Ser His Cys Phe Ser Ser Leu Ile Thr Phe
290 295 300

His Leu Gln Gln Lys His Ser Gly Val Asp Pro Arg Met Glu Ile Arg
305 310 315 320

Arg Gln Ser Thr Trp Glu Leu Gln Val Phe Lys Asn Arg Trp Asn Lys
325 330 335

Ala Ile Arg Glu Asp Lys Phe Trp Ala Asp Pro Phe Arg Ser Ile Arg
340 345 350

Arg Gln Lys Ser Gly Trp Leu Glu Lys Glu Lys Arg Arg His Ser Gln
355 360 365

<210> 37
<211> 368
<212> PRT
<213> NONE

<400> 37

Met Glu Thr Ala Ala Leu Phe Leu Ile Ser Ala Leu Ala Val Val Val
1 5 10 15

Thr Val Phe Leu Leu Ser Pro Ser Leu Ile Val Ser Phe Val Pro Ser
20 25 30

Phe Ile Thr Lys Gln Arg Val Thr Cys Pro Leu Asp Ile Val Ile Asp
35 40 45

Ile Leu Phe Arg Leu Pro Val Lys Thr Leu Ile Gln Phe Lys Cys Val
50 55 60

Ser Lys His Trp Asn Gln Ile Leu Val Ser Met His Leu Met Arg Ser
65 70 75 80

Gln Gln Ala Pro Asn Arg Ile Ile Cys Tyr Tyr Glu Ile Pro Asn Tyr
85 90 95

Tyr His Met Leu Asn Pro Pro Ala Asp Gln Asp Gln Ser Lys Gln Phe
100 105 110

Asp Tyr His Leu Arg Asn Pro Ala Thr Arg Gln Val Gln His Leu Pro
115 120 125

```
Glu Pro Pro Phe Glu Pro Pro Ala Arg His Phe Asp Leu Pro Asn His
    130                 135                 140

Gly Glu Leu Gly Phe Asn Asp Asp Phe Gly Val Gly Leu Asp Leu Val
145                 150                 155                 160

Ser Asn Val Val Lys Val Val Leu Ile Arg Tyr Tyr Cys Phe Ala Gly
                165                 170                 175

Ile Asp Gly His Ala Val Ser Lys Phe Thr Ser Pro Val Tyr Val Tyr
            180                 185                 190

Thr Leu Asp Gly Gly Trp Arg Lys Leu Gly Val Glu Cys Pro Tyr Pro
        195                 200                 205

Glu Asn Trp Leu Ser Leu Asp Val Trp Met Leu Arg Ser Asn Tyr Glu
    210                 215                 220

Glu Glu Leu Cys Trp Ile Lys Asp Ser Thr Gly Ile Gly Pro Phe Pro
225                 230                 235                 240

Arg Glu Leu Phe Val Ile Gly Cys Trp Lys Asp Asp Leu Leu Val Ala
                245                 250                 255

Gly Leu Val Glu Asp Gln Glu Asn Leu Val Val Tyr Asp Met Val Lys
            260                 265                 270

Gln Glu Ile Lys Val Val Met Pro Asp Val Val Val Asp Arg Leu Phe
        275                 280                 285

Thr Tyr Thr Glu Thr Leu Ser Phe Leu Arg Ser Ser His His Leu Cys
    290                 295                 300

Asn Trp Leu Asp Leu Gly Val Ala Cys Thr Trp Gln Asn Ser Glu Lys
305                 310                 315                 320

Ile Glu Gly Gly Glu Phe Leu Tyr Ser Gly Leu Pro Ser Ala Ile Gly
                325                 330                 335

Ile Gly Asn Glu Ala Pro Arg Ile Ser Ser Asp Gln His Ser Gly Glu
            340                 345                 350

Asp Asp Gly Gly Gln Glu Ala Leu Leu Ala Gly Arg Lys Val Pro Leu
        355                 360                 365
```

<210> 38
<211> 381

<212> PRT
<213> NONE

<400> 38

```
Met Val Met Ser Phe Met Ile Leu Pro Trp Arg Pro Glu Leu Val Phe
1               5                   10                  15

Ile Gly Thr Ser Ala Met Gly Ser Ile Gln Val Thr Gly Ser Phe Ala
            20                  25                  30

Ala Val Trp Arg Ala Asp Leu Asn Lys Ser Ser Asp Ala Ser Ser Ser
        35                  40                  45

Ala Phe Lys Gln Phe Gln Cys Gln Leu Ser Ser Trp Val Met Lys Val
    50                  55                  60

Ser Ser Ile Ile Lys Tyr Glu His Lys Lys Met Glu Val Leu His Pro
65                  70                  75                  80

Glu Ile Val Phe Phe Asn Pro Leu His Ser Glu Glu Lys Asn Ala Ile
                85                  90                  95

Leu Cys Tyr Asp Val Arg Thr Glu Glu Leu Glu Leu Phe Ala Lys Leu
            100                 105                 110

Glu Gly Lys Pro Asp Val Tyr Arg Leu Gln Val Phe Gln Pro Met Val
        115                 120                 125

Ser Cys Trp Ala Thr Pro Ile Pro Arg Tyr Glu Ala Leu Arg Gly Met
    130                 135                 140

Tyr Asp Gly Ser Tyr Ser Phe Trp Val Gln Ser Thr Ser Glu Ser Lys
145                 150                 155                 160

Ser Pro Leu Leu Asn Ile Cys Lys Phe Met Lys Ser Thr Phe Tyr Lys
                165                 170                 175

Asp Tyr Met Lys Asn Val Thr Glu Glu Ala Thr Leu Asp Met Leu Ala
            180                 185                 190

Glu Ile Ile Gln Arg Arg Lys Lys Lys Ile Ser Glu Ile Lys Glu Glu
        195                 200                 205

Val Thr Cys Lys Thr Leu Ala Glu Leu Leu Tyr Pro Pro Val Ser Ile
    210                 215                 220

Glu Ser Val Cys Thr Arg Asn Met Val Val Pro Ser Arg Lys Arg Lys
225                 230                 235                 240
```

Leu Gly Gly Met Val Ile Thr Glu Arg Lys Gln Pro Arg Phe Val Asp
245 250 255

Asp Lys Glu Ala Gly Phe Lys Asp Val Val Ser Tyr Val Ala Ala Val
260 265 270

Gly Asn Pro Lys Glu Val Glu Gln Gly Leu Met Cys Glu Thr Glu Gln
275 280 285

Leu Pro Val Gln His Pro Asn Phe Glu Asn Phe Val Ser Asn Trp Gln
290 295 300

Trp Asn Thr His Thr His Leu Phe Pro Asp Asp Lys Glu Ala Asp Phe
305 310 315 320

Glu Asp Asp Asp Ser Tyr Met Ala Ala Leu Val Lys Trp Lys Glu Leu
325 330 335

Lys His Gly Phe Tyr Ile Glu Thr Trp Ala Glu Ile Pro His Pro Phe
340 345 350

Gly Gly Arg Thr Leu Glu Asp Gln Gly Lys Ala Met Leu Ala Cys Tyr
355 360 365

Phe Gly Lys Gly Arg Leu Met Phe Lys Asp Val Val Gly
370 375 380

<210> 39
<211> 384
<212> PRT
<213> NONE

<400> 39

Met Lys Gln Lys Leu Ser Ser Arg Leu Thr Asn Phe Phe Ser Val Asp
1 5 10 15

Ala Phe Gly Ala Ala Arg Arg Leu Asn Gln Pro Pro Leu Pro Asp Val
20 25 30

Asp Val Arg Glu Glu Tyr Ala Asn Ala Phe Arg Thr Glu Ser Tyr Leu
35 40 45

Glu Phe Trp Thr Arg Val Phe Thr Val Ser Asp Gly Gly Gly Ser Ala
50 55 60

Thr Gly Ile Pro Ala Asn Ser Thr Asn Ser Thr Ala Ala Arg Leu Ser
65 70 75 80

```
Ser Tyr Arg Leu Phe Val Glu His Leu Leu Asp Pro Asp Gln Leu Thr
                85                  90                  95

Val Thr Arg Val Leu Asp Ala Ala His Ile Pro Ser Ser Ala His Ser
            100                 105                 110

Leu Leu Ala Gln Tyr Phe Ala Gln Thr Ala Asp Ala Ser Ser Ser Cys
        115                 120                 125

Gly Ser Leu Leu Lys Asp Ile Asn Arg Val Arg Val Ala Ile Arg Thr
    130                 135                 140

Leu Lys Ser Ala Leu Lys Ser Leu Glu Thr Gly Ser Ile His Asn Ala
145                 150                 155                 160

Asp Asp Gln Phe Gln Val Ile Leu Ser Gln Leu Ala Thr Phe Ala Asp
                165                 170                 175

Thr His Asn Pro Phe Asn Gln Ser Asn Arg Ser Pro Val Met Val Arg
            180                 185                 190

Ser Ala Gln Ile Asn Cys Thr Lys Leu Leu Lys Pro Leu Glu Ser Ile
        195                 200                 205

Arg Asp Lys Ala His Ala Asn Val Arg Leu Arg Ala Arg Leu Lys His
    210                 215                 220

Gly Ser Ala Met Phe Leu Val Ala Leu Thr Ala Ser Leu Thr Val Ile
225                 230                 235                 240

Leu Ala Thr His Ala Leu Ala Leu Leu Ile Ala Ala Pro Thr Met Val
                245                 250                 255

Val Ala Ser Ile Glu Leu Val Ser Thr Lys Lys Leu Asn Lys Val Val
            260                 265                 270

Ser Glu Leu Asp Val Ala Ala Lys Gly Met Tyr Ile Leu Ser Arg Asp
        275                 280                 285

Leu Glu Thr Ile Asn Gly Leu Val Ala Arg Leu Ser Asp Glu Leu Glu
    290                 295                 300

His Met Cys Gly Ala Val Lys Phe Trp Leu Asp Arg Gly Glu Thr Trp
305                 310                 315                 320

Val Arg Ala Ala Asn Gly Glu Val Trp Lys Glu Leu Arg Lys Asn Glu
```

325 330 335

Arg Gly Phe Ser Gln His Leu Asp Glu Leu Glu Glu His Leu Tyr Leu
340 345 350

Cys Phe Met Thr Ile Asn Arg Ala Arg Asn Leu Val Val Lys Glu Ile
355 360 365

Leu Asp Pro Gly Arg Ala Met Arg Pro Asp Pro Asn Ile Leu Ser Lys
370 375 380

<210> 40
<211> 386
<212> PRT
<213> NONE

<400> 40

Met Ala Ser Asp Ala Lys Thr Glu Pro Pro Thr His Lys Ser Pro Ser
1 5 10 15

Lys Asn Val Ala Ile Ile Phe Gly Val Thr Gly Leu Val Gly Arg Glu
20 25 30

Ile Ala Lys Lys Leu Ile Ser Ile Thr Glu Ser Trp Thr Val Tyr Gly
35 40 45

Val Ser Arg Arg Pro Asp Lys Leu Pro Ile Ser Ser Pro Asn Tyr His
50 55 60

Phe Ile Pro Cys Asp Leu Leu Asn Pro Leu Asp Thr Gln Thr Lys Leu
65 70 75 80

Ser Pro Ile Ser Asn Leu Ile Thr His Leu Phe Trp Val Thr Trp Ala
85 90 95

Ala Asn Phe Pro Leu Asp Ser Lys Gln Cys Cys Asp Glu Asn Arg Ser
100 105 110

Met Met Ser Asn Ala Leu Gln Pro Ile Leu Ser Ser Asn Ser Gln Ser
115 120 125

Leu Lys His Val Ser Leu Gln Thr Gly Leu Lys His Tyr Ile Ser Leu
130 135 140

Arg Asp Phe Val Asn Gly Gly Gly Ile Arg Arg Phe Tyr Asp Glu Asp
145 150 155 160

Cys Pro Arg Ala Glu Asp Gly Phe Asn Phe Tyr Tyr Ser Leu Glu Asp

165 170 175

Leu Leu Lys Glu Lys Leu Leu Glu Gly Ser Gly Ala Gly Trp Ser Val
180 185 190

Ile Arg Pro Gly Leu Val Met Gly Ser Ser Thr Thr Ser Ile Tyr Asn
195 200 205

Val Ile Gly Ser Leu Cys Val Tyr Gly Val Ile Cys Arg Arg Met Asp
210 215 220

Leu Pro Phe Val Phe Gly Gly Thr Lys Glu Cys Trp Glu Glu Ala Tyr
225 230 235 240

Ile Asp Gly Ser Asp Ser Gly Leu Val Ala Glu His His Ile Trp Ala
245 250 255

Ala Thr Asp Glu Arg Val Arg Ser Thr Ala Glu Arg Ala Leu Asn Ser
260 265 270

Val Asn Gly Ser Ser Phe Ser Trp Lys Gly Ile Trp Ala Val Ile Ala
275 280 285

Glu Lys Ile Gly Val Glu Ala Ser Glu Glu Gly Leu Asp Glu Gly Phe
290 295 300

Arg Phe Ala Ala Ala Met Gly Gly Leu Gly Gly Val Trp Ala Glu Ile
305 310 315 320

Val Lys Glu Glu Gly Leu Val Glu Thr Glu Met Glu Glu Leu Ala Asn
325 330 335

Trp Glu Phe Leu Asp Val Leu Phe Arg Phe Pro Ile Lys Leu Leu Gly
340 345 350

Ser Arg Glu Lys Ser Asp Arg Leu Gly Phe Thr Ala Arg Arg Glu Thr
355 360 365

Ala Glu Ser Ala Ala Tyr Trp Ile Asp Ser Met Arg Arg Glu Lys Leu
370 375 380

Ile Pro
385

<210> 41
<211> 413
<212> PRT
<213> NONE

<400> 41

Met Ala Ser Ile Ser Ser Ser Val Ile Asn Arg Phe Leu Ser Leu Phe
1 5 10 15

Leu Ile Leu Leu His Leu Gly Cys Phe Val Phe Leu Ser Arg Asp Gln
20 25 30

Ser Ala Ser His Pro Thr Arg Lys Arg Lys Ser Ser Ser Ser Asp Asp
35 40 45

Asp Tyr Lys Asp Asp His Ser Pro Pro Ala Ala Ser Arg Arg Arg Arg
50 55 60

Ser Ser Pro Leu Leu Ser Ser Ser Trp Ser Tyr Ile Lys Arg Val Phe
65 70 75 80

Thr Cys Lys Ser Ile Tyr Asn Ser Leu Pro Pro Pro Leu Pro Pro Pro
85 90 95

Ile Ser Pro Ala Gln Thr Ser Ala Arg Ser Ser Gln Lys Ser Ser Leu
100 105 110

Leu Ile Ala Ile Glu Asn Asp Ala Val Phe Pro Ala Lys Ile Ser His
115 120 125

His Pro Pro His Pro Ile Ser Ala Ser Pro Glu Ser Asp Ile Ser Ser
130 135 140

Ala Asp Arg Phe Pro Leu Arg Asn Asp Ile Phe Pro Cys Thr Val Cys
145 150 155 160

Gly Glu Val Leu Gln Lys Pro His Ile Leu Glu Gln His Leu Ala Val
165 170 175

Lys His Ala Val Ser Glu Leu Arg Asp Gly Asp Ser Gly Asn Asn Ile
180 185 190

Val Arg Ile Ile Phe Lys Ala Gly Trp Asn Ser Ala Thr Lys Ser Pro
195 200 205

Glu Ile His Arg Ile Leu Lys Ile His Asn Ser Pro Lys Ile Leu Thr
210 215 220

Arg Phe Glu Glu Tyr Arg Glu Phe Val Lys Ser Lys Ala Ser Lys Ile
225 230 235 240

```
Ser Asn Val Ala Gly Gly Gly Gly Arg Ile Arg Asp Glu Arg Cys Ile
                245                 250                 255

Ala Asp Gly Asn Glu Leu Leu Arg Phe His Cys Ala Thr Phe Val Cys
            260                 265                 270

Asp Leu Gly Leu Asn Gly Gly Gly Gly Cys Gly Ala Ala Gly Trp Pro
        275                 280                 285

Thr Ser Ser Leu Cys Asn Gln Gln Tyr Cys Ser Val Cys Gly Ile Ile
    290                 295                 300

Lys Ser Gly Phe Ser Pro Lys Met Asp Gly Ile Ser Thr Leu Ser Thr
305                 310                 315                 320

Ser Trp Arg Ala His Ala Ala Ile Pro Ala Glu Ile Glu Glu Glu Phe
                325                 330                 335

Lys Phe Met Asn Ile Lys Arg Ala Met Leu Val Cys Arg Val Ile Ala
            340                 345                 350

Gly Arg Val Gly Ser Glu Met Glu Glu Ala Asp Lys Glu Gly Cys Gly
        355                 360                 365

Tyr Asp Ser Leu Val Gly Arg Gly Arg Gly Gly Gly Gly Gly Val His
    370                 375                 380

Leu Gly Asn Leu Asp Asp Glu Glu Leu Leu Val Phe Asn Pro Lys Ala
385                 390                 395                 400

Val Leu Pro Cys Phe Val Ile Val Tyr Thr Cys Glu Ala
                405                 410


<210>  42
<211>  450
<212>  PRT
<213>  NONE


<220>
<221>  misc_feature
<222>  (247)..(247)
<223>  Xaa can be any naturally occurring amino acid

<400>  42

Met Leu Glu Leu Arg Glu Arg Glu Asp Glu Glu Ala Gly Glu Glu Thr
1               5                   10                  15

Lys Gln Cys Arg Glu Met Lys Leu Ile Leu Leu Gln Leu Glu Asp Thr
            20                  25                  30
```

```
Tyr Arg Lys Gln Arg Ala Lys Gln Tyr Trp Leu Leu Phe Gly Asp Leu
        35                  40                  45

Asn Thr Lys Leu Tyr His Val Val Ala Asn Gly Thr Arg Lys Arg Asn
    50                  55                  60

Val Met Thr Gly Leu Arg Ala Thr Asp Gly Thr Trp Lys Asn Asn Val
65                  70                  75                  80

Gln Glu Met Ala Thr Ile Ala Ile Tyr Ile Val Glu Ala Lys Val Ala
                85                  90                  95

Ile Phe Gln Gly Tyr Gln Trp Lys Ile Gly Asp Gly Ala Lys Ile Leu
            100                 105                 110

Val Trp Glu Asp Pro Trp Ile Arg Arg Asn Asn Asp Met Val Glu Ser
        115                 120                 125

Val Ser Pro Leu Ser Asp Trp Asp Leu Arg Val Asn Ala Leu Leu Ser
    130                 135                 140

Ala Glu Gly Asp Glu Trp Asp Glu Glu Arg Val Arg Ser Leu Phe His
145                 150                 155                 160

Gln Arg Gly Ala Glu Glu Ile Leu Ser Ile Pro Leu Gln Gly Gly Gly
                165                 170                 175

Glu Asp Thr Ala Phe Trp Ser Tyr Ser Asn Asn Ala Met Phe Ser Glu
            180                 185                 190

Leu Gly Glu Thr Gly Val Gly Val Cys Val Arg Asp Ala Met Ala Met
        195                 200                 205

Leu Cys Phe Ser Glu Leu Gly Glu Thr Gly Val Gly Val Cys Val Arg
    210                 215                 220

Asp Ala Met Ala Met Val Ile Asn Leu Leu Asp Asp Thr Thr Arg Gly
225                 230                 235                 240

Gly Met Leu Met Gln Leu Xaa Phe Gln Ser Trp Gly Lys Arg Val Leu
                245                 250                 255

Val Cys Val Cys Glu Met Pro Tyr Gly Asp Ala Glu Leu Lys Tyr Gly
            260                 265                 270

Ser Pro Leu Leu Pro Phe Arg Ser Gln Ser Gly Arg Arg Phe Leu Thr
```

275 280 285

Pro Phe Leu Ser Pro Pro Leu Ser Lys Ser Arg Asp Arg Glu Arg Ser
290 295 300

Ser Ser Ser Asn Gly Val Arg Asp Ser Ile Arg Arg Ser Leu Ile Ile
305 310 315 320

Phe Ile Thr Gln Gln Val Pro Pro Pro Tyr Ala Ile Ala Ser Phe Phe
325 330 335

Val Phe Val Ser Lys Leu Phe Ile Ser Ala Asp Phe Pro Pro Pro His
340 345 350

Leu Cys Phe Ser Leu His Phe Pro Asp Ser Leu Leu Ala Lys Ser Phe
355 360 365

Phe Val Asn Arg Phe Ser Ile Cys Leu Ala Gly Thr Gly Glu Cys Gln
370 375 380

Ile Asp Asp Asp Ser Thr Ser Ser Arg Ser Ile Phe Asp Phe Leu Pro
385 390 395 400

Ser Phe Ile Ser Ser Ser Asn Ser Ser Lys Thr Ser Asn Val Cys Leu
405 410 415

Thr Asn Leu Ser Ser Ile Leu Ser Pro Asp Cys Gly Phe Leu His Pro
420 425 430

Met Ile Ile Thr Gly Gly Lys Pro Ala Ala Ile Val Tyr Ile Gln Leu
435 440 445

Glu Ala
450

<210> 43
<211> 504
<212> PRT
<213> NONE

<400> 43

Met Ala Trp Phe Arg Thr Leu Thr Arg Leu Ser Thr Thr Val Lys Ser
1 5 10 15

Phe Pro Pro Pro Pro Ile Arg Thr Thr Pro Val Ala Thr Ser Leu Ser
20 25 30

Tyr Phe Thr Thr Val Ala Ala Asp Ser Ala Pro Pro Pro His Pro Thr

```
            35                  40                  45

Leu Ser Pro Ser Pro Thr His Cys Gly Ser Leu Lys Pro Thr Thr Ser
    50                  55                  60

Gly Glu Lys Ala Arg Val Val Val Leu Gly Ser Gly Trp Ala Gly Cys
65                  70                  75                  80

Arg Leu Met Lys Gly Ile Asp Thr Thr Leu Tyr Asp Val Val Cys Val
                85                  90                  95

Ser Pro Arg Asn His Met Val Phe Thr Pro Leu Leu Ala Ser Thr Cys
            100                 105                 110

Val Gly Thr Leu Glu Phe Arg Ser Val Ala Glu Pro Val Gly Arg Ile
        115                 120                 125

Gln Pro Ala Ile Ser Ser Glu Pro Gly Ser Tyr Phe Phe Leu Ser Asn
    130                 135                 140

Cys Lys Gly Ile Asp Pro His Asn His Leu Val Lys Cys Glu Thr Val
145                 150                 155                 160

Thr Asp Gly Pro Asn Ala Val Glu Pro Trp Lys Phe Thr Ile Ala Tyr
                165                 170                 175

Asp Lys Leu Val Ile Ala Leu Gly Ala Glu Ala Thr Thr Phe Gly Ile
            180                 185                 190

Gln Gly Val Lys Glu His Ala Ile Phe Leu Arg Glu Val His Gln Ala
        195                 200                 205

Gln Glu Ile Arg Arg Lys Leu Leu Leu Asn Leu Met Leu Ser Asp Val
    210                 215                 220

Pro Gly Thr Thr Glu Gln Glu Lys Ser Arg Leu Leu His Cys Val Val
225                 230                 235                 240

Val Gly Gly Gly Pro Thr Gly Val Glu Phe Ser Gly Glu Leu Ser Asp
                245                 250                 255

Phe Ile Met Arg Asp Val Arg Gln Arg His Ala His Val Lys Asp Tyr
            260                 265                 270

Ile Arg Val Thr Leu Ile Glu Ala Asn Glu Ile Leu Ser Ser Phe Asp
        275                 280                 285
```

Asp Arg Leu Arg Gln Tyr Ala Thr Lys Gln Leu Thr Lys Ser Gly Val
    290                 295                 300

Arg Leu Val His Gly Ile Val Lys Asp Val Glu Ala Asp Lys Ile Val
305                 310                 315                 320

Leu Asp Asn Gly Thr Glu Val Pro Tyr Gly Leu Leu Val Trp Ser Thr
                325                 330                 335

Gly Val Gly Pro Ser Pro Leu Val Lys Ser Leu Asp Leu Pro Lys Ala
            340                 345                 350

Pro Gly Gly Arg Ile Gly Ile Asp Glu Trp Leu Arg Val Pro Asn Val
        355                 360                 365

Pro Asp Val Phe Ala Ile Gly Asp Cys Ser Gly Phe Val Glu Ser Thr
    370                 375                 380

Gly Lys Pro Val Leu Pro Ala Leu Ala Gln Val Ala Glu Arg Gln Gly
385                 390                 395                 400

Lys Tyr Leu Ala Ser Leu Leu Asn Arg Ile Gly Lys Ala Gly Gly Gly
                405                 410                 415

Arg Ala Asn Ser Gly Ala Glu Val Asp Phe Gly Thr Pro Phe Val Tyr
            420                 425                 430

Lys His Leu Gly Ser Met Ala Thr Leu Gly Arg Tyr Lys Ala Leu Val
        435                 440                 445

Asp Leu Arg Gln Ser Lys Glu Gly Lys Gly Ile Ser Leu Ala Gly Phe
    450                 455                 460

Val Ser Trp Phe Ile Trp Arg Ser Ala Tyr Leu Thr Arg Val Ile Ser
465                 470                 475                 480

Trp Arg Asn Arg Phe Tyr Val Ala Val Asn Trp Met Thr Thr Met Val
                485                 490                 495

Phe Gly Arg Asp Ile Ser Arg Ile
            500

<210>  44
<211>  504
<212>  PRT
<213>  NONE

<400>  44

```
Met Ala Trp Phe Arg Thr Leu Thr Arg Leu Ser Thr Thr Val Lys Ser
1               5                   10                  15

Phe Pro Pro Pro Pro Ile Arg Thr Thr Pro Val Ala Thr Ser Leu Ser
            20                  25                  30

Tyr Phe Thr Thr Val Val Ala Asp Asn Ala Pro Pro Pro His Pro Thr
        35                  40                  45

Leu Ser Pro Ser Pro Thr His Cys Gly Ser Leu Lys Pro Thr Thr Asn
    50                  55                  60

Gly Glu Lys Ala Arg Val Val Val Leu Gly Ser Gly Trp Ala Gly Cys
65                  70                  75                  80

Arg Leu Met Lys Gly Ile Asp Thr Thr Leu Tyr Asp Val Val Cys Val
                85                  90                  95

Ser Pro Arg Asn His Met Val Phe Thr Pro Leu Leu Ala Ser Thr Cys
            100                 105                 110

Val Gly Thr Leu Glu Phe Arg Ser Val Ala Glu Pro Val Gly Arg Ile
        115                 120                 125

Gln Pro Ala Ile Ser Ser Glu Pro Gly Ser Tyr Phe Phe Leu Ser Asn
    130                 135                 140

Cys Lys Gly Ile Asp Pro His Asn His Leu Val Lys Cys Glu Thr Val
145                 150                 155                 160

Thr Asp Gly Pro Asn Ala Val Glu Pro Trp Lys Phe Thr Ile Ala Tyr
                165                 170                 175

Asp Lys Leu Val Ile Ala Ser Gly Ala Glu Ala Thr Thr Phe Gly Ile
            180                 185                 190

Gln Gly Val Lys Glu His Ala Ile Phe Leu Arg Glu Val His Gln Ala
        195                 200                 205

Gln Glu Ile Arg Arg Lys Leu Leu Leu Asn Leu Met Leu Ser Asp Val
    210                 215                 220

Pro Gly Thr Thr Glu Gln Glu Lys Ser Arg Leu Leu His Cys Val Val
225                 230                 235                 240

Val Gly Gly Gly Pro Thr Gly Val Glu Phe Ser Gly Glu Leu Ser Asp
                245                 250                 255
```

Phe Ile Met Lys Asp Val Arg Gln Arg His Ala His Val Lys Asp Tyr
260 265 270

Ile Arg Val Thr Leu Ile Glu Ala Asn Glu Ile Leu Ser Ser Phe Asp
275 280 285

Asp Arg Leu Arg Gln Tyr Ala Thr Lys Gln Leu Thr Lys Ser Gly Val
290 295 300

Arg Leu Val Arg Gly Ile Val Lys Asp Val Glu Ala Asp Lys Ile Val
305 310 315 320

Leu Asp Asn Gly Thr Glu Val Pro Tyr Gly Leu Leu Val Trp Ser Thr
325 330 335

Gly Val Gly Pro Ser Pro Leu Val Lys Ser Leu Asp Leu Pro Lys Ser
340 345 350

Pro Gly Gly Arg Ile Gly Ile Asp Glu Trp Leu Arg Val Pro Asn Val
355 360 365

Pro Asp Val Phe Ala Ile Gly Asp Cys Ser Gly Phe Val Glu Ser Thr
370 375 380

Gly Lys Pro Val Leu Pro Ala Leu Ala Gln Val Ala Glu Arg Gln Gly
385 390 395 400

Lys Tyr Leu Ala Ser Leu Leu Asn Arg Ile Gly Lys Ala Gly Gly Gly
405 410 415

Arg Ala Asn Ser Gly Ala Asp Val Asp Phe Gly Thr Pro Phe Val Tyr
420 425 430

Lys His Leu Gly Ser Met Ala Thr Leu Gly Arg Tyr Lys Ala Leu Val
435 440 445

Asp Leu Arg Gln Ser Lys Glu Gly Lys Gly Ile Ser Leu Ala Gly Phe
450 455 460

Val Ser Trp Phe Ile Trp Arg Ser Ala Tyr Leu Thr Arg Val Ile Ser
465 470 475 480

Trp Arg Asn Arg Phe Tyr Val Ala Val Asn Trp Met Thr Thr Met Val
485 490 495

Phe Gly Arg Asp Ile Ser Arg Ile
500

<210> 45
<211> 514
<212> PRT
<213> NONE

<400> 45

Met Ala Pro Phe Asp Phe Lys Ser Thr Asn His Phe Val Ala Ser Ser
1               5                   10                  15

Asp Gly Thr Thr Leu Gln Ile Tyr Asn Leu Met Ala Lys Ala Met Val
            20                  25                  30

His Pro Phe Ser Ile Arg Lys Ala His Trp Glu Glu Val Ala Asn Ile
        35                  40                  45

Asn Met Ile Thr Leu Glu Asp Asp Asp Asp Asp Asp Glu Glu Thr Gln
    50                  55                  60

Gln Ile Ser Tyr Asp Gly Leu Leu Phe Phe Asp Gly His Gly Arg Ile
65                  70                  75                  80

Glu Gly Thr Lys Ala Val Thr Val Leu Ala Gln His Leu Lys Arg Leu
                85                  90                  95

Arg Leu Leu Val Asn Pro Glu Gly Tyr Cys Phe Gln Glu Ser Val Glu
            100                 105                 110

Leu Ala Asp Phe Gly Ile His Tyr Leu Arg Leu Asn Asp Leu Phe Ser
        115                 120                 125

Trp Pro His His Gly Val Asn Leu Ala Thr Thr Ser Val Trp Gly Arg
    130                 135                 140

Ser Glu Leu Lys Tyr Gln Val Thr Lys Pro Lys Pro Thr Thr Thr Ala
145                 150                 155                 160

Asp His His His Glu His Gly Ala Asn His Leu Gln Asn Lys Leu Glu
                165                 170                 175

Glu Leu Lys Arg Leu Leu Lys Glu Lys Glu Asp Glu Leu Glu Lys Ala
            180                 185                 190

Asp Glu Val Lys Asp Glu Glu Leu Glu Asn Leu Arg Lys Glu Lys Asp
        195                 200                 205

Glu Glu Met Arg Val Leu Arg Lys Lys Lys Asp Glu Glu Lys Val Lys
    210                 215                 220

```
Ala Arg Lys Ala Glu Glu Ala Leu Glu Lys Leu Lys Glu Glu Ile Glu
225                 230                 235                 240

Asp Ser Lys Glu Ser Lys Asp Glu Glu Met Glu Gln Leu Arg Lys Gln
                245                 250                 255

Leu Lys Arg Lys Asp Glu Glu Met Arg Ile Leu Lys Arg Lys Lys Asp
            260                 265                 270

Lys Asp Gln Glu Ala Asn Ser Lys Arg Ile Asp Glu Leu Glu Lys Leu
        275                 280                 285

Lys Glu Glu Met Glu Asp Ala Asn Glu Glu Leu Arg Arg Ser Leu Lys
    290                 295                 300

Leu His Glu Gln Asn Gly Lys Val Asp Ile Lys Ser Thr Arg Arg Ile
305                 310                 315                 320

Ala Glu Leu Glu Lys Gln Lys Asn Glu Glu Leu Glu Arg Ile Thr Arg
                325                 330                 335

Glu Lys Asn Asp Glu Ile Ala Arg Ile Lys Lys Asp Leu Glu Gln Cys
            340                 345                 350

Ser Lys Val Ser Trp Ala Ile Ser Pro Ser Val Arg Tyr Leu Pro Lys
        355                 360                 365

Gly Ala Glu Gly Glu Arg Val His Leu Thr Arg Arg Arg Arg Val Pro
    370                 375                 380

Pro Glu Thr Phe Val Tyr Thr Glu Gln Ser Tyr Asp Phe Val Ser Ser
385                 390                 395                 400

Asn Met Pro Arg Ile Glu Ala Cys Leu Asn Val Phe Asn Thr Ala Gln
                405                 410                 415

Phe Val Asp Glu Asp Ser Thr Gly Trp Pro Asn Pro Thr Ser Phe Asn
            420                 425                 430

Val Leu Ile Gly Ser Ser Asn Arg Gly Phe Asp Arg Asn Asn Trp Trp
        435                 440                 445

Phe Ala Lys Thr Asp His Pro Glu Gly Gly Leu Thr Met Leu Ala Tyr
    450                 455                 460

Tyr Asp Gly Ser Phe Ser Gln Phe Ser Ser Gly Pro Asp Arg Thr Ile
```

465 470 475 480

Arg Leu Pro Phe His Lys Asp Ala Ser Leu Ala Pro Tyr Lys Pro Phe
485 490 495

Phe Leu Leu Asn Val His Ser Phe Gly Val Lys Leu Ala Phe Met Tyr
500 505 510

Arg Val

<210> 46
<211> 574
<212> PRT
<213> NONE

<400> 46

Met Ala Ala Ala His Leu Val Ser Asp Ser Gln Ser Glu Met Arg Gly
1 5 10 15

Val Thr Thr Thr Thr Asn Ala Thr Val Asp Thr Val Asn Ala Ala Ala
20 25 30

Thr Ala Ile Val Ser Ala Glu Ser Arg Val Gln Pro Ala Ala Val Pro
35 40 45

Val Ile Lys Glu Met Ala Asp Lys Val Leu His Arg Ile Ala Leu Phe
50 55 60

Leu Gly Leu Thr Met Leu Lys Arg Arg Trp Gly Gly Cys Trp Ser Met
65 70 75 80

Tyr Trp Cys Phe Gly Ala His Lys Ser Asp Lys Arg Ile Gly His Ala
85 90 95

Val Leu Val Pro Glu Pro Glu Ser Gln Arg Val Ala Pro Ser Ser Ser
100 105 110

Val Thr Gln Ser His Ser Thr Ala Val Val Leu Pro Phe Ile Ala Pro
115 120 125

Pro Ser Ser Pro Ala Ser Phe Leu Gln Ser Asp Pro Ser Ser Val Thr
130 135 140

Gln Ser Pro Ala Gly Lys Leu Ser Leu Asn Ser Leu Ser Ala Asn Ala
145 150 155 160

Tyr Ser Pro Arg Gly Pro Pro Ser Met Phe Ala Ile Gly Pro Tyr Ala

165 170 175

His Glu Thr Gln Leu Val Thr Pro Pro Val Phe Ser Ala Phe Thr Thr
180 185 190

Glu Pro Ser Thr Ala Pro Phe Thr Pro Pro Pro Glu Ser Val Gln Leu
195 200 205

Thr Thr Pro Ser Ser Pro Glu Val Pro Phe Ala Gln Leu Leu Thr Ser
210 215 220

Ser Leu Glu Arg Ala Arg Arg Asn Ser Gly Pro Thr Pro Lys Tyr Gly
225 230 235 240

Phe Ser Asn Tyr Glu Phe Pro His Val Tyr Pro Gly Ser Pro Gly Ala
245 250 255

Gln Leu Ile Ser Pro Gly Ser Thr Val Ser Tyr Ser Gly Thr Ser Ser
260 265 270

Pro Phe Pro Asp Arg His Pro Val Leu Gly Phe Pro Thr Gly Glu Ala
275 280 285

Pro Lys His Ile Gly Tyr Glu His Phe Asn Thr Arg Lys Trp Gly Ser
290 295 300

Arg Leu Gly Ser Gly Ser Leu Thr Pro Asp Gly Val Gly Arg Gly Ser
305 310 315 320

Arg Leu Ala Ser Gly Thr Thr Thr Pro Asp Gly Ile Gly Leu Gly Ser
325 330 335

Arg Leu Ala Ser Gly Thr Thr Thr Pro Asp Gly Ile Gly Leu Gly Ser
340 345 350

Arg Leu Ala Ser Gly Thr Thr Thr Pro Asp Gly Ile Gly Leu Gly Ser
355 360 365

Arg Leu Ala Ser Gly Thr Val Thr Pro Asp Gly Ile Gly Leu Gly Ser
370 375 380

Arg Leu Gly Ser Gly Thr Ala Thr Pro Asp Gly Gly Ser Ile Tyr Ser
385 390 395 400

Arg Met Gly Ser Gly Ala Leu Thr Pro Gly Ile Gln Glu Gly Val Leu
405 410 415

```
Leu Met Asn Pro Val Leu Glu Ala Ala His Leu Pro Leu Leu Gln Asn
            420                 425                 430

Gly Ala Ala Lys Thr His Glu Ala Met Val Asp His Arg Val Ser Phe
        435                 440                 445

Glu Leu Ser Gly Glu Asp Val Ala Arg Cys Leu Glu Asn Lys Ser Met
    450                 455                 460

Ala Ser Asn Arg Thr Phe Leu Glu Ser Val Asp Asp His Gly Glu Ser
465                 470                 475                 480

Gly Glu Ser Leu Met Arg Ser Glu Val Arg Leu Arg Val Gly Asp Thr
                485                 490                 495

Ser Ser Asn Gly Ser Pro Gly Lys Pro Thr Ser Gly Glu Val Ala Glu
            500                 505                 510

Asp Glu Pro Ser Phe Arg Arg Gln Arg Ser Val Thr Leu Gly Ser Ile
        515                 520                 525

Lys Glu Phe Asn Phe Asp Ser Ser Lys Gly Glu Thr Ile Asp Lys Ser
    530                 535                 540

Glu Trp Trp Ala Asn Glu Ala Ile Ala Gly Lys Glu Ser Lys Pro Ala
545                 550                 555                 560

Asn Gly Trp Ser Phe Phe Pro Ile Leu Gln Pro Glu Val Ser
                565                 570
```